# EUROPEAN PATENT SPECIFICATION

(11) **EP 1 501 848 B1**
(45) Date of publication and mention of the grant of the patent: **08.08.2007**
(21) Application number: 03718663.2
(22) Date of filing: 08.05.2003
(51) Int. Cl.: C07H 19/00

(54) **SYNTHESIS OF LOCKED NUCLEIC ACID DERIVATIVES**
SYNTHESE VON LOCKED NUCLEIC ACID-DERIVATEN
SYNTHESE DE DERIVES D'ACIDES NUCLEIQUES LNA

(30) Priority: 08.05.2002 DK 200200712; 16.08.2002 DK 200201214
(43) Date of publication of application: 02.02.2005
(73) Proprietor: Santaris Pharma A/S, 2970 Hørsholm (DK)
(72) Inventor: SORENSEN, Mads, Detlef, DK-2200 Kobenhavn N (DK); WENGEL, Jesper, DK-5260 Odense S. (DK); KOCH, Troels, DK-2300 Copenhagen S (DK); CHRISTENSEN, Signe, M., DK-2200 Copenhagen N (DK); Rosenbohm, Christoph, 3460 Birkerod (DK); Pedersen, Daniel Sejer, Darwin College, Cambridge CB3 9EU (GB)
(74) Representative: Plougmann & Vingtoft A/S
(86) International application number: PCT/DK2003/000305
(87) International publication number: WO 2003/095467

(56) References cited:
- WO-A-00/66604
- WO-A-02/28875
- ULRICH JORDIS ET AL: "Synthesis of (1S,4S)-2-Thia-5-azabicyclo(2.2.1)heptane" INDIAN JOURNAL OF CHEMISTRY, vol. 28B, April 1989 (1989-04), pages 294-296, XP002252478
- LISBET KVAERNO ET AL: "Synthesis of a Novel Bicyclic Nucleoside Restricted to an S-Type Conformation and Initial Evaluation of Its Hybridization Properties When Incorporated into Oligodeoxynucleotides " J. ORG. CHEM., vol. 66, 2001, pages 5106-5112, XP002252479
- VELIMIR POPSAVIN ET AL: "An alternative synthesis of (+)-epiallo-muscarine from D-glucose" CARBOHYDRATE RESEARCH, vol. 269, 1995, pages 343-347, XP002252480
- SANJAY K. SINGH ET AL: "Synthesis of 2'-Amino-LNA: A Novel Conformationally Restricted High-Affinity Oligonucleotide Analogue with a Handle" J. ORG. CHEM., vol. 63, 1998, pages 10035-10039, XP002252481
- GEORGE W. J. FLEET ET AL: "The synthesis from D-xylose of the potent and specific enantiomeric glucosidase inhibitors, 1,4-dideoxy-1,4-imino-D-arabinitol and 1,4-dideoxy-1,4-imino-L-arabinitol" TETRAHEDRON, vol. 42, no. 20, 1986, pages 5685-5692, XP002252482
- CHRISTOPH ROSENBOHM ET AL: "Synthesis of 2'-amino-LNA: a new strategy" ORGANIC & BIOMOLECULAR CHEMISTRY, vol. 1, no. 4, 2003, pages 655-663, XP002252483
- TORSTEN BRYLD ET AL: "Synthesis and antiviral evaluation of novel conformationally locked mucleosides and masked 5'-phosphate derivatives thereof" J. CHEM. SOC., PERKIN TRANS., vol. 1, 2002, pages 1655-1662, XP002252484

## Description

### FIELD OF THE INVENTION

The present invention relates to a novel strategy for the synthesis of Locked Nucleic Acid derivatives, such as amino-LNA, thio-LNA, seleno-LNA and methylene-LNA, which provides scalable high yielding reactions utilising intermediates that also can produce other LNA analogues such as oxy-LNA. The invention further relates to a novel strategy for the synthesis of α-L-LNA analogues and precursors.

### BACKGROUND OF THE INVENTION

Professor Imanishi (WO 98/39352) and Professor Wengel (WO 99/14226) independently invented Locked Nucleic Acid (LNA) in 1997 and the first LNA monomer was based on the 2'-O-CH₂-4' bicyclic structure (oxy-LNA). This LNA analogue has since then showed promising results as antisense drug candidates. Other LNA analogues has also been synthesized exhibiting similar high affinity/specificity for example 2'-NH-CH₂-4', 2'-N(CH₃)-CH₂-4' (amino-LNA) (Singh, S. K.; Kumar, R.; Wengel, J. J.Org.Chem. 1998, 63, 10035-10039; Singh, S. K.; Kumar, R.; Wengel, J. J.Org.Chem. 1998, 63, 6078-6079), and 2'-S-CH₂-4' (thio-LNA) (Singh, S. K.; Kumar, R.; Wengel, J. J.Org.Chem. 1998, 63, 6078-6079, Kumar, R.; Singh, S. K et al. Biorg.Med.Chem.Lett. 1998, 8, 2219-2222). Large quantities of amino-LNA are crucial for its use in antisense. Scaling-up the previously described method of synthesis of amino-LNA has appeared to be difficult and encountered several major problems.

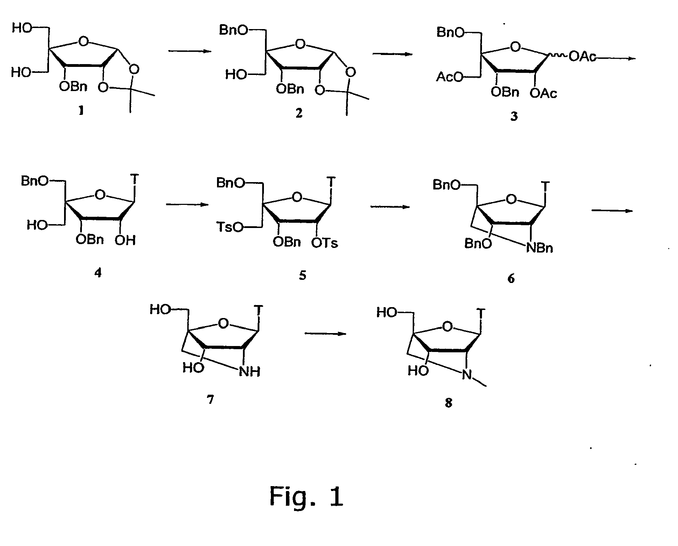

The first difficult reaction in the scale up work proved to be the regioselective benzylation of 3-*O*-benzyl-1,2-*O*-isopropylidene-4-*C*-hydroxymethyl-α-D-*erythro*-pentofuranose (Koshkin, A.; Singh, S. K-; Nielsen, P.; Rajwanshi, V. K.; Kumar, R.; Meldgaard, M.; Olsen, C. E.; Wengel, J. Tetrahedron 1998, 54, 3607-3630) (see Figure 1, compound 1).

Working in the 100 g range the reaction yielded a product-mixture of compound **2**, the 1'-benzylated and the di-benzylated material even under optimised conditions. The maximum yield of the desired compound **2** was 59% dropping to an average of 45-50% compared to 71% on smaller scale. Furthermore, compound **2** could only be isolated through tedious chromatography of closely eluting products.

The second key step in the original strategy causing problems during scale-up synthesis was the double nucleophilic substitution of the di-*O*-tosyl nucleoside **5** using benzylamine giving nucleoside **6** (Singh, S. K.; Kumar, R.; Wengel, J. J.Org.Chem. 1998, 63, 10035-10039). The reaction on larger scale (22 g) apparently afforded a second product identified as the oxy-LNA derivative. The desired *N*-benzylated-amino-LNA product **6** was obtained in only 15% together with 13% of the oxy-LNA by-product. For comparison, the reaction gives 52% of nucleoside 6 on a 8 g scale with no side reaction reported (Singh, S. K.; Kumar, R.; Wengel, J. J.Org.Chem. 1998, 63, 10035-10039).

Yet another problem encountered appeared to be the debenzylation of nucleoside 6 using ammonium formate and 10% Pd/C In methanol. It appeared to be only partial debenzylation as verified by mass spectroscopy, and the product **7** proved to be difficult to isolate from the reaction mixture.

The first synthesis of an oxy-LNA nucleoside was performed by a linear approach using uridine as starting material (Obika, S.; Nanbu, D.; Harl, Y.; Morio, J. A. K.; In, Y.; Ishida, T.; Imanishi, T. Tet.Lett. 1997, 38, 8735-8738) but by virtue of being a convergent synthesis the route developed by Wengel and coworker (Koshkin, A.; Singh, S. K.; Nielsen, P.; Rajwanshi, V. K.; Kumar, R.; Meldgaard, M.; Olsen, C. E.; Wengel, J. Tetrahedron 1998, 54, 3607-3630; Koshkin,A.A. et al., J.Org.Chem. 2001, 66, 8504-8512) became the method of choice for the synthesis of LNA nucleosides. WO 02/28875 discloses the use of S_{N2} substitution reactions in the synthesis of LNA analogues. However, the S_{N2} substitution reactions shown in WO 02/28875 involve two independent reactions; one is a nucleophilic substitution on the nucleobase and the other is a deprotection step facilitating a nucleophile ring closures. None of the reactions involve inversion of stereochemistry on the carbohydrate moiety.

Amino- and thio-LNA was originally synthesised quite differently, but according to the present Invention there are common intermediates that can be used for amino-LNA, thio-LNA, seleno-LNA, α-L-LNA as well as methylene-LNA at late stages in the overall synthesis.

Ulrich et al. Indian Journal of Chemistry, 1989, 28B, 294-296 and Popsavin et al. Carbo. Res., 1995, 269, 343-347) both teach the construction of simple bicyclic systems using a sulfur nucleophile but none of the reactions involve nucleosides.

### SUMMARY OF THE INVENTION

The present Invention provides a novel strategy for the synthesis of LNA derivatives, such as α-L-oxy-LNA, amino-LNA, α-L-amino-LNA, thio-LNA, α-L-thio-LNA, seleno-LNA and methylene-LNA.

The compounds of the formula I are important intermediates that may be reacted with varieties of nucleophiles leading to a wide variety of LNA analogues, e.g. amino-LNA, thfo-LNA, seleno-LNA and methylene-LNA.

One aspect of the invention relates to a method for synthesis of LNA analogues of the formula IV starting from compounds of formula I, d. claim 1.

Another aspect of the present invention relates to the novel compounds (intermediates) of the formula I and IX as defined in claims 20 and 21.

A stil further aspect of the invention relates to oligonucleotides comprising a compound of formula XI, cf. claim 25.

The main advantages of the present invention comprises the following:
- tedious separation of regloisomers is eliminated,
- the low-yielding step of double nucleophilic substitution of di-O-tosyl nucleoside using benzylamine is avoided,
- the method enables the utilisation of a starting intermediates which is common to the known oxy-LNA synthesis,
- the method comprises a novel intermediate that when reacted with appropriate nucleophilic can produce a variety of LNA analogues, i.e. amino-LNA, thio-LNA, seleno-LNA, methylene-LNA, and α-L-LNA,
- the method comprises an alternative method for N-methylation, and hereby avoids methylation at the nucleobase,
- employs cheap and commercial available reagents,
- comprises scalable reactions giving access to large quantities of LNA analogue phosphoramidites.

### DESCRIPTION OF THE FIGURES

Figure 1 illustrates a known method for the preparation of amino-LNA according to Singh, S. K.; Kumar, R.; Wengel, J. J.Org.Chem. 1998, 63, 10035-10039.

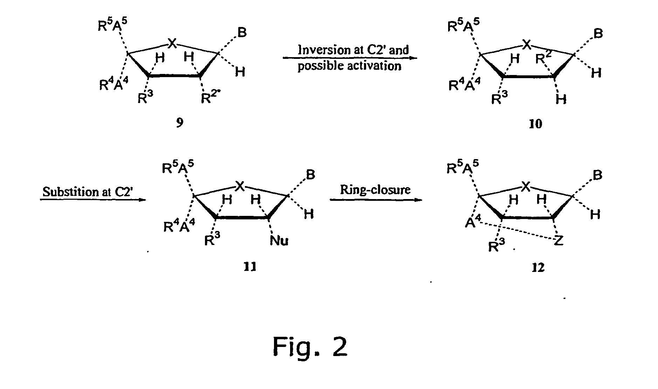

Figure 2 illustrates the generalised method for the preparation of the LNA analogues.

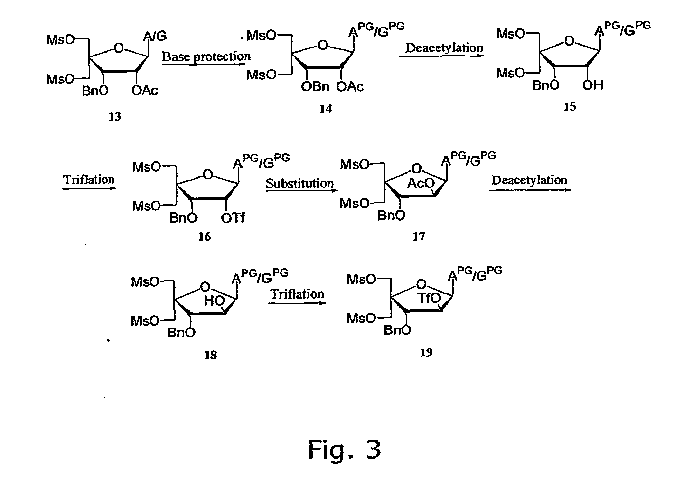

Figure 3 illustrates inversion at C2' for a compound not having a pyrimidine base.

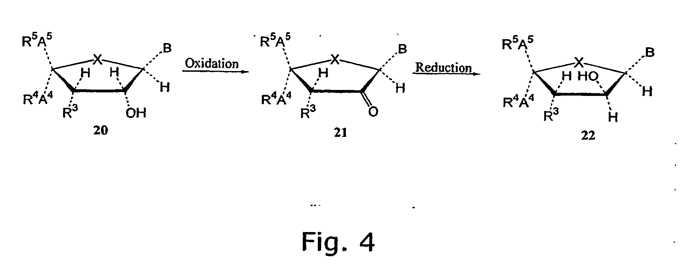

Figure 4 illustrates a further alternative for inversion at C2'.

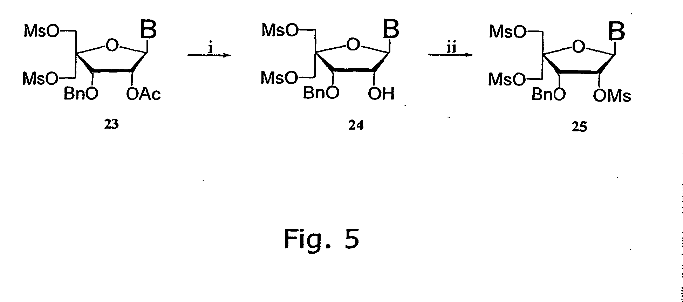

Figure 5 illustrates the synthesis of a preferred compound of the formula VII. The known compound 1-(2-*O*-acetyl-3-*O*-benzyl-4-*C*-methanesulfonyloxymethyl-5-*O*-methanesulfonyl-β-D-*erythro*-pentofuranosyl) thymine **(23)** is converted by a mild deacetylation for the liberation of the 2'-hydroxy group to the compound **(24)** without the subsequent ring-closure that affords the oxy-LNA skeleton. The 2'-hydroxy group is then mesylated to 1-(3-*O*-benzyl-4-*C*-methanesulfonyloxymethyl-2,5-*O*-dimethanesulfonyl-β-D-*erythro-*pentofuranosyl) thymine **(25).** Legend: i) 50% methanolic ammonia; ii) MsCl, pyridine.

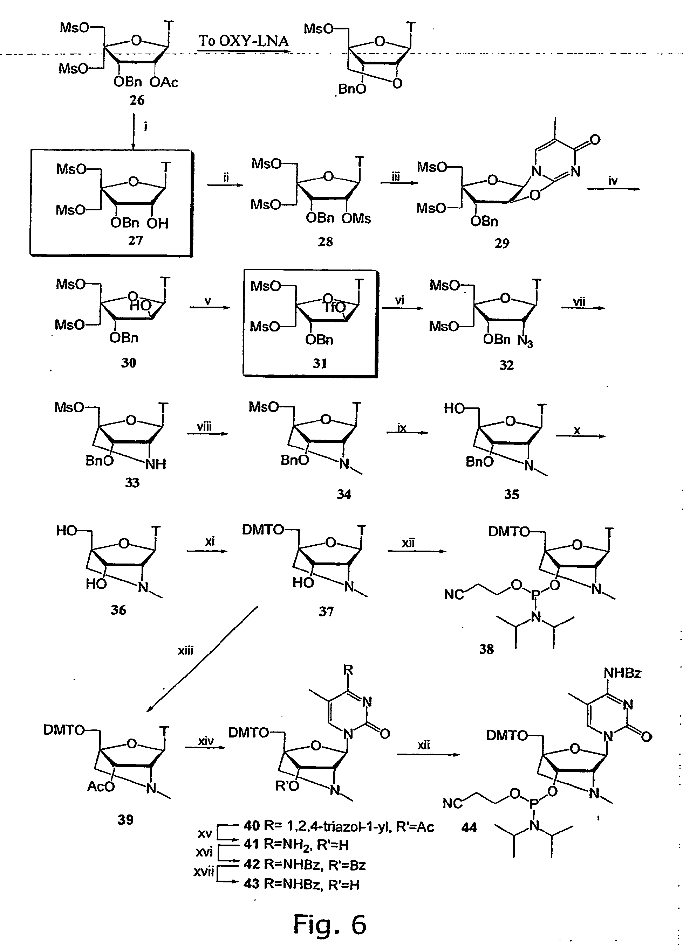

Figure 6 illustrates a preferred example for the preparation of two amino-LNA phosphoramidite that are useful in the preparation of oligonucleotides. Legend: i) half sat. NH₃ in MeOH; ii) MsCl, anh. pyridine, anh. CH₂Cl₂; iii) DBU, DMF; iv) acetone, 0.1M H₂SO₄; v) Tf₂O, DMAP, anh. pyridine, anh. CH₂Cl₂; vi) NaN₃, anh. DMF; vii) PMe₃, NaOH (aq), THF; viii) CH₂O, HCO₂H; ix) a) NaOBz, DMF, b) NaOMe; x) 20% Pd(OH)₂/C, H₂, AcOH; xi) DMTCl, anh. pyridine; xii) NC(CH₂)₂OP(N(iPr)₂)₂, DCI, CH₃CN, CH₂Cl₂. xiii) AC₂O, pyridine; xiv) Et₃N, 1,2,4-triazole, POCl₃, MeCN; xv) 1:1 MeCN, sat. aq NH₃; xvi) BzCl, pyridine; xvii) LiOH (aq), THF.

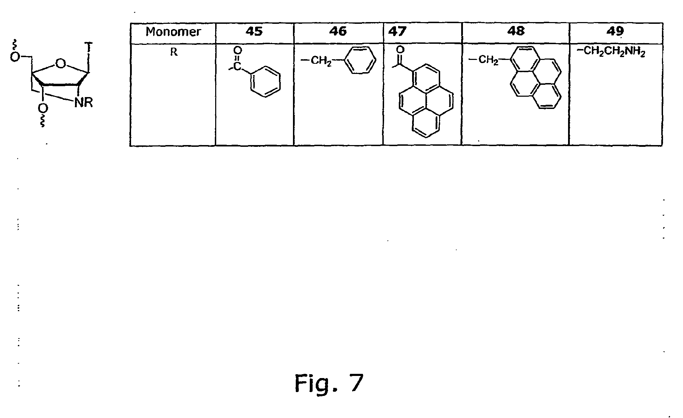

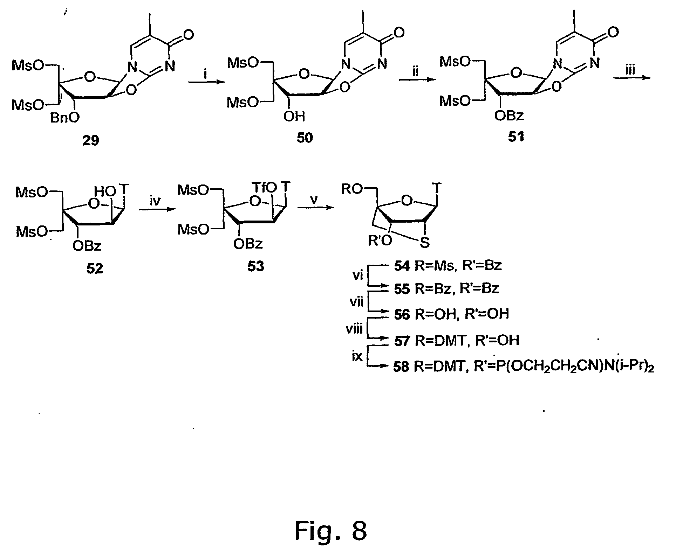

Figure 7 illustrates further acylation and alkylation of the 2'-amino group of amino-LNA Figure 8 illustrates a preferred example for the preparation of a thio-LNA phosphoramidite that is useful in the preparation of oligonucleotides. Legend: i) Pd/C, H₂, Acetone, MeOH; ii) BzCl, Pyridine, DMF; iii) 0.25 M aq. H₂SO₄, DMF, 80°C; iv) Tf₂O, DMAP, CH₂Cl₂, 0°C; v) Na₂S, DMF; vi) NaOBz, DMF, 100°C; vii) NH₃, MeOH; viii) DMT-Cl, Pyridine; ix) P(OCH₂CH₂CN)(N(i-Pr)₂)₂, 4,5-Dicyanoimidazole, CH₂Cl₂.

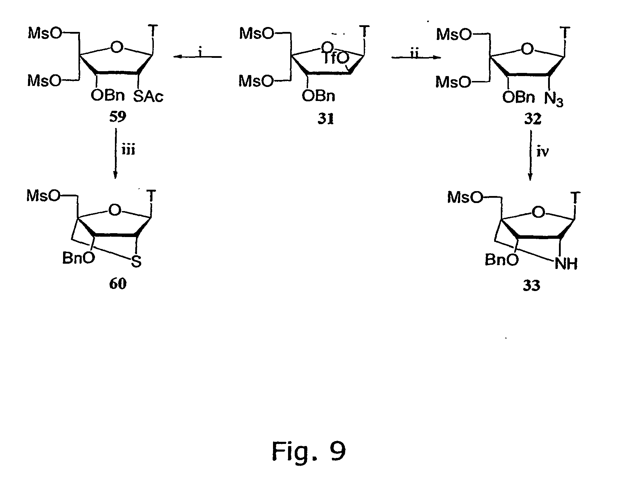

Figure 9 Illustrates the synthesis of an amino-LNA analogue **33** and a thio-LNA analogue **60** from the key intermediate **31** (a preferred example of a compound of Formula I). Legend: i) potassium thioacetate, DMF, ii) sodium azide in DMF, iii) LiOH in THF, iv) NaOH (aq.), Me₃P, THF.

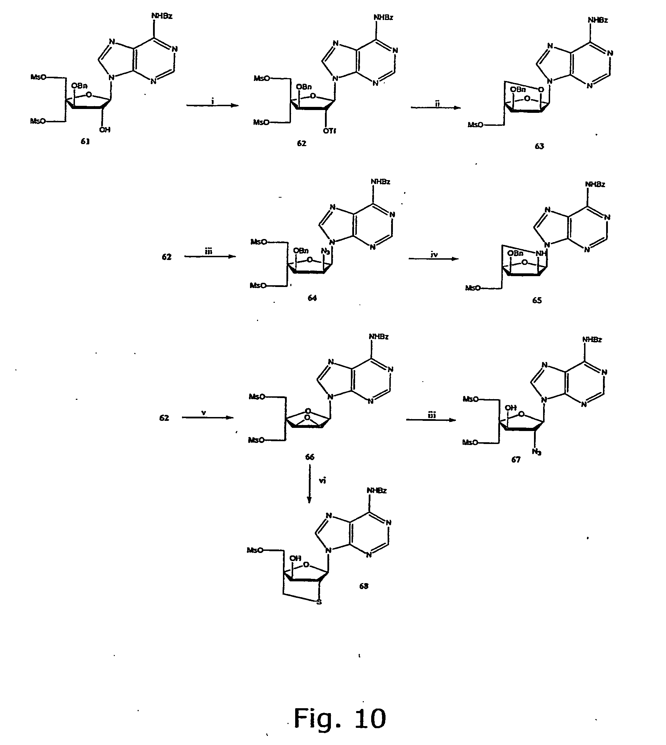

Figure 10 illustrates the synthesis of the α-L-oxy-LNA A **(63),** α-L-amino-LNA A **(65),** as well as the synthesis of an epoxide **(66)** from the key intermediate **62,** which is opened up with different nucleophiles to form either an azide **(67)** or a thio-LNA **(68)**. Legend: i) Tf₂O, pyridine, DCM, ii) LiOH, aq, THF, iii) NaN₃, DMF, iv) NaOH, PMe₃, THF, v) MsOH, DCM, vi) Na₂S, DMF.

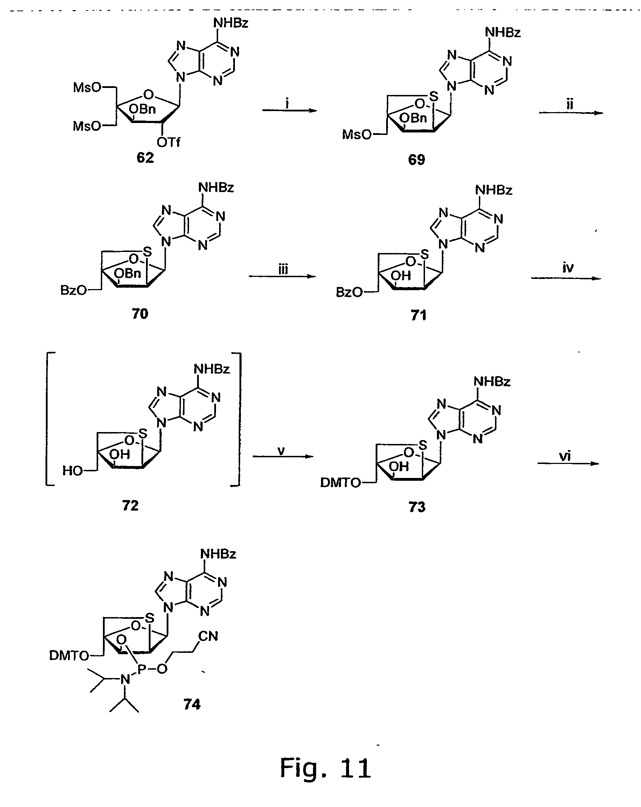

Figure 11 illustrates a preferred example for the preparation of an α- L -thio-LNA phosphoramidite that is useful in the preparation of oligonucleotides. Legend: i) Na₂S, DMF, ii) NaOBz, DMSO, 100°C, iii) MsOH, DCM, iv) LiOH, aq, THF, v) DMT-Cl, DMAP, Pyridine vi) P(OCH₂CH₂CN)-(N(i-Pr)₂)₂, 4,5-Dicyanoimidazole, CH₂Cl₂.

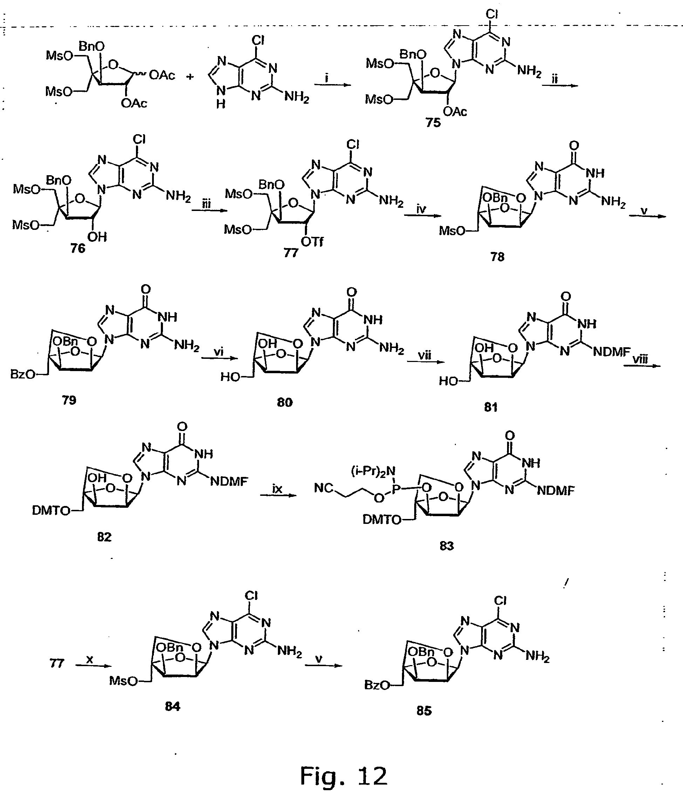

Figure 12 illustrates a preferred example for the preparation of an α- L-LNA-G phosphoramidite that is useful in the preparation of oligonucleotides. Legend: i) BSA, TMSOTf, ClCH₂CH₂Cl, ii) half sat. methanolic NH₃, iii) Tf₂O, DMAP, pyridine, CH₂Cl₂, iv) HOCH₂CH₂CN, NaH, THF; v) NaOBz, DMSO; vi) NH₄HCO₂, Pd(OH)₂-C, MeOH; vii) (CH₃O)₂CHN(CH₃)₂, DMF; viii) DMT-Cl, pyridine, ix) NC(CH₂)₂OP(N(iPr)₂)₂, 4,5-dicyanoimidazole, MeCN, CH₂Cl₂, x) LiOH, aq, THF.

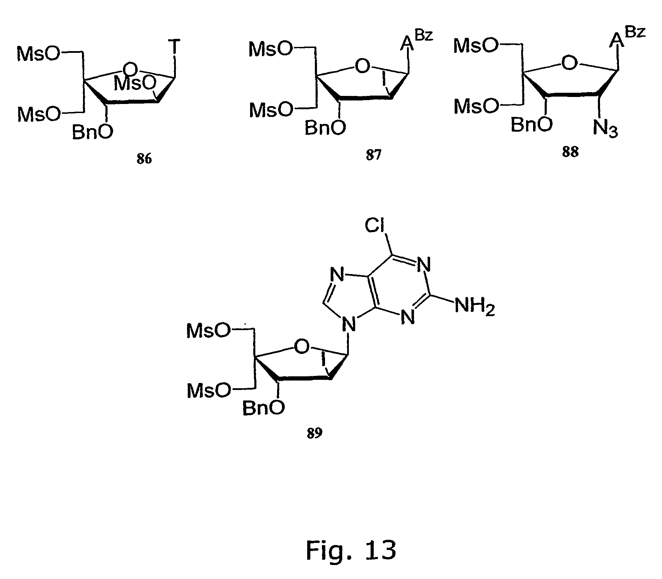

Figure 13 illustrates particularly interesting compounds according to the invention.

### DETAILED DESCRIPTION OF THE INVENTION

### Synthesis of LNA analogues

A main aspect of the present invention relates to a method for the synthesis an LNA analogue of the general formula IV 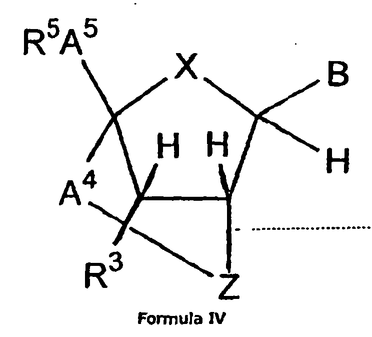 wherein
X is selected from -CH₂-, -NR^{H}-, -O-, and -S-;
Z is selected from -CH₂-, -NR^{H}-, -S-, and -Se-;
B is a nucleobase;
R³ is selected from -R^{H}, -N₃, -NR^{H}R^{H*}, -NR^{H}C(O)R^{H*}, -C(O)NR^{H}R^{H*}, -OR^{H}, -OC(O)R^{H}, -C(O)OR^{H}, -SR^{H}, -SC(O)R^{H}, and tri(C₁₋₆-alkyl/aryl)silyloxy;
each R^{H} and R^{H}* independently being selected from hydrogen, optionally substituted C₁₋₆-alkyl, optionally substituted aryl, and optionally substituted aryl-C₁₋₆-alkyl;
A⁴ and A⁵ independently are selected from C₁₋₆-alkylene; and
R⁵ is selected from iodo, bromo, chloro, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen;
   said method comprising the following steps:
   treating an intermediate of the general formula I: 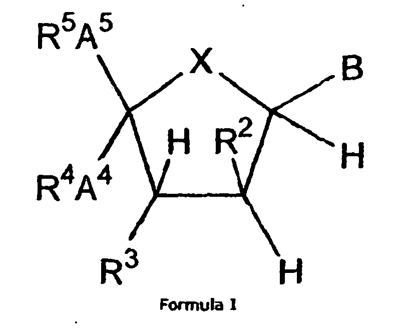
wherein
X, B, R³, A^{*}, and A⁵ are as defined above;
R² is selected from iodo, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen; R³ and R² may together form an epoxide and R⁴ and R⁵ independently are as defined for R⁵ above ;
with a nucleophile selected from halogen, ⁻N₃, ⁻NR^{H}R^{H*}, ⁻SR^{H}, ⁻⁻S, ⁻SeR^{H}, ⁻⁻Se, ⁻NR^{H}C(O)R^{H*}, ⁻SC(O)R^{H}, and organometallic hydrocarbyl radicals,
so as to substitute R², and
effecting ring-closure between the C2' and C4' positions so as to yield the LNA analogue of the formula IV.

It has been found that the Intermediates of the formula I play an Important role In the synthesis of the LNA analogues. Hence, the particular selection of substituents in the Intermediates has proved to be important for the efficient route to the LNA analogues. It should be understood that the substituents X, B, R³, A⁴, A⁵, and R⁵ most often will be unaltered In the synthesis, i.e. these substituents will be "carried over" from Formula I to formula IV. Also the absolute orientation of these substituents will also be preserved.

This being said, it may be necessary to protect the nucleobase as will be appreciated by the person skilled in the art (see further below under the definition of "nucleobase" and Figure 3).

In an interesting embodiment, the substituents of the compound of the formula I are selected so that R² is selected from C₁₋₆-alkylslfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen;
R³ is optionally substituted aryl(C₁₋₆-alkyl)oxy; and
R⁴ and R⁵ are independently selected from C₁₋₆-alkylsulfonyloxy optionally substituted with one or more halogen, and arylsulfonyloxy optionally substituted with one or more substituents selected from nitro, halogen. C₁₋₆-alkyl, and C₁₋₆-alkyl substituted with one or more halogen.

Also, interesting is the embodiments where A⁴ and A⁵ are both methylene, as well as the embodiments where X is -O-.

Although the configuration of the intermediate (Formula I) is generally open, it is presently -believed that one interesting configuration for the intermediate is represented by the formula II 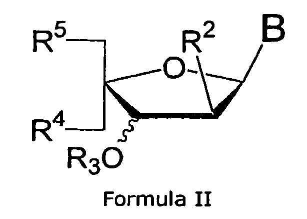 wherein B, R², R³, R⁴, and R⁵ are as defined above. This being said, the mirror-image of formula II may be equally applicable. In one embodiment OR³ and R² may form an epoxide.

In a particularly interesting embodiment, the substituents of the intermediate (Formula I or Formula II) are chosen so that B is selected from adenine, guanine, 2,6-diaminopurine, thymine, 2-thiothymine, cytosine, methyl cytosine, uracil, 5-fluorocytosine, xanthine, 6-aminopurine, 2-aminopurine, 6-chloro-2-amino-purine, and 6-chloropurine, R² is selected from C₁₋₆-alkylsulfonyloxy substituted with one or more halogen, R³ is benzyl, and R⁴ and R⁵ are Independently selected from C₁₋₆-alkylsulfonyloxy optionally substituted with one or more substituents selected from halogen and phenyl optionally substituted with one or more substituents selected from nitro, halogen and C₁₋₆-alkyl, and arylsulfonyloxy optionally substituted with one or more substituents selected from nitro, halogen, C₁₋₆-alkyl, and C₁₋₆-alkyl substituted with one or more halogen.

The substituents R⁴ and R⁵ are preferably identical in that offers advantages in the preparation of the intermediate (see further below).

Particular examples of the groups (independently) applicable as R⁴ and R⁵ are methanesulfonyloxy, trifluoromethanesulfonyloxy, ethanesulfonyloxy, 2,2,2-trifluoroethanesulfonyloxy, propanesulfonyloxy, iso-propanesulfonyloxy, butanesulfonyloxy, nonafluorobutanesulfonyloxy, pentanesulfonyloxy, cyclopentanesulfonyloxy, hexanesulfonyloxy, cyclohexanesulfonyloxy, α-toluenesulfonyloxy, 2-chloro-α-toluenesulfonyloxy, *ortho-*toluenesulfonyloxy, *meta*-toluenesulfonyloxy, *para*-toluenesulfonyloxy, benzenesulfonyloxy, *ortho*-bromobenzenesulfonyloxy, *meta*-bromobenzenesulfonyloxy, *para*-bromobenzenesulfonyloxy, *ortho*-nitrobenzenesulfonyloxy, *meta*-nitrobenzenesulfonyloxy, and *para*-nitrobenzenesulfonyloxy. The currently most promising group is methanesulfonyloxy.

In one particularly interesting variant, the intermediate has the formula III 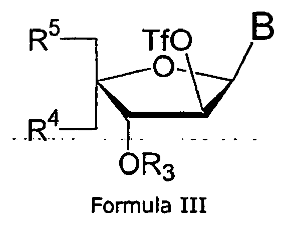 wherein B, R³, R⁴ and R⁵ are as defined above.

A further interesting variant (in combination with Formula I, Formula II or Formula III) is where the substituents are chosen so that B is selected from adenine, guanine, 2,6-diaminopurine, thymine, 2-thiothymine, cytosine, methyl cytosine, uracil, 5-fluorocytosine, xanthine, 6-aminopurine, 2-aminopurine, 6-chloro-2-amino-purine, and 6-chloropurine, R³ is benzyl, and R⁴ and R⁵ are both methanesulfonyloxy. In particular, A⁴ and A⁵ are preferably both methylene.

The intermediate of Formula I is reacted with a nucleophile selected from halogen, ⁻N₃, ⁻NR^{H}R^{H*}, ⁻SR^{H}, ⁻⁻S, ⁻NR^{H}C(O)R^{H*}, ⁻SC(O)R^{H}, and organometallic hydrocarbyl radicals, so as to substitute R².

It is currently believed that the substitution of R² proceeds via a S_{N}2 mechanism with inversion of the relative orientation of the substituent in the C2' position.

The "C2' position" refers to the normal nomenclature for nucleosides, where the carbon carrying the nucleobase B is C1', the carbon carrying R² (or R^{2*}) is C2', and the carbon carrying R⁴A⁴ is C4'.

The organometallic hydrocarbyl radicals typically has the formula MR^{H} where M is a metal such as Mg (e.g. in the form R^{H}MgBr prepared from the halide and magnesium (Grignard)), Cu (R^{H}₂CuLi e.g. prepared from 2R^{H}Li + CuI), U (e.g. BuLi)), etc.

The organometallic hydrocarbyl radicals are applicable for the preparation of LNA analogues where Z is -CH₂- (methylene-LNA). The sulphur nucleophiles are of course applicable where Z is -S-, and the nitrogen nucleophiles are applicable where Z is -NR^{H}-.

This being said, it is currently believed that particularly interesting nucleophile are those selected from ⁻N₃, ⁻NR^{H}R^{H}*, ⁻SR^{H}, ⁻⁻S, ⁻NR^{H}C(O)R^{H}*, and ⁻SC(O)R^{H}.

The conditions for the reaction of the compound of Formula I with a nucleophile is typically so that the temperature is 0-150°C, such as 20-100°C, the reaction time is typically 5 min to 24 hours, such as 2-8 hours, and the molar ratio of the nucleophile to the compound of the Formula I is typically in the range of 10:1 to 1:1, such as in the range of 5:1 to 1:1. The solvent used for the reaction is typically a polar aprotic solvent.

Examples of useful polar aprotic solvents for this reaction are tetrahydrofuran (THF), dimethylformamide (DMF), dimethylsulfoxide (DMSO), acetonitrile (AcCN), diethylether, etc.

After substitution of the group R² with the nucleophile, the (new) group in the C2' position (i.e. the nucleophile attached to the C2' position) is subjected to such conditions that ring-closure between the C2' and C4' positions is effected so as to yield the LNA analogue of the formula IV. The exact conditions for effecting ring closure will depend on the nucleophile used, or rather the (new) group in the C2' position.

The conditions for the ring-closure reaction is typically so that the temperature is 0-100°C, such as 20-50°C, and the reaction time is typically 5 min to 24 hours, such as 2 -8 hours,. The solvent used for the reaction Is typically a polar solvents.

Examples of such polar solvents are DMF, THF, acetonitrile, DMSO, C₁₋₄-alcohols and aqueous mixtures thereof.

The reagent useful for facilitating the ring-closure is typically under basic conditions using bases such as hydroxides, alkoxides, amines, deprotonated amines, etc.

In particular, in the embodiments where Z is -S-, Na₂S (of the type S⁻⁻) is a useful nucleophile that facilitates both substition and ringclosure (see preparation of **54).** The temperature is typically 0-100°C, such as 15-40°C, the reaction time is typically 5 min to 18 hours, such as 10 min to 4 hours, and the molar ratio of the nucleophile to the compound of the Formula I is typically in the range of 10:1 to 1:1, such as in the range of 2:1 to 1:1. The polar aprotic solvent is typically DMF, THF, DMSO, acetonitrile, pyridine, N-methyl pyrrolidone (NMP), hexamethylphosphoramide (HMPA), etc.

In an other embodiment potassium thioacetate (of the ⁻SC(O)R^{H} type) is a useful nucleophile. In this instance, the ring-closure can be effected under the influence of lithium hydroxide in a polar aprotic solvent (see preparation of **60**). The temperature is typically 0-100°C, such as 15-40°C, the reaction time is typically 5 min to 18 hours, such as 5 min to 2 hours, and the molar ratio of the nucleophile to the compound of the Formula I is typically in the range of 10:1 to 1:1, such as in the range of 3:1 to 1:1. The polar aprotic solvent is typically DMF, THF, DMSO, acetonitrile, pyridine, N-methyl pyrrolidone (NMP), hexamethylphosphoramide (HMPA), etc.

In the embodiment where Z is -NH-, sodium azide is a useful nucleophile. In this instance, the ring-closure is effected under the influence of sodium hydroxide and trimethylphosphane in a polar aprotic solvent. The temperature is typically 0-50°C, such as 15-30°C, the reaction time is typically 1-24 hours, such as 2-8 hours, and the molar ratio of the nucleophile to the compound of the Formula I is typically in the range of 10:1 to 1:1, such as in the range of 5:1 to 1:1. The polar aprotic solvent is typically DMF, THF, DMSO, acetonitrile, pyridine, N-methyl pyrrolidone (NMP), hexamethylphosphoramide (HMPA), etc.

When the resulting LNA analogues is one where Z is -NH-, the inventors have found that it is possible to convert the LNA analogues into another LNA analogue where the nitrogen is alkylated by reaction with an alkanal. Thus, the method may in this instance (Z = -NH-) further comprises the step of converting the LNA analogue wherein Z is -NH- to an LNA analogues where Z is -N(C₁₋₆-alkyl)- or N(aryl) by reacting a-solution of the former LNA analogue with a reducing agent and a C₁₋₆-alkanal or an aromatic aldehyde or where Z is N(acyl) by reacting with an acid chloride or an acid anhydride. Preferably where the aldehyd is formaldehyde, benzaldehyde, pyrene-1-carbaldehyde, or phthalimidoacetaldehyde and the reducing agent is NaBCNH₃, or wherein the acid chloride is benzoyl chloride or pyren-1-ylcarbonyl chloride (see Figure 7). The method of the invention relates not only to the compounds of Formula IV but equally to amino-LNA analogues in general.
Amino-LNA analogues are particularly interesting compounds of the invention. For example, 9-mers oligonucleotides mixed sequence containing two or three of the novel modified 2'-amino-LNA monomers **45-49** (see Figure 7) hybridize efficiently and in general with very high thermal stabilities comparable with those obtained for the LNA or *N*-methyl 2'-amino-LNA references (ΔTₘ/°C in a thermal denaturation assay towards complementary RNA compound calculated per monomer: **45**=+9.1, **46**=+7.3, **47**=+6.5, **48**=+3 and **49**=+7). Also, a (almost) fully modified *N*-benzoyl 2'-amino-LNA 9-mers oligonucleotides shows remarkably efficient binding towards DNA and RNA complements (Tₘ /°C 75 and 73, ΔTₘ/°C +6.3 and +6.1).

The triflate for Formula III is particularly useful as an intermediate for a wide range of LNA analogues by reaction with appropriate nucleophiles. As an example, the triflate **31** (see Figure 9) is used in the synthesis of thio-LNA (2-oxo-5-thiobicyclo[2.2.1]heptane skeleton) accomplished by a substitution reaction with the nucleophile potassium thioacetate in DMF producing compound **59**. Ring-closure of the thio-LNA nucleoside was achieved by hydrolysis of the thioacetate with aq. LiOH in THF to produce **60** in quantitative yield. The structure of **60** was confirmed by NOE experiments showing an unusually high NOE effect between H6 of the nucleobase and H3' (9.0%) as expected due to the extreme north conformation adopted by the nucleoside. Similarly, reaction of the triflate **31** with the nucleophile sodium azide in DMF produced compound **32** which was subsequently ring-closed to the amino-LNA nucleoside **33** under the influence of aqueous sodium hydroxide and trimethylphosphane in THF.

In one particularly interesting embodiment of intermediates of formula I, R³ and R² together form an epoxide. Within the embodiment wherein formula I is such that R³ and R² together form an epoxide, A⁴ and A⁵ independently are selected from C₁₋₆-alkylene; and R⁵ is C₁₋₆-alkylsulfonyloxy optionally substituted with one or more substituents selected from halogen and phenyl optionally substituted with one or more substituents selected from nitro, halogen and C₁₋₆-alkyl, and arylsulfonyloxy optionally substituted with one or more substituents selected from nitro, halogen, C₁₋₆-alkyl, and C₁₋₆-alkyl substituted with one or more halogen; such as compound **66** in figure 10.

### Synthesis-of α-L-LNA analogues

The present invention also relates to a method for the synthesis an α-L-LNA analogue e.g. α-L-oxy-LNA, α-L-thio-LNA or α-L-amino-LNA of the general formula VIII 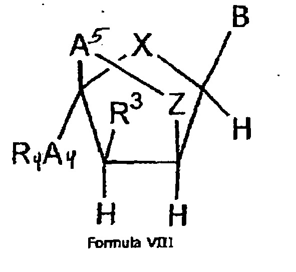 wherein
X is selected from -CH₂-, -NR^{H}-. -O-, and -S-;
Z is selected from -CH₂-, -NR^{H}-, -O-, -S-, and -Se-:
B is a nucleobase ;
R³ is selected from -R^{H}, -N₃, -NR^{H}R^{H}, -NR^{H}(O)R^{H*}, -C(O)NR^{H}R^{H*}, -OR^{H}, OC(O)R^{H}, -C(O)OR^{H}, -SR^{H}, -SC(O)R^{H}, and tri(C₁₋₆-alkyl/aryl)silyloxy;
each R^{H} and R^{H*} independently being selected from hydrogen, optionally substituted C₁₋₆-alkyl, optionally substituted aryl, and optionally substituted aryl-C₁₋₆-alkyl; A⁴ and A⁵ Independently are selected from C₁₋₆-alkylene; and
R⁴ is selected from iodo, bromo, chloro, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen;
said method comprising the following steps:
treating an intermediate of the general formula IX: 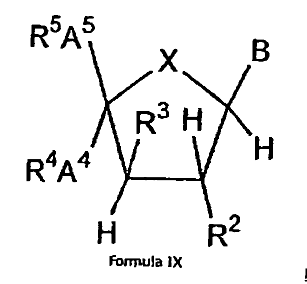
wherein
X, B, R³, A⁴, and A⁵ are as defined above;
R² is selected from iodo, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen;
R³ and R⁴ may together form an epoxide ; and
R⁴ and R⁵ independently are as defined for R⁴ above;
with a nucleophile selected from halogen, ⁻N₃, ⁻NR^{H}R^{H*},⁻OR^{H}, ⁻OH, ⁻SR^{H}, ⁻⁻S, ⁻SeR^{H}, ⁻⁻Se, ⁻NR^{H}C(O)R^{H*}, ⁻SC(O)R^{H}, and organometallic: hydrocarbyl radicals,
so as to substitute R², and
effecting ring-closure between the C2' and C4' positions so as to yield the LNA analogue of the formula VIII.

The interesting embodiment of intermediates of formula I wherein R2 and R3 together forming an epoxide is particularly interesting in the synthesis of α-L-oxy-LNA. α-L-thio-LNA or α-L-amino-LNA using a compound of Formula IX, discussed Infra.

In a further particularly interesting embodiment, the intermediate of formula IX has the formula X 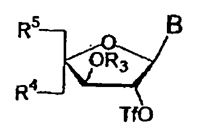 wherein B, R³, R⁴ and R⁵ are as defined above.

The intermediate of Formula IX is reacted with a nucleophile selected from halogen, ⁻N₃, ⁻NR^{H}R^{H*}, ⁻OR^{H}, ⁻OH, ⁻SR^{H}, ⁻⁻S, ⁻NR^{H}C(O)R^{H*}, ⁻SC(O)R^{H}, and organometallic hydrocarbyl radicals, so as to substitute R².

One particular advantage of using the common intermediate, X, in this invention in the reaction with hydroxide or an alkoxide such as 3-hydroxylpropionitrile alkoxide as the nucleophile is that the α-L-structure is made in one-pot. Thus, substitution of the triflate by hydroxide or 3-hydroxylpropionitrile alkoxide produces an alcohol that is immediately cyclised.

Embodiments relating to the synthesis of LNA analogues described supra are also applicable to the synthesis of α-L-LNA analogues.

### The novel intermediates

It is believed that the majority of the Intermediates (compounds of Formula I) represent novel compounds, thus the present Invention also provides compounds of the formula I 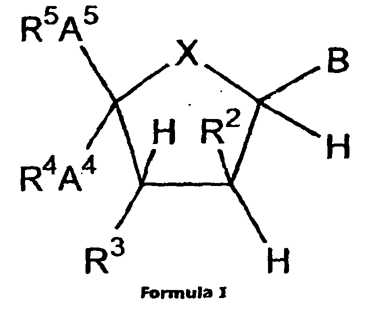 wherein
X is selected from -CH₂-, -NR^{H}-, -O-, and -S-;
B is a nucleobase;
R² is selected from iodo, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen;
R³ is selected from -R^{H}, -N₃, -NR^{H}R^{H*}, -NR^{H}C(O)R^{H*}, -C(O)NR^{H}R^{H*}, -OR^{H}, -OC(O)R^{H}, -C(O)OR^{H}, -SR^{H}, -SC(O)R^{H}, and tri(C₁₋₆-alkyl/aryl)silyloxy;
R³ and R² may together form an epoxide;
each R^{H} and R^{H}* independently being selected from hydrogen, optionally substituted C₁₋₆-alkyl, optionally substituted aryl, and optionally substituted aryl-C₁₋₆-alkyl;
A⁴ and A⁵ independently are selected from C₁₋₆-alkylene; and
R⁴ and R⁵ independently are selected from iodo, bromo, chloro, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen, or R⁴ and R⁵ together constitutes a tetra(C₁₋₆-alkyl)disiloxanylidene group;
with the proviso that the compound is not selected from
1-(3-azido-3-deoxy-2,5-di-*O*-methanesulfonyl-4-*C*-(methansulfonyloxymethyl)-β-D-*erythro*-pentofuranosyl)thymine, and
1-(3-*O*-benzyl-2,5-di-*O*-methanesufonyl-4-*C*-(methansulfonyloxymethyl)-β-D-*erythro-*pentofuranosyl)thymine.

Particular and preferred subgroups of the compounds of formula I are as described above for the compound I under Synthesis of LNA analogues. In particular, particular subclasses of compounds have the formula II, in particular and the formula III.

Examples of particularly interesting specific compounds are those illustrated in Figure 13.

It is presently believed that a particularly interesting compound which is particularly useful for the preparation of (1*R*,3*R*,4*R*,7*S*)-7-Benzyloxy-1-methansulfonyloxymethyl-3-(thymin-1-yl)-2-oxa-5-azabtcyclo[2:2:1]heptane **(33)** and (1*R*,3*R*,4*R*,7*S*)-7-Benzyloxy-1-methansulfonyloxymethyl-3-(thymin-1-yl)-2-oxa-5-thiabicyclo[2:2:1]heptane **(60)** is 1-(3-*O*-Benzyl-5-*O*-methanesutfonyl-4-*C*-methanesutfonyloxymethyl-2-*O-*trifluoromethanesulfonyl-β-D-threo-pentofuranosyl)thymine **(31)** (see Figure 9).

Particular and preferred subgroups of the compounds of formula I are described above under Synthesis of α-L-LNA analogues. In particular, a particular subclass of compounds has the formula IX and particularly formula X, and wherein R² and R³ together form an epoxide.

### Preparation of the novel intermediates

The compounds (intermediates) of the formula I can be prepared by Inversion of the orientation of the C2' substituent in a similar compound in which the C2' substituent is a leaving group. Thus, a compound of the formula I 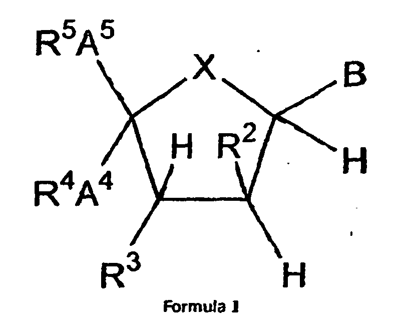 wherein
X is selected from -CH₂-, -NR^{H}-, -O-, and -S-;
B is a nucleobase;
R² is selected from iodo, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen;
R³ is selected from -R^{H}, -N₃, -NR^{H}R^{H*}, -NR^{H}C(O)R^{H*}, -C(O)NR^{H}R^{H*}, -OR^{H}, -OC(O)R^{H}, -C(O)OR^{H}, -SR^{H}, -SC(O)R^{H}, and tri(C₁₋₆-alkyl/aryl)silyloxy;
each R^{H} and R^{H*} independently being selected from hydrogen, optionally substituted C₁₋₆-alkyl, optionally substituted aryl, and optionally substituted aryl-C₁₋₆-alkyl;
A⁴ and A⁵ independently are selected from C₁₋₆-alkylene; R³ and R² may together form an epoxide and R⁴ and R⁵ independently are selected from iodo, bromo, chloro, C₁₋₃-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen,
may be prepared by inversion of orientation of the substituent in the C2' position of a compound of the formula VII 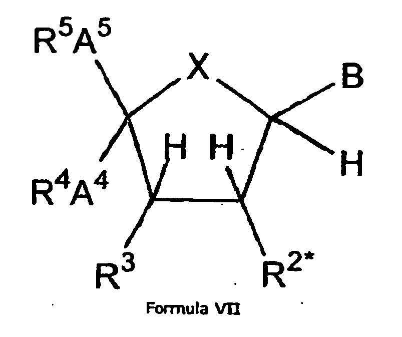 wherein
R^{2*} is a leaving group selected from lodo, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen; and
X, B, R³, R⁴, A⁴, R⁵ and A⁵ are as defined above.

Particular examples of R^{2*} groups are iodo, methanesulfonyloxy, trifluoromethanesulfonyloxy, ethanesulfonyloxy, 2,2,2-trifluoroethanesulfonyloxy, propanesulfonyloxy, isopropanesulfonyloxy, butanesulfonyloxy, nonafluorobutanesulfonyloxy, pentanesulfonyloxy, cyclopentanesuffonyloxy, hexanesulfonyloxy, cyclohexanesulfonyloxy, α-toluenesulfonyloxy, 2-chloro-α-toluenesulfonyloxy, *ortho*-toluenesulfonyloxy, *meta*-toluenesulfonyloxy, *para*-toluenesulfonyloxy, benzenesulfonyloxy, *ortho*-bromobenzenesulfonyloxy, *meta-*bromobenzenesulfonyloxy, *para*-bromobenzenesulfonyloxy, *ortho*-nitrobenzenesulfonyloxy, *meta*-nitrobenzenesulfonyloxy, and *para*-nitrobenzenesulfonyloxy, of which trifluoromethylsulfonyloxy is a particularly preferred example.

Particular and preferred subgroups of the compounds of formula VII corresponds to those described above for the compound I under Synthesis of LNA analogues, *mutatis mutantis.* In particular, particular subclasses of compounds have the configuration corresponding to formula II, especially the configuration corresponding to formula III, except for the orientation of the substituent on C2'.

In a particularly Interesting embodiment of compounds of formula I, R³ and R² together form an epoxide.

The novel compound illustrated In Formula I can be prepared by the general route shown in Figure 2.

The inversion of the orientation of the substituent on C2' can effect in various ways. If the nucleobase is a pyrimidine base, the inversion can be facilitated by formation of a 2,2'-anhydro intermediate under suitable conditions, e.g. use of a proton sponge e.g. DBU. The temperature is typically 0-100°C, such as 15-30°C, the reaction time is typically 5 min to 24 hours, such as 1-6 hours, and the molar ratio of the base to the compound of the Formula VII is typically In the range of 5:1 to 1:1, such as in the range of 3:1 to 1:1. The polar aprotic solvent is typically DMF, THF, DMSO, or CH₃CN.

Although the above-mentioned method for the synthesis of the compound of Formula I takes advantage of the 2,2'-anhydronucleoside construct, and therefore only is applicable for nucleobases (such as pyrimidines) in which such a construct is possible, it should be understood that other routes will be similarly applicable for the inversion of orientation of the substituent in the C2' position of a compound of the formula VII.

As an example, which is generally applicable for all nucleobases, and very useful in the instances where the nucleobase is a purine type nucleobase, the inversion is effected by reaction of the compound of the formula VII with an oxygen nucleophile.

A more specific example of the convergent synthesis strategy for the synthesis of an intermediate having a purine-type nucleobase is illustrated in Figure 3. Compound **13** is base protected **(14)** after which the 2'-OAc is hydrolysed selective as described elsewhere herein **(15)**. The liberated 2'-OH is triflated **(16)** and reacted with a suitable oxygen nucleophile (e.g. an acetate, benzoate, etc.) to invert the stereochemistry **(17).** The resulting ester is then selective hydrolysed as described elsewhere herein and the 2'-OH now in the *threo* configuration **(18).** Compound **18** is a purine equivalent to compound **30** which can subsequently be converted to 2'-O-mesylate, i.e. an intermediate of the formula I, following the route illustrated in Figure 6.

As a further alternative, inversion can also be effected by oxidation of a compound of Formula VII where R²* is OH, followed by subsequent stereo- and regioselective reduction, e.g. as outlined in Figure 4.

The starting materials of Formula VII for the method according to the invention may be prepared as described in the literature (Koshkin, A.; Fensholdt, J.; Pfundheller, H.M.; Lomholt, C. J.Org. Chem. 2001. 66, 8504-8512).

As a more specific example, the preferred general intermediate shown in formula III can be prepared as shown below (Figure 5). Thus, (2-*O*-acetyl-3-*O*-benzyl-4-*C-*methanesulfonyl oxymethyl- 5-*O*-methanesulfonyL-β-D-erythro-pentofuranosyl)-nucleobase) **(23)** is converted by a mild deacetylation for the liberation of the 2'-hydroxy group to the compound **(24)** without the subsequent ringclosure that affords the oxy-LNA skeleton. The 2'-hydroxy group is then mesylated to afford (3-*O*-benzyl-4-*C*-methanesulfonyloxymethyl- 2,5-*O*-dimethanesulfonyl-β-D-*erythro*-pentofuranosyl)-nucleobase) **(25).**

### Definitions

In the present context, the term "C₁₋₆-alkyl" means a linear, cyclic or branched hydrocarbon group having 1 to 6 carbon atoms, such as methyl, ethyl, propyl, *iso*-propyl, butyl, *tert*-butyl, *iso*-butyl, pentyl, cyclopentyl, hexyl, cyclohexyl, in particular methyl, ethyl, propyl, *iso*-propyl, *tert*-butyl, *iso-*butyl and cyclohexyl.

The term "C₁₋₆-alkylene" is intended to mean a linear hydrocarbon biradical having 1-6 carbon atoms, such as methylene, 1,2-ethylene, 1,3-propylene, 1,2-propylene, 1,4-butylene, etc.

The term "optionally substituted" in connection with the terms "C₁₋₆-alkyl" and "C₁₋₆-alkylene" Is intended to mean that the group in question may be substituted one or several times, preferably 1-3 times, with group(s) selected from hydroxy C₁₋₆-alkoxy (i.e. C₁₋₆-alkyl-oxy), carboxy, C₁₋₆-alkoxycarbonyl, C₁₋₆-alkylcarbonyl, aryl, aryloxycarbonyl, aryloxy, arylcarbonyl, amino, mono- and di(C₁₋₆-alkyl)amino; carbamoyl, mono- and di(C₁₋₆-alkyl)aminocarbonyl, C₁₋₆-alkylcarbonylamino, cyano, carbamido, halogen, where any aryl may be substituted as specifically describe below for "optionally substituted aryl".

In the present context the term "aryl" means a.fully or partially aromatic carbocyclic ring or ring system, such as phenyl, naphthyl, 1,2,3,4-tetrahydronaphthyl, anthracyl, and phenanthracyl, among which phenyl is a preferred example.

The term "optionally substituted" in connection with the term "aryl" is intended to mean that the group in question may be substituted one or several times, in particular 1-3 times) with group(s) selected from hydroxy, C₁₋₆-alkyl, C₁₋₆-alkoxy, carboxy, C₁₋₆-alkoxycarbonyl, C₁₋₆-alkylcarbonyl, aryl, amino, mono- and di(C₁₋₆-alkyl)amino, and halogen, wherein aryl may be substituted 1-3 times with C₁₋₄-alkyl, C₁₋₄-alkoxy, nitro, cyano, amino or halogen.

In the present context, the term "tri(C₁₋₆-alkyl/aryl)silyloxy" means a silyl group substituted with 0-3 C₁₋₆-alkyl groups and/or 0-3 aryl groups, with the provision that the total number of alkyl and aryl groups is 3. Illustrative examples are trimethylsilyloxy, allyldimethylsilyloxy, dimethylphenylsilyloxy, diphenylmethylsilyloxy, isopropyldimethylsilyloxy, *tert*-butyidimethylsilyloxy, *tert*-butyldiphenylsilyloxy, triethylsilyloxy, triisopropylsilyloxy, diethylisopropylsilyloxy, dimethylthexylisopropylsilyloxy, tribenzylsilyloxy, tri-*para*-xylylsilyloxy, triphenylsilyloxy, diphenylmethylsilyloxy, di-*tert*-butylmethylsilyloxy, tris(trimethylsilyloxy)silyloxy, *tert-*butylmethoxyphenylsilyloxy, and *tert*-butoxydiphenylsilyloxy.

In the present context, the term "tetra(C₁₋₆-alkyl)disiloxanylidene" means a -O-Si(C₁₋₆-alkyl)₂-O- Si(C₁₋₆-alkyl)₂-O- biradical. A typical example is 1,3-(1,1,3,3-tetraisopropyl)-disiloxanylidene.

"Halogen" includes fluoro, chloro, bromo, and iodo.

In the present context, the terms "nucleobase" covers naturally occurring nucleobases as well as non-naturally occurring nucleobases, i.e. heteroaromatic cyclic groups, e.g. monocyclic groups, bicyclic groups, tricyclic groups, etc. It should be clear to the person skilled in the art that various nucleobases which previously have been considered "non-naturally occurring" have subsequently been found in nature. Thus, "nucleobase" includes not only the known purine and pyrimidine heterocycles, but also heterocyclic analogues and tautomers thereof. Illustrative examples of nucleobases are adenine, guanine, thymine, cytosine, uracil, purine, xanthine, diaminopurine, 8-oxo-N⁶-methyladenine, 7-deazaxanthine, 7-deazaguanine, N⁴,N⁴-ethanocytosin, N⁶,N⁶-ethano-2,6-diaminopurine, 5-methylcytosine, 5-(C³-C⁶)-alkynylcytosine, 5-fluorouracil, 5-bromouracil, pseudoisocytosine, 2-hydroxy-5-methyl-4-triazolopyridin, isocytosine, isoguanin, inosine, N⁶-allylpurines, N⁶-acylpurines, N⁶-benzylpurine, N⁶-halopurine, N⁶-vinylpurine, N⁶-acetylenic purine, N⁶-acyl purine, N⁶-hydroxyalkyl purine, N⁶-thioalkyl purine, N²-alkylpurines, N⁴-alkylpyrimidines, N⁴-acylpyrimidines, N⁴-benzylpurine, N⁴-halopyrimidines, N⁴-vinylpyrimidines, N⁴-acetylenic pyrimidines, N⁴-acyl pyrimidines, N⁴-hydroxyalkyl pyrimidines, N⁶ -thioalkyl pyrimidines, 6-azapyrimidine, including 6-azacytosine, 2- and/or 4- mercaptopyrimidine, uracil, C⁵-alkylpyrimidines, C⁵-benzylpyrimidines, C⁵-halopyrimidines, C⁵-vinylpyrimidine, C⁵-acetylenic pyrimidine, C⁵-acyl pyrimidine, C⁵-hydroxyalkyl purine, C⁵-amidopyrimidine, C⁵-cyanopyrimidine, C⁵-nitropyrimidine, C⁵-aminopyrimdine, N²-alkylpurines, N²-alkyl-6-thiopurines, 5-azacytidinyl, 5-azauracilyl, trazolopyridinyl, imidazolopyridinyl, pyrrolopyrimidinyl, and pyrazolopyrimidinyl. Functional oxygen and nitrogen groups on the base can be protected and deprotected if necessary or desirable. Suitable protecting groups are well known to those skilled in the art, and included trimethylsilyl, dimethylhexylsilyl, *t*-butyldimethylsilyl, and *t*-butyldiphenylsilyl, trityl, alkyl groups, acyl groups such as acetyl and propionyl, methanesulfonyl, and p-toluenesulfonyl. Preferred bases include adenine, guanine, 2,6-diaminopurine, thymine, 2-thiothymine, cytosine, methyl cytosine, uracil, 5-fluorocytosine, xanthine, 6-aminopurine, 2-aminopurine, 6-chloro-2-amino-purine, and 6-chloropurine. Especially interesting nucleobases are adenine, guanine, thymine, cytosine, and uracil, which are considered as the naturally occurring nucleobases in relation to therapeutic and diagnostic application in humans.

### EXAMPLES

For reactions conducted under anhydrous conditions glassware was dried overnight in an oven at 150 °C and was allowed to cool in a dessicator over anhydrous KOH. Anhydrous reactions were carried out under an atmosphere of argon. Solvents were HPLC grade, of which DMF, pyridine, acetonitrile and dichloromethane were dried over molecular sieves (4 Å from Grace Davison) and THF was freshly destilled from Na-benzophenone to a water content below 20 ppm. TLC was run on Merck silica 60 F₂₅₄ aluminum sheets. Dry Column Vacuum Chromatography (DCVC) was performed according to the published procedure. ¹H, ¹³C, ¹⁹F, and ³¹P NMR spectra were recorded at respectively 400 MHz, 100 MHz, 376 MHz, and 121 MHz with solvents as internal standard (δ_{H}: CDCl₃ 7.26 ppm, DMSO-d₆ 2.50; δ_{C}: CDCl₃ 77.0 ppm, DMSO-d₆ 39.4 ppm). ³¹P NMR was run with 85% H₃PO₄ as external standard. J values are given in Hz. Assignments of NMR spectra are based on 2D spectra and follow the standard carbohydrate/nucleoside nomenclature (the carbon atom of the 4'-C-substitiuent is numbered C1") even though the systematic compound names of the bicyclic nucleoside derivatives are given according to the von Baeyer nomenclature. Crude compounds were used without further purification if they were ≥95% pure by TLC and HPLC-MS (RP C18 column, UV detection). Elemental analyses were obtained from the University of Copenhagen, Microanalytical Department.

**1-(2,5-Di-*O*-acetyl-4-*C*-acetyloxymethyl-3-*O*-benzyl-β-D-*erythro*pentofuranosyl)thymine.** To a stirred solution of 3-*O*-benzyl-4-*C*-hydroxymethyl-1,2-*O-*isopropylidene-α-D-*erythro*-pentofuranose **1** (Youssefyeh, R. D.; Verheyden, J. P. H.; Moffatt, J. G. *J.Org.Chem.* **1979,** *44*, 1301-1309). (200 mg, 0.64 mmol) in acetic acid (3.69 mL, 64.4 mmol) at 0 °C was added acetic anhydride (0.61 mL, 6.44 mmol) and concd H₂SO₄ (0.34 µL, 6.44 µmol). After 25 min the reaction mixture was allowed to warm to rt. Stirring was continued for 2 h after which the mixture was poured into ice cooled sat. aq NaHCO₃ (150 mL). The solution was extracted with dichloromethane (2 × 150 mL), and the combined organic phases were washed with sat. aq NaHCO₃ (2 × 100 mL), dried (Na₂SO₄), filtered and evaporated to dryness in vacuo to give the crude anomeric mixture of the acetylated glycoside donor as a colorless liquid (258 mg, 0.59 mmol). The liquid (246 mg, 0.56 mmol) was dissolved in anhyd acetonitrile (5 mL) with stirring. Thymine (144 mg, 1.14 mmol) and *N,O*-bis(trimethylsilyl)acetamide (0.99 mL, 4.00 mmol) were added, and the mixture was heated to reflux for 1.5 h and then cooled to 0°C. Trimethylsilyl triflate (0.23 mL, 1.25 mmol) was added dropwise during 5 min and the mixture was heated to 80 °C for 3.5 h. The reaction mixture was allowed to cool to rt, and ice cooled sat. aq NaHCO₃ (10 mL) was added. Extraction was performed with dichloromethane (2 × 20 mL), and the combined organic phases were washed successively with sat. aq NaHCO₃ (2 × 20 mL) and brine (20 mL), dried (Na₂SO₄), filtered and evaporated to dryness in vacuo. The residue was purified by DCVC (0-1% MeOH in dichloromethane v/v) to give the nucleoside (259 mg, 91%) as a white solid material. FAB-MS *m*/*z* found 505.0 ([MH]⁺, calcd 505.2); ¹H NMR (CDCl₃) δ9.93 (s, 1H, NH), 7.37-7.28 (m, 5H, Ph), 7.09 (d, *J* = 0.9, 1H, H6), 5.79 (d, *J* = 3.5, 1H, H1'), 5.53 (dd, *J* = 6.3, 3.7, 1H, H2'), 4.64-4.08 (m, 7H, CH₂Ph, H3', H5'a, H5'b, H1"a, H1"b), 2.11 (s, 3H, CH₃C(O)), 2.10 (s, 3H, CH₃C(O)), 2.07 (s, 3H, CH₃C(O)), 1.91 (s, 3H, CH₃); ¹³C NMR (CDCl₃) δ170.4, 169.9, 163.9, 149.9 (CH₃C(O), C2, C4), 137.1, 136.8, 128.3, 128.0, 127.8 (C6, Ph), 111.0 (C5), 90.6 (C1'), 84.2 (C4'), 77.0 (C3'), 74.2 (CH₂Ph), 73.7 (C2'), 63.6, 62.2 (C5', C1"), 20.6, 20.5 (CH₃C(O)), 12.3 (CH₃).

**1-(3-*O*-Benzyl-4-*C*-hydroxymethyl-β-D-*erythro*-pentofuranosyl)thymine.** Nucleoside 1-(2,5-Di-*O*-acetyl-4-*C*-acetyloxymethyl-3-*O*-benzyl-*β*-D-erythropentofuranosyl)thymine (149 mg, 0.30 mmol) was dissolved in a sat. solution of NH₃ in MeOH (15 mL). The mixture was stirred overnight at rt in a sealed flask and evaporated to dryness under reduced pressure. The residue was dissolved in EtOAc (30 mL) and washed with water (10 mL). The aq phase was extracted with EtOAc (30 mL) and the combined organic phases were coevaporated to dryness with acetonitrile (2 × 10 mL) under reduced pressure. The residue was purified by DCVC (1-4% MeOH in dichloromethane v/v), affording the nucleoside (93 mg, 84%) as a viscous liquid. R_{f} = 0.32 (10% MeOH In EtOAc, v/v); FAB-MS *m*/*z* found 379.0 ([MH]⁺, calcd 379.1); ¹H NMR (DMSO-d₆) *δ* 11.29 (br s, 1H, NH), 7.73 (d, *J* = 1.3, 1H, H6), 7.40-7.26 (m, 5H, Ph), 5.90 (d, *J* = 6.2, 1H, H1'), 5.51 (d, *J* = 7.5,-1H, OH), 5.18 (t, *J* = 5.0, 1H, OH), 4.86 (t, *J* = 5.49, 1H, OH), 4.81 (d, *J* = 11.7, 1H), 4.56 (d, *J* = 11.7, 1H), 4.36 (q, *J* = 6.3, 1H, H2'), 4.08 (d, *J* = 5.5, 1H, H3'), 3.60-3.50 (m, 4H) (H5', H1", CH₂Ph), 1.79 (d, *J* = 1.1, 3H, CH₃); ¹³C NMR (DM5O-d₆) δ 163.6 (C4), 150.7 (C2), 138.6, 136.3, 128.0, 127.2 (C6, Ph), 109.3 (C5), 87.7, 87.5 (C1', C4'), 78.5 (C3'), 73.3 (C2'), 72.7, 62.8, 61.3 (C5', C1", CH₂Ph), 12.2 (CH₃); Anal. calcd for C₁₈H₂₂N₂O₇·0.25 H₂O: C, 56.5; H, 5.9; N, 7.3. Found: C, 56.5; H, 5.9; N, 7.0.

**1-(3-*O*-Benzyl-2,5-di-*O*-methanesulfonyl-4-*C*-(methanesulfonyloxymethyl)- β-D-*erythro-*pentofuranosyl)thymine (28).** Nucleoside 1-(3-*O*-Benzyl-4-*C*-hydroxymethyl-β-D-*erythro*-pentofuranosyl)thymine (0.83 g, 3.2 mmol) was dissolved in anhyd pyridine (20 mL) and cooled to 0 °C with stirring. Methanesulfonyl chloride (0.85 mL, 11 mmol) was added dropwise and the reaction was allowed to reach 15°C over 3 h. The reaction was quenched with sat. aq NaHCO₃ (50 mL) and transferred to a separatory funnel with brine (50 mL) and EtOAc (100 mL). The phases were separated and the aq phase extracted with EtOAc (2 × 50 mL). The combined organic phases were extracted with brine (100 mL), dried (Na₂SO₄), filtered and evaporated in vacuo to give a viscous yellow liquid. The liquid was dissolved in a mixture of dichloromethane and toluene and evaporated in vacuo to give nucleoside **28** (1.48 g, 93%) as a white foam. Analytical data were identical to those previously published. (Håkansson, A. E.; Koshkin, A.; Sørensen, M. D.; Wengel, J. J.Org.Chem. 2000, 65, 5161-5166.)

**1-(3-*O*-Benzyl-5-*O*-methanesulfonyl-4-*C*-methanesulfonyloxymethyl-β-D-*erythro*pentofuranosyl)thymine (27).** Nucleoside **26** (Koshkin et al., J. Org. Chem. 2001, 66, 8504-8512) (30 g, 52 mmol) was dissolved in MeOH (600 mL), and the solution was cooled to 0 °C. Freshly prepared sat. methanolic ammonia (600 mL) was added, and the mixture was allowed to reach rt. After 5 h at rt the reaction was quenched with glacial acetic acid (50 mL) and transferred to a beaker, where it was neutralised with sat. aq NaHCO₃. EtOAc (900 mL) and brine (500 mL) was added and the phases were separated. The aq phase was extracted with EtOAc (3 × 500 mL) and the combined organic phases were washed with sat. aq NaHCO₃ (500 mL) and brine (500 mL). The organic phase was dried (Na₂SO₄), filtered and the solvent removed in vacuo to afford **27** (27 g, 97%) as a white foam. R_{f} = 0.33 (100% EtOAc); ESI-MS *m*/*z* found 557.0 ([MNa]⁺, calcd 557.1); ¹H NMR (CDCI₃) δ 10.21 (br s, 1H, NH), 7.33-7.25 (m, 6H, Ph, H6), 5.77 (d, J = 3.9, 1H, H1'), 4.84 (d, *J* = 11.4, 1H, H3'), 4.59-4.57 (m, 3H), 4.42-4.37 (m, 3H), 4.26-4.19 (m, 2H) (H2', H2", H5", CH₂Ph, OH), 2.98 (s, 3H, CH₃), 2.76 (s, 3H, CH₃), 1.80 (s, 3H, CH₃); ¹³C NMR (CDCI₃) δ 162.5 (C4), 151.0 (C2), 136.7 (Ph), 136.2 (C6), 128.5, 128.3, 128.2 (Ph), 111.3 (C5), 92.1 (C1'), 84.0 (C4'), 77.7 (C3'), 74.1, 73.5 (C2', CH₂Ph), 68.6, 68.3 (C5', C1"), 37.2, 37.1 (Ms), 12.0 (CH₃); Anal. calcd for C₂₀H₂₆N₂O₁₁S₂: C, 44.9; H, 4.9; N, 5.2. Found: C, 45.0; H, 4.7; N, 5.1.

**1-(3-*O*-Benzyl-2,5-di-*O*-methanesulfonyl-4-*C*-(methanesulfonyloxymethyl)-β-D-*erythro*-pentofuranosyl)thymine (28).** Nucleoside **27** (20 g, 37 mmol) was dissolved in anhyd dichloromethane (100 mL) and anhyd pyridine (100 mL) was added. The solution was cooled to 0 °C and methanesulfonyl chloride (4.4 mL, 56 mmol) was added dropwise. After 2 h the reaction was quenched with sat. aq NaHC0₃ (200 mL), and the phases were separated. The aq phase was extracted with dichloromethane (2 × 150 mL), and the combined organic phases were washed with aq HCl (1 M, 2 × 200 mL), sat. aq NaHCO₃ (2 × 250 mL) and brine (250 mL). The organic phase was dried (Na₂SO₄), filtered and the solvent was removed in vacuo. The crude product was co-evaporated with toluene affording **28** (22 g, 96%) as a white foam. R_{f} = 0.41 (100% EtOAc); ESI-MS *m*/*z* found 635.0 ([MNa]⁺, calcd 635.1). All analytical data were identical to those previously reported.(Håkansson, A. E.; Koshkin, A.; Sørensen, M. D.; Wengel, J. J.Org.Chem. 2000, 65, 5161-5166)

**2,2' -Anhydro-1-(3-*O*-benzyl-5-*O*-methanesulfonyl-4-*C-*methanesulfonyloxymethyl-β-D-*threo*-pentofuranosyl)thymine (29)**. Nucleoside **28** (10 g, 16.3 mmol) was dissolved in anhyd acetonitrile (100 mL) and DBU (2.69 mL, 18.0 mmol) was added. The product slowly precipitated from the reaction mixture. After 2 h the reaction was completed and concentrated in vacuo to facilitate precipitation. The reaction mixture was cooled to -20 °C and the product collected by filtration to afford nucleoside **29** (7.64 g, 91%) as a white solid material. FAB-MS *m*/*z* found 517.0 ([MH]⁺, calcd 517.1); ¹H NMR (DMSO-d₆) δ 7.79 (d, *J* = 1.3, 1H, H6), 7.45-7.32 (m, 5H, Ph), 6.40 (d, *J* = 6.0, 1H, H1'), 5.60 (dd, *J* = 6.1, 2.8, 1H, H2'), 4.82 (d, *J* = 11.5, 1H, CH₂Ph), 4.70 (d, *J* = 11.5, 1H, CH₂Ph), 4.51 (d, *J* = 2.8, 1H, H3'), 4.43 (d, *J* = 10.6, 1H), 4.36 (d, *J* = 6.2, 1H), 4.33 (d, *J* = 5.9, 1H), 4.25 (d, *J* = 11.0, 1H) (H5', H1"), 3.22 (s, 3H, Ms), 3.16 (s, 3H, Ms), 1.80 (s, *J* = 1.1, 3H, CH₃); ¹³C NMR (DMSO-d₆) δ 171.5 (C4), 159.1 (C2), 136.9, 132.1, 128.5, 128.1, 127.9 (C6, Ph), 117.1 (C5), 89.1 (C1'), 86.1 (C2'), 85.4 (C4'), 83.7 (C3'), 72.4 (CH₂Ph), 68.6, 68.0 (C5', C1"), 36.9, 36.8 (Ms), 13.6 (CH₃); Anal. calcd for C₂₀H₂₄N₂O₁₀S₂: C, 46.5; H, 4.7; N, 5.4. Found: C, 46.6; H, 4.8; N, 5.3.

**1-(3-*O*-Benzyl-5-*O*-methanesulfonyl-4-*C*-methanesulfonyloxymethyl-*β*-D-*threo*pentofuranosyl)thymine (30)**. Nucleoside **29** (3.70 g, 7.16 mmol) was suspended in a mixture of acetone (160 mL) and aq H₂SO₄ (0.1 M, 160 mL). The mixture was heated to reflux overnight with stirring. After cooling to rt a white solid precipitated. The volume was reduced to approx. ½ in vacuo and a white solid was isolated by filtration. The solid was washed thoroughly with water and dried in vacuo to give nucleoside **30** (3.77 g, 98%) as a white solid. FAB-MS *m*/*z* found 535.0 ([MH]⁺, calcd 535.1); ¹H NMR (DMSO-d₆) δ 11.35 (s, 1H, NH), 7.41-7.32 (m, 6H, H6, Ph), 6.20 (d, J = 5.0, 1H, H1'), 6.10 (d, *J* = 4.8, 1H, 2'-OH), 4.77 (d, *J* = 11.9, 1H, CH₂Ph), 4.67 (d, *J* = 11.9, 1H, CH₂Ph), 4.56 (d, *J* = 10.6, 1H), 4.50-4.41 (m, 3H), 4.32 (d, J = 10.6, 1H), 4.16 (d, J = 3.7, 1H, H3'), 3.25 (s, 3H, Ms), 3.20 (s, 3H, Ms), 1.79 (s, 3H, CH₃); ¹³C NMR (DMSO-d₆) δ 163.9 (C4), 150.6 (C2), 137.8, 137.6, 128.4, 127.9, 127.7 (C6, Ph), 108.2 (C5), 84.8 (C1'), 84.3 (C3'), 81.7 (C4'), 73.3 (C2'), 72.3 (CH₂Ph), 68.1, 67.6 (C5', C1"), 37.0, 36.8 (Ms), 12.2 (CH₃); Anal. calcd for C₂₀H₂₆N₂O₁₁S₂: C, 44.9; H, 4.9; N, 5.2. Found: C, 44.5; H, 4.8; N, 5.1.

**1-(3-*O*-Benzyl-5-*O*-methanesulfonyl-4-*C*-methanesulfonyloxymethyl-2-*O-*trifluoromethanesulfonyl-β-D-*threo*-pentofuranosyl)thymine (31).** Nucleoside **30** (300 mg, 0.56 mmol) was dissolved in anhyd pyridine (2 × 5 mL) and concentrated in vacuo to remove water traces. The compound was dissolved in a mixture of anhyd dichloromethane (20 mL) and anhyd pyridine (0.45 mL, 5.60 mmol) followed by the addition of DMAP (274 mg, 2.24 mmol). After cooling to 0 °C trifluoromethanesulfonic anhydride (0.19 mL, 1.12 mmol) was added dropwise during 30 min. The reaction mixture was stirred for an additional 1.5 h and poured into ice cooled sat. aq NaHCO₃ (20 mL). The organic phase was separated and washed successively with aq HCl (1 M, 2 × 20 mL) and sat. aq NaHCO₃ (2 × 20 mL), dried (Na₂SO₄), filtered and evaporated in vacuo. The residue was purified by DCVC (0-100% EtOAc in *n*-heptane v/v) yielding nucleoside **31** (302 mg, 80%) as a white foam. FAB-MS *m*/*z* found 667.0 ([MH]⁺, calcd 667.0); ¹H NMR (DMSO-d₆) δ 11.62 (br s, 1H, NH), 7.51 (s, 1H, H6), 7.40-7.33 (m, 5H, Ph), 6.45 (br s, 1H, H1'), 5.91 (t, *J* = 6.0, 1H, H2'), 4.97 (d, *J* = 5.7, 1H, H3'), 4.82-4.36 (m, 6H, CH₂Ph, H5'a, H5'b, H1"a, H1"b), 3.30 (s, 3H, Ms), 3.24 (s, 3H, Ms), 1.81 (s, 3H, CH₃); ¹³C NMR (DMSO-d₆) δ 163.3 (C4), 150.0 (C2), 136.5, 128.3, 128.0, 127.8 (C6, Ph), 117.6 (q, *J* = 320, CF₃), 110.1 (C5), 88.0 (C1'), 81.7, 81.0 (C3', C4'), 73.1 (CH₂Ph), 68.0, 67.6 (C5', C1"), 36.7, 36.6 (Ms), 11.8 (CH₃); Anal. calcd for C₂₁H₂₅F₃N₂O₁₃S₃: C, 37.8; H, 3.8; N, 4.2. Found: C, 38.1; H, 3.8; N, 4.1.

**1-(2-Azido-3-*O*-benzyl-2-deoxy-5-*O*-methanesulfonyl-4-*C-*(methanesulfonyloxymethyl)-β-D-*erythro*-pentofuranosyl)thymine (32).**
**Method A:** To a solution of nucleoside **31** (215 mg, 0.32 mmol) in anhyd DMF (10 mL) NaN₃ (23 mg, 0.35 mmol) and 15-crown-5 (64 µL, 0.32 mmol) was added. The mixture was stirred at 80 °C for 1 h and then cooled to rt whereupon water (20 mL) was added. The solution was extracted with EtOAc (50 mL) and the organic phase was washed with sat. aq NaHCO₃ (2 × 20 mL), dried (Na₂SO₄), filtered and evaporated to dryness In vacuo. The residue was purified by DCVC (50-100% EtOAc in n-heptane v/v) yielding nucleoside **32** (164 mg, 91% from **31**) as a white foam. Analytical data were identical to those reported above.
**Method B**: A solution of nucleoside **30** (5.35 g, 10 mmol) in anhyd dichloromethane (300 mL) was cooled to 0 °C. Anhyd pyridine (8.08 mL, 100 mmol) and DMAP (4.89 g, 40 mmol) was added followed by the dropwise addition of triflouromethansulfonic anhydride (3.3 mL, 20 mmol). After 2 h at 0 °C the reaction was quenched by the addition of ice cold sat. aq NaHCO₃ (200 mL) and the reaction mixture was transferred to a separatory funnel. The phases were separated and the aq phase was extracted with dichloromethane (200 mL). The combined organic phases were washed with aq HCl (1.0 M, 2 × 300 mL) and sat. aq NaHCO₃ (300 mL), dried (Na₂SO₄), filtered and concentrated in vacuo to give a white solid. The solid was dissolved in anhyd DMF (300 mL) and NaN₃ (1.86 g, 30 mmol) was added. After stirring at rt for 4 h brine (300 mL) was added and the mixture was transferred to a separatory funnel. The aq phase was extracted with dichloromethane (3 × 200 mL) and the combined organic phases were dried (Na₂SO₄), filtered and concentrated in vacuo yielding a yellow residue that was purified by DCVC (∅ 5 cm, 25-100% EtOAc In *n*-heptane v/v, 5% Increments, 100 mL fractions) affording nucleoside **32** (5.1 g, 91% from **30**) as a white solid. Analytical data were identical to those reported above.

**(1*R*,3*R*,4*R*,7*S*)-7-Benzyloxy-1-methansulfonyloxymethyl-3-(thymin-1-yl)-2-oxa-5-azabicyclo[2:2:1]heptane (33).** To a solution of **32** (5.83 g, 10.4 mmol) in THF (300 mL) at rt aq NaOH (2.0 M, 104 mL, 208 mmol) and PMe₃ in THF (1.0 M, 20.8 mL, 20.8 mmol) was added with stirring. After 8 h the THF was partly removed under reduced pressure. Brine (200 mL) and EtOAc (300 mL) was added and the phases were separated. The aq phase was extracted with EtOAc (2 × 300 mL) and dichloromethane (2 × 300 mL). The combined organic phases were dried (Na₂SO₄), filtered and concentrated in vacuo to give nucleoside **33** (4.22 g, 93%) as a white solid. R_{f} = 0.15 (10% MeOH in EtOAc, v/v); ESI-MS m/z found 438.0 ([MH]⁺, calcd 438.1); ¹H NMR (DMSO-d₆) δ 11.33 (br s, 1H, NH), 7.46 (s, 1H, H6), 7.36-7.27 (m, 5H, Ph), 5.44 (s, 1H, H1'), 4.67 (d, *J* = 11.7, 1H), 4.59 (d, *J* = 11.5, 1H), 4.56 (d, *J* = 11.9, 1H), 4.52 (d, *J* = 11.7, 1H) (H5', CH₂Ph), 3.84 (s, 1H, H3'), 3.65 (s, 1H, H2'), 3.26 (s, 3H, Ms), 3.06 (d, *J* = 10.1, 1H, H1"a), 2.78 (d, *J* = 9.9, 1H, H1"b), 1.77 (s, 3H, CH₃); ¹³C NMR (DMSO-d₆) δ 163.9 (C4), 150.1 (C2), 137.9, 134.7, 128.2, 127.7, 127.6 (C6, Ph), 108.3 (C5), 88.4 (C1'), 85.6 (C4'), 76.3 (C3'), 70.9, 66.6 (CH₂Ph, C5'), 59.4 (C2'), 50.1 (C1"), 36.9 (Ms), 12.3 (CH₃); Anal. calcd for C₁₉H₂₃N₃O₇S: C, 52.1; H, 5.3; N, 9.6. Found: C, 52.0; H, 5.2; N, 9.2.

(**1*R*,3*R*,4*R*,7*S*)-7-Benzyloxy-1-methansulfonyloxymethyl-5-methyl-3-(thymin-1-yl)-2-oxa-5-azabicyclo[2:2:1]heptane (34).** To a solution of **33** (4.22 g, 9.64 mmol) in formic acid (20 mL) formaldehyde (37% aq solution, 20 mL) was added with stirring and the reaction mixture was heated to 80 °C. After 1 h the reaction was diluted with EtOAc (150 mL) and quenched by carefully pouring it into sat. aq NaHCO₃ (100 mL). The phases were separated and the organic phase was washed with sat. aq NaHCO₃ (4 × 100 mL). The combined aq phases were extracted with dichloromethane (2 × 200 mL). The combined organic phases were dried (Na₂SO₄), filtered and concentrated under reduced pressure. Purification by DCVC (Ø 6 cm, 0-15% MeOH in EtOAc v/v, 1% increments, 100 mL fractions) afforded nucleoside **34** (3.89 g, 90%) as an off-white solid. R_{f} = 0.30 (10% MeOH in EtOAc, v/v); ESI-MS *m*/*z* found 452.1 ([MH]⁺, calcd 452.1); ¹H NMR (DMSO-d₆) δ 11.34 (br s, 1H, NH), 7.43 (s, 1H, H6), 7.34-7.28 (m, 5H, Ph), 5.58 (s, 1H, H1'), 4.67 (m, 4H, H5', CH₂Ph), 3.88 (s, 1H, H3'), 3.58 (s, 1H, H2'), 3.27 (s, 3H, Ms), 2.98 (d, *J* = 9.7, 1H, H1"a), 2.76 (d, *J* = 9.7, 1H, H1"b), 2.57 (s, 3H, NCH₃), 1.76 (s, 3H, CH₃); ¹³C NMR (DMSO-d₆) δ 163.9 (C4), 149.9 (C2), 137.6 (Ph), 134.6 (C6), 128.3, 127.7 (Ph), 108.4 (C5), 86.1 (C1'), 85.3 (C4'), 77.3 (C3'), 71.0, 66.3 (CH₂Ph, C5'), 64.9 (C2'), 58.7 (C1"), 40.8 (NCH₃), 36.9 (Ms), 12.3 (CH₃); Anal. calcd for C₂₀H₂₅N₃O₇S·0.25 H₂O: C, 52.7; H, 5.6; N, 9.1. Found: C, 52.9; H, 5.6; N, 8.9.

(**1*R*,3*R*,4*R*,7*S*)-7-Benzyloxy-1-hydroxymethyl-5-methyl-3-(thymin-1-yl)-2-oxa-5-azabicyclo[2:2:1]heptane (35).** Compound **34** (3.00 g, 6.64 mmol) was dissolved in anhyd DMF (30 mL) and sodium benzoate (1.93 g, 13.3 mmol) was added. The reaction mixture was heated to 100 °C for 7 h and then cooled to rt. Sodium methoxide (1.44 g, 26.6 mmol) was added and after 1 h the reaction was diluted with dichloromethane (100 mL) and washed with brine (2 × 100 mL). The combined aq phases were extracted with dichloromethane (2 × 50 mL). The combined organic phases were dried (Na₂SO₄) and concentrated under reduced pressure. The residue was dissolved in aq HCl (1 M, 15 mL) and lyophilised yielding an off-white solid. Purification by DCVC (∅ 4 cm, 0-10% MeOH in dichloromethane v/v, 0.5% increments, 50 mL fractions) afforded the hydrochloride salt of nucleoside **35** (2.72 g, 98%) as an off-white solid. R_{f} = 0.19 (7% MeOH in dichloromethane, v/v); ESI-MS *m*/*z* found 374.1 ([MH]⁺, calcd 374.2), 408.1, 410.1 ([MCl]⁻, calcd 408.1, 410.1); ¹H-NMR (DMSO-d₆) δ 11.43 (br s, 1H, NH), 7.63 (s, 1H, H6), 7.45-7.29 (m, 5H, Ph), 5.60 (s, 1H, H1*'*), 4.80 (t, *J* = 5.7, 1H, 5'-OH), 4.67-4.50 (m, 2H, CH₂Ph), 3.87 (s, 1H, H3'), 3.67 (d, *J* = 6.0, 2H, H5'), 3.38 (s, 1H, H2'), 2.88 (d, *J* = 9.2, 1H, H1*"*a), 2.66 (d, *J* = 9.5, 1H, H1*"*b), 2.57 (s, 3H, NCH₃), 1.75 (s, 3H, CH₃); ¹³C NMR (DMSO-d₆) δ 164.0 (C4), 149.8 (C2), 137.0 (Ph), 134.4 (C6), 128.5, 127.8 (Ph), 108.9 (C5), 88.4 (C1'), 88.0 (C4'), 77.8 (C3'), 71.0, (CH₂Ph), 66.0, 65.7 (C2', C5'), 61.4 (C1*"*), 40.1 (NCH₃), 12.6 (CH₃); Anal. calcd for C₁₉H₂₃N₃O₅·HCl·H₂O: C, 53.3; H, 6.1; N, 9.8. Found: C, 53.0; H, 6.3; N, 9.6.

**(1*R*,3*R*,4*R*,7*S*)-7-Hydroxy-7-hydroxymethyl-5-methyl-3-(thymin-l-yl)-2-oxa-5-azabicyclo[2:2:1]heptane (36).** Compound **35** (2.60 g, 6.64 mmol) was dissolved in glacial acetic acid (50 mL) and the reaction flask was evacuated and filled with argon several times. Pd(OH)₂ on charcoal (20% moist, 200 mg) was added and the reaction flask was evacuated and filled with hydrogen gas several times. The reaction was stirred vigorously under an atmosphere of hydrogen gas for 8 h. The catalyst was removed by filtration through a plug of celite. The celite was washed thoroughly with hot methanol (200 mL). The solvents were removed in vacuo. The residue was dissolved in water (10 mL) and lyophilised yielding the acetate salt of nucleoside 36 (2.10 g, 97%) as off-white flakes. R_{f} = 0.11 (0.5% Et₃N, 10% MeOH, 89.5% EtOAc, v/v/v); ESI-MS *m*/*z* found 284.1 ([MH]⁺, calcd 284.1). All analytical data were identical to those previously reported. ⁷

**(1*R*,3*R*,4*R*,7*S*)-1-(4,4'-Dimethoxytrityloxymethyl)-7-hydroxy-5-methyl-3-(thymin-1-yl)-2-oxa-5-azabicyclo[2:2:1]heptane (37).** Compound **36** (2.00 g, 5.83 mmol) was dissolved in anhyd pyridine (2 × 50 mL) and concentrated in vacuo. The nucleoside was dissolved in anhyd pyridine (50 mL) and 4,4'-dimethoxytrityl chloride (2.96 g, 8.74 mmol) was added and the reaction was stirred at rt for 9 h. The reaction was concentrated to ½ volume in vacuo and the residue was diluted with EtOAc (100 mL). The organic phase was washed with sat. aq NaHCO₃ (3 × 100 mL) and brine (100 mL), dried (Na₂SO₄), filtered and concentrated under reduced pressure. Purification by DCVC (∅ 4 cm, 0-10% MeOH in EtOAc + 0.5% TEA v/v, 0.5% increments, 50 mL fractions) afforded nucleoside **37** (3.13 g, 92%) as off-white solid. R_{f} = 0.38 (0.5% Et₃N, 10% MeOH, 89.5% EtOAc, v/v/v); ESI-MS *m*/*z* found 586.2 ([MH]⁺, calcd 586.2). All analytical data were identical to those previously reported. (Singh, S. K.; Kumar, R.; Wengel, J. J.Org.Chem. 1998, 63, 10035-10039)

**(1*R*,3*R*,4*R*,7*S*)-7-(2-Cyanoethoxy(diisopropylamino)phosphinoxy)-1-(4,4'-dimethoxytrityloxymethyl)-5-methyl-3-(thymin-1-yl)-2-oxa-5-azabicyclo[2.2.1]heptane (38).** Compound **37** (500 mg, 0.85 mmol) was dissolved in anhyd dichloromethane (4 mL) and 4,5-dicyanoimidazole in MeCN (1.0 M, 0.59 mL, 0.59 mmol) was added at ambient temperature with stirring. 2-Cyanoethyl-*N,N*,*N*'*,N*'-tetraisopropylphosphorodiamidite (0.27 mL, 0.85 mmol) was added dropwise to the reaction mixture. After 2 h the reaction was diluted with dichloromethane (10 mL) and transferred to a separatory funnel and extracted with sat. aq NaHCO₃ (2 × 15 mL) and brine (15 mL). The combined aq phases were extracted with dichloromethane (10 mL). The organic phases were pooled and dried (Na₂SO₄). After filtration the organic phase was evaporated in vacuo to give nucleoside **29** as a slightly yellow foam (660 mg, 98% yield). R_{f} = 0.56 (0.5% Et₃N, 10% MeOH, 89.5% EtOAc, v/v/v); ESI-MS *m*/*z* found 786.3 ([MH]⁺, calcd 786.4). ¹⁹P NMR (CDCl₃) δ 149.8, 149.6. (Singh, S. K.; Kumar, R.; Wengel, J. J.Org.Chem. 1998, 63, 10035-10039)

(**1*R*,3*R*,4*R*,7*S*)-1-(4,4'-Dimethoxytrityloxymethyl)-7-hydroxy-5-*N*-methyl-3-(4-*N-*benzoyl-5-methyl-cytosine-1-yl)-2-oxa-5-azabicyclo[2:2:1]heptane (43).** Compound **37** (1.5 g, 2.5 mmol) was dissolved in anhyd pyridine (25 mL). Acetic anhydride (2.4 mL, 25 mmol) was added and the reaction stirred for 24 h at ambient temperature. The reaction was quenched with water (25 mL) and extracted with EtOAc (2 × 25 ml). The combined organic phases were washed with sat. aq. NaHCO₃ (2 × 50 ml), brine (50 ml), and dried (Na₂SO₄). The organic phase was filtered and evaporated in vacuo to give compound **39** as a white foam. Residual water was removed from the crude product by evaporation from anhyd MeCN. The product was then dissolved in anhyd MeCN (50 ml) and Et₃N (3.5 mL, 25.3 mmol) was added followed by 1,2,4-triazole (1.75 g, 25 mmol). The reaction mixture was cooled on an icebath and POCl₃ (0.48 mL, 5.0 mmol) was added dropwise to give a white slurry. After 15 min the reaction mixture was allowed to reach room temperature. The resulting yellow slurry was stirred under argon at ambient temperature. After 4.5 h the reactionmixture was poured into a slurry of sat. aq NaHCO₃ (50 mL) and ice and extracted with EtOAc (3 × 25 mL). The combined organic phases were washed with brine (100 ml) and dried (Na₂SO₄). Filtration and evaporation in vacuo afforded the trialzolide **40** as a pink foam which was immidiately dissolved in anhyd MeCN (50 ml) and sat. aq NH₄OH (50 mL) was added. After stirring for 16 h solid NaCl was added until the phases separated. The aq phase was extrated with EtOAc (3 × 50 mL) and the combined organic phases dried (Na₂SO₄), filtered and evaporated to give nucleoside **41** as an off-white solid. The product was dissolved in anhyd pyridine (50 mL) and benzoyl chloride (0.87 mL, 7.5 mmol) was added. The reaction was stirred for 3 h under argon and then concentrated in vacuo. The residue was diluted with EtOAc (100 mL) and extracted with sat. aq NaHCO₃ (100 mL). The phases were separated and the aq phase extracted with EtOAc (2 × 100 ml). The combined organic phases were washed with brine (200 ml) and dried (Na₂SO₄). Filtration and evaporation of the organic phase produced a clear oil **42** that was dissolved in THF (100 mL). LiOH (aq, 1.0 M, 25 mL) was added and the reaction was stirred for 2 h. The reaction mixture was transferred to a separatory funnel with EtOAc (100 mL) and brine (100 mL) and extracted with EtOAc (2 × 100 ml). The combined organic phases were washed with brine (200 ml) and dried (Na₂SO₄). Filtration and evaporation in vacou gave a yellow foam that was purified by DCVC (∅ 4 cm, 50-100% EtOAc, *n*-heptane v/v (the column was pretreated with 1% Et₃N in heptane v/v), 5% increments, 100 mL fractions)) affording nucleoside **43** (1.12 g, 65%) as a white solid. R_{f} = 0.56 (EtOAc); ESI-MS *m*/*z* found 689.3 ([MH]⁺, calcd. 689.3); ¹H NMR (DMSO-d₆) *δ* 8.16 (s, 2H, Bz), 7.86 (s, 1H, H6), 7.61-7.44 (m, 5H, Bz, DMT), 7.36-7.24 (m, 7H, Bz, DMT), 6.92 (dd, 4H, *J* = 9.0, 2.4, DMT), 5.64 (s, 1H, H1'), 5.41 (d, *J* = 5.3, 1H, H3'), 4.14 (d, *J* = 5.3, 1H, H2*'*), 5.64 (s, 1H, H1'), 3.75 (s, 6H, OCH₃), 3.39 (d, *J* = 10.8, 1H, H5*'*), 3.28 (d, *J* = 10.8 Hz, 1H, H5'), 2.89 (d, *J* = 9.5, 1H, H1"), 2.59 (s, 3H, NCH₃), 2.58 (d, *J* = 9.2, 1H, H1*"*), 1.73 (s, 3H, CH₃); ¹³C NMR (DMSO-d₆) δ 178.2 (PhC(O)), 160.3 (C4), 158.2 (Ph), 147.0 (C2), 144.8 (Ph), 137.4 (C6), 135.4, 135.2, 132.5, 129.9, 129.3, 128.4*"* 128.0, 127.7, 126.9, 113.3 (Ph), 108.6 (C5), 88.9 (C1'), 85.7 (C4'), 85.0 (Ph), 70.5 (C3'), 67.0 (C5'), 59.6, 58.6 (C2', C1"), 55.1 (OCH₃), 40.1 (NCH₃), 14.1 (CH₃); Anal. calcd. for C₄₀H₄₀N₄O₇: C, 69.7; H, 5.9; N, 8.1. Found: C, 69.5; H, 5.9; N, 7.7.

**(1*R*,3*R*,4*R*,7*S*)-1-(4,4'-Dimethoxytrityloxymethyl)-7-(2-cyanoethoxy(diisopropylamino)phosphinoxy)-5-*N*-methyl-3-(4-*N*-benzoyl-5-methyl-cytosine-1-yl)-2-oxa-5-azabicyclo[2:2:1]heptane (44)**. Compound **43** (0.50 g, 0.73 mmol) was dissolved in anhyd dichloromethane (10 mL) and 4,5-dicyanoimidazole in MeCN (1.0 M, 0.51 mL, 0.51 mmol) was added at ambient temperature with stirring. 2-Cyanoethyl-*N,N,N',N'-*t*e*traisopropylphosphorodiamidite (0.23 mL, 0.74 mmol) was added dropwise to the reaction mixture. After 2 h the reaction was diluted with dichloromethane (20 mL) and transferred to a separatory funnel and extracted with sat. aq NaHCO₃ (2 × 30 mL) and brine (30 mL). The combined aq phases were extracted with dichloromethane (30 mL). The organic phases were pooled and dried (Na₂SO₄). After filtration the organic phase was evaporated in vacuo to give a yellow foam. Purification by DCVC (Ø 4 cm, 0-100% EtOAc, *n*-heptane, 0.5% Et₃N v/v/v (the column was pretreated with 1% Et₃N in heptane v/v), 5% increments, 50 mL fractions) afforded nucleoside **44** (0.58 g, 92%) as a white solid. R_{f} = 0.67 (20% heptane, 79.5% EtOAc, 0.5% Et₃N, v/v/v); ESI-MS *m*/*z* found 889.2 ([MH]⁺, calcd 889.4); ³¹P NMR (DMSO-d₆) δ 148.4, 147.4

**1-(3-*O*-Benzoyl-5-*O*-methanesulfonyl-4-*C*-methanesulfonyloxymethyl-β-D-*threo*pentofuranosyl)thymine (52)**. Anhydronucleoside **29** (30.00 g, 58.1 mmol) was heated to 70°C in a mixture of methanol (1000 ml) and acetone (1000 ml) until a clear solution was obtained and the solution was allowed to reach room temperature. The reaction flask was flushed with argon and Pd/C (10 wt.% Pd on carbon, 6.2 g, 5.8 mmol) was added. The mixture was stirred vigorously under an atmosphere of hydrogen gas (balloon). After 23 h the slurry was filtered through a pad of celite. The catalyst was recovered from the celite and refluxed in DMF (1000 ml) for 1 h. The hot DMF slurry was filtered through a pad of celite and the organic phases pooled and evaporated *in vacuo* to give 2,2'-anhydro-1-(3-hydroxy-5-*O*-methanesulfonyl-4-*C*-methanesulfonyloxymethyl-β-D-*threo*pentofuranosyl)thymine (50) as a yellow powder. Residual solvents were removed on a high vacuum pump overnight. The crude nucleoside **50** (23 g) was heated to 70°C in DMF (300 ml) to give a clear yellow solution that was allowed to cool to room temperature. Benzoyl chloride (81.7 g, 581 mmol, 67.4 ml) was added followed by anhydrous pyridine (70 ml). After 18 h the reaction was quenched with methanol (200 ml) and excess methanol was removed *in vacuo.* To the dark brown solution of nucleoside **51** (2,2'-anhydro-1-(3-*O*-benzoyl-5-*O*-methanesulfonyl-4-*C*-methanesulfonyloxymethyl-β-D-*threo*pentofuranosyl)thymine) aqueous H₂SO₄ (0.25 M, 400 ml) was added. The solution was heated to 80°C on an oil bath (At approx 50°C precipitation occurs. The solution becomes clear again at 80°C). After 22 h at 80°C the solution was allowed to cool down to room temperature. The reaction mixture was transferred to a separatory funnel with EtOAc (1000 ml). The organic phase was extracted with sat. aq. NaHCO₃ (2 × 1000 ml). The combined aqueous phases were extracted with EtOAc (1000 + 500 ml). The organic phases were pooled and extracted once more with sat. aq. NaHCO₃ (1000 ml), dried (Na₂SO₄), filtered and evaporated *in vacuo* to give a yellow liquid. Residual solvents were removed on a high vacuum pump overnight to give a yellow syrup. The product was purified by Dry Column Vacuum Chromatography (∅ 10 cm, 50-100% EtOAc in *n*-heptane (v/v), 100 ml fractions, 10% increments, followed by 2-24% MeOH in EtOAc (v/v), 100 ml fractions, 2% increments). Fractions containing the product were combined and evaporated *in vacuo* affording nucleoside **52** (25.1 g, 79%) as a white foam.
R_{f} = 0.54 (5% MeOH in EtOAc, v/v); ESI-MS *m*/*z* found 549.0 ([MH]⁺, calcd 549.1); ¹H NMR (DMSO-d₆) δ11.39 (br s, 1H, NH), 8.10-8.08 (m, 2H, Ph), 7.74-7.70 (m, 1H, Ph), 7.60-7.56 (m, 2H, Ph), 7.51 (d, *J* = 1.1, 1H, H6), 6.35 (d, *J* = 4.9, 1H, H1*'*), 6.32 (d, *J* = 5.3, 1H, 2*'*-OH), 5.61 (d, *J* = 4.0, 1H, H3*'*), 4.69 (d, *J* = 10.8, 1H), 4.59 (m, 1H, H2*'*), 4.55 (d, *J* = 10.8, 1H), 4.52 (d, *J* = 10.8, 1H), 4.46 (d, *J* = 10.6, 1H) (H5' and H1"), 3.28 (s, 3H, Ms), 3.23 (s, 3H, Ms), 1.81 (s, 3H, CH₃); ¹³C NMR (DMSO-d₆) δ164.5, 163.6 (C4, PhC(O)), 150.3 (C2), 137.7 (C6), 133.8, 129.6, 128.7, 128.6 (Ph), 108.1 (C5), 84.8 (C1'), 81.1 (C4'), 78.0 (C3*'*), 73.2 (C2*'*), 68.0, 67.1 (C5*'*, C1"), 36.7, 36.6 (Ms), 11.9 (CH₃); Anal. calcd for C₂₀H₂₄N₂O₁₂S₂·0.33 H₂O: C, 44.34; H, 4.65; N, 4.85. Found: C, 44.32; H, 4.58; N, 4.77.

**(1*R*,3*R*,4*R*,7*R*)-7-Benzoyloxy-1-methansulfonyloxymethyl-3-(thymin-1-yl)-2-oxa-5-thiabicyclo[2:2:1]heptane (54).** 1-(3-*O*-Benzoyl-5-*O*-methanesulfonyl-4-*C-*methanesulfonyl-oxymethyl-β-D-*threo*-pentofuranosyl)thymine **(52)** (10.00 g, 18.23 mmol) was dissolved in anhydrous dichloromethane (500 ml) and cooled to 0°C. Pyridine (15 ml) and DMAP (8.91 g, 72.9 mmol) was added followed by dropwise addition of trifluoromethanesulfonic anhydride (10.30 g, 36.5 mmol, 6.0 ml). After 1 h the reaction was quenched with sat. aq. NaHCO₃ (500 ml) and transferred to a separatory funnel. The organic phase was extracted with 1.0 M aq HCl (500 ml), sat. aq NaHCO₃ (500 ml) and brine (500 ml). The organic phase was evaporated *in vacuo* with toluene (100 ml) to give 1-(3-*O*-benzoyl-5-*O*-methanesulfonyl-4-*C*-methanesulfonyloxymethyl-2-*O-*trifluoromethanesulfonyl-β-D-*threo*-pentofuranosyl)thymine **(53)** as a yellow powder. The crude nucleoside **53** was dissolved in anhydrous DMF (250 ml) and Na₂S (1.57 g, 20.1 mmol) was added to give a dark green slurry. After 3 h the reaction was quenched with half sat. aq. NaHCO₃ (500 ml) and extracted with CH₂Cl₂ (500 + 2 × 250 ml). The combined organic phases were extracted with brine (500 ml), dried (Na₂SO₄), filtered and concentrated *in vacuo* to give a yellow liquid. Residual solvent was removed overnight on a high vacuum pump to give a yellow gum that was purified by Dry Column Vacuum Chromatography (∅ 6 cm, 50-100% EtOAc in *n*-heptane (v/v), 50 ml fractions, 10% increments, followed by 2-20% MeOH in EtOAc (v/v), 50 ml fractions, 2% increments) to give nucleoside **54** (6.15 g, 72%) as a yellow foam.
R_{f} = 0.27 (20% n-heptane in EtOAc, v/v); ESI-MS *m*/*z* found 469.0 ([MH]⁺, calcd 469.1); ¹H NMR (CDCl₃) δ 8.70 (br s, 1H, NH), 8.01-7.99 (m, 2H, Ph), 7.67 (d, *J* = 1.1, 1H, H6), 7.65-7.61 (m, 1H, Ph), 7.50-7.46 (m, 2H, Ph), 5.98 (s, 1H, H1'), 5.34 (d, *J* = 2.4, 1H, H3'), 4.66 (d, *J* = 11.7, 1H, H5'a), 4.53 (d, *J* = 11.5, 1H, H5'b), 4.12 (m (overlapping with residual EtOAc), 1H, H2'), 3.15-3.13 (m, 4H, H1"a and Ms), 3.06 (d, *J* = 10.6, 1H, H1"b), 1.98 (d, *J* = 1.1, 3H, CH₃); ¹³C NMR (CDCl₃) δ 165.2, 163.5 (C4, PhC(O)), 149.9 (C2), 134.1, 133.9, 129.8, 128.7, 128.3 (C6, Ph), 110.7 (C5), 91.1 (C1'), 86.8 (C4'), 72.6 (C3'), 65.8 (C5'), 50.5 (C2'), 37.9 (Ms), 35.1 (C1"), 12.5 (CH₃); Anal. calcd for C₁₉H₂₀N₂O₈S₂.0.33 EtOAc: C, 49.21; H, 4.72; N, 5.47. Found: C, 49.25; H, 4.64; N, 5.48.

**(1*R*,3*R*,4*R*,7*R*)-7-Benzoyloxy-1-benzoyloxymethyl-3-(thymin-1-yl)-2-oxa-5-thiabicyclo[2:2:1]heptane (55)** Nucleoside **54** (1.92 g, 4.1 mmol) was dissolved in anhydrous DMF (110 ml). Sodium benzoate (1.2 g, 8.2 mmol) was added and the mixture was heated to 100°C for 24 h. The reaction mixture was transferred to a separatory funnel with half sat. brine (200 ml) and extracted with EtOAc (3 × 100 ml). The combined organic phases were dried (Na₂SO₄), filtered and evaporated *in vacuo* to give a brown liquid. The product was put on a high vacuum pump to remove residual solvent. The resulting brown gum was purified by Dry Column Vacuum Chromatography (Ø 4 cm, 0-100% EtOAc in n-heptane (v/v), 50 ml fractions, 10% increments, followed by 2-10% MeOH in EtOAc (v/v), 50 ml fractions, 2% increments) to afford nucleoside **55** (1.64 g, 81%) as a slightly yellow foam. R_{f} = 0.57 (20% n-heptane in EtOAc, v/v); ESI-MS *m*/*z* found 495.1 ([MH]⁺, calcd 495.1); ¹H NMR (CDCl₃) δ 9.02 (br s, 1H, NH), 8.07-7.99 (m, 4H, Ph), 7.62-7.58 (m, 2H, Ph), 7.47-7.42 (m, 5H, Ph and H6), 5.95 (s, 1H, H1'), 5.46 (d, *J* = 2.2, 1H, H3'), 4.93 (d, *J* = 12.8, 1H, H5'a), 4.60 (d, *J* = 12.8, 1H, H5'b), 4.17 (d, *J* = 2.2, 1H, H2'), 3.27 (d, *J* = 10.6, 1H, H1"a), 3.16 (d, *J* = 10.6, 1H, H1"b), 1.55 (d, *J* = 1.1, 3H, CH₃); ¹³C NMR (CDCl₃) δ 165.8, 165.1, 163.7 (C4, 2 × PhC(O)), 150.0 (C2), 133.9, 133.7, 133.6, 129.8, 129.6, 129.0, 128.8, 128.6, 128.5 (C6, 2 × Ph), 110.3 (C5), 91.3 (C1'), 87.5 (C4'), 72.9 (C3'), 61.3 (C5'), 50.6 (C2'), 35.6 (C1"), 12.3 (CH₃); Anal. calcd for C₂₅H₂₂N₂O₇S: C, 60.72; H, 4.48; N, 5.66. Found: C, 60.34; H, 4.49; N, 5.35.

(1***R*,3*R*,4*R*,7*R*)-7-Hydroxy-1-hydroxymethyl-3-(thymin-1-yl)-2-oxa-5-thiabicyclo[2:2:1]heptane (56).** Nucleoside **55** (1.50 g, 3.0 mmol) was dissolved in methanol saturated with ammonia (50 ml). The reaction flask was sealed and stirred at ambient temperature for 20 h. The reaction mixture was concentrated *in vacuo* to give a yellow gum that was purified by Dry Column Vacuum Chromatography (Ø 4 cm, 0-16% MeOH in EtOAc (v/v), 1% increments, 50 ml fractions) affording nucleoside **56** (0.65 g, 76%) as clear, crystals. R_{f} = 0.31 (10% MeOH in EtOAc, v/v); ESI-MS m/z found 287.1 ([MH]⁺, calcd 287.1); ¹H NMR (DMSO-d₆) δ11.32 (br s, 1H, NH), 7.96 (d, *J* = 1.1, 1H, H6), 5.95 (s, 1H, H6), 5.70 (d, *J* = 4.2, 1H, 3'-OH), 5.62 (s, 1H, H1'), 4.49 (t, *J* = 5.3, 1H, 5'-OH), 4.20 (dd, *J* = 4.1 and 2.1, 1H, H3'), 3.77-3.67 (m, 2H, H5'), 3.42 (d, *J* = 2.0, 1H, H2'), 2.83 (d, *J* = 10.1, 1H, H1"a), 2.64 (d, *J* = 10.1, 1H, H1"b), 1.75 (d, *J* = 1.1, 3H, CH₃); ¹³C NMR (DMSO-d₆) δ 163.8 (C4), 150.0 (C2), 135.3 (C6), 107.5 (C5), 90.2, 89.6 (C1' and C4'), 69.4 (C3'), 58.0 (C5'), 52.1 (C2'), 34.6 (C1"), 12.4 (CH₃); Anal. calcd for C₁₁H₁₄N₂O₅S: C, 46.15; H, 4.93; N, 9.78. Found: C, 46.35; H, 4.91; N, 9.54.

***(1R,3R,4R,7R)-1-(*4,4'-Dimethoxytrityloxymethyl)-7-hydroxy-5-methyl-3-(thymin-1-yl)-2-oxa-5-thiabicyclo[2:2:1]heptane (57).** Nucleoside **56** (0.60 g, 2.1 mmol) was dissolved in anhydrous pyridine (10 ml). 4,4'-Dimethoxytrityl chloride (0.88 g, 2.6 mmol) was added and the reaction was stirred at ambient temperature for 3 h. The reaction mixture was transferred to a separatory funnel with water (100 ml) and extracted with EtOAc (100 + 2 × 50 ml). The combined organic phases were washed with sat. aq NaHCO₃ (100 ml), brine (100 ml) and evaporated to dryness *in vacuo* to give a viscous yellow liquid. The product was redissolved in toluene (50 ml) and concentrated *in vacuo* to give a yellow foam. The foam was dried on a high vacuum pump overnight and purified by Dry Column Vacuum Chromatography (Ø 4 cm, 10-100% EtOAc in *n*-heptane (v/v), 10% increments, 50 mL fractions) affording nucleoside **57** (1.08 g, 88%) as a white foam. R_{f} = 0.24 (20% *n*-heptane in EtOAc, v/v); ESI-MS *m*/*z* found 587.1 ([M-H]⁺, calcd 587.19); ¹H NMR (CDCl₃) δ 8.96 (br s, 1H, NH), 7.74 (d, J = 1.1, 1H, H6), 7.46-7.44 (m, 2H, Ph), 7.35-7.22 (m, 9H, Ph), 7.19-7.7.15 (m, 2H, Ph), 6.86-6.80 (m, 2H, Ph), 5.82 (s, 1H, H1'), 4.55 (dd, J = 9.3 and 2.1, 1H, H3'), 3.79 (s, 6H, OCH₃), 3.71 (d, *J* = 2.0, 1H, H2'), 3.50 (s, 2H, H5'), 2.81 (d, *J* = 10.8, 1H, H1"a), 2.77 (d, *J* = 10.8; 1H, H1*"*b), 2.69 (d, *J* = 9.2, 1H, 3'-OH), 1.42 (s, 3H, CH₃); ¹³C NMR (CDCl₃) δ 158.7(C4), 150.1 (C2), 144.1, 135.2, 135.1, 130.1, 129.1, 128.1, 128.0, 127.1, 127.0 (C6, Ph), 113.3 (Ph), 110.0 (C5), 90.2 (C(Ph)₃), 89.6 (C1'), 87.0 (C4'), 71.7 (C3'), 60.9 (C5'), 55.2 (C2'), 34.7 (C1"), 12.2 (CH₃); Anal. calcd for C₃₂H₃₂N₂O₇S·0.5 H₂O: C, 64.31; H, 5.57; N, 4.69. Found: C, 64.22; H, 5.67; N, 4.47.

**(1*R*,3*R*,4*R*,7*R*)-7-(2-Cyanoethoxy(diisopropylamino)phosphinoxy)-1-(4,4'-dimethoxytrityloxymethyl)-3-(thymin-1-yl)-2-oxa-5-thiabicyclo[2.2.1]heptane (58)**. Nucleoside **57** (0.78 g, 1.33 mmol) was dissolved in anhydrous dichloromethane (5 ml) and a 1.0 M solution of 4,5-dicyanoimidazole in acetonitrile (0.93 ml, 0.93 mmol) was added followed by dropwise addition of 2-cyanoethyl-*N,N,N;N'-*tetraisopropylphosphorodiamidite (0.44 ml, 1.33 mmol). After 2 h the reaction was transferred to a separatory funnel with dichloromethane (40 ml) and extracted with sat. aq NaHCO₃ (2 × 25 ml) and brine (25 ml). The organic phase was dried (Na₂SO₄), filtered and evaporated *in vacuo* to give nucleoside **58** (1.04 g, 99%) as a white foam. R_{f} = 0.29 and 0.37 - two diastereoisomers (20% *n*-heptane in EtOAc, v/v); ESI-MS m/z found 789.3 ([MH]⁺, calcd 789.30); ³¹P NMR (DMSO-d₆) δ 150.39, 150.26

**(1*R*,3*R*,4*R*,*7S*)-7-Benryloxy-1-methansulfonyloxymethyl-3-(thymin-1-yl)-2-oxa-5-thiabicyclo[2:2:1]heptane (60).** Nucleoside **31** (0.10 g, 0.17 mmol) was dissolved in anhyd DMF (1 mL) and potassium thioacetate (25 mg, 0.22 mmol) was added. The reaction was stirred at ambient temperature for 5 h and transferred to a separatory funnel with brine (10 mL). The aq phase was extracted with dichloromethane (3 × 10 mL) and the combined organic phases dried (Na₂SO₄), filtered and evaporated in vacuo to give a yellow liquid. The crude product **59** was dissolved in THF (2 mL) and LiOH·H₂O (35 mg in 1 mL water, 0.84 mmol) was added. After 20 min the reaction was completed and quenched by the addition of glacial acetic acid (0.5 mL). The THF was removed in vacuo and the residue dissolved in dichloromethane (10 mL) and extracted with sat. aq NaHCO₃ (2 × 10 mL). The aq phases were extracted with dichloromethane (10 mL). The combined organic phases were dried (Na₂SO₄), filtered and evaporated in vacuo to give a yellow liquid that was purified by DCVC (∅ 1 cm, 0-80% EtOAc in *n*-heptane v/v, 2.5% increments, 10 mL fractions). Fractions containing nucleoside **60** were combined and evaporated in vacuo to afford a white powder (36 mg, 47% from **31).** R_{f} = 0.38 (80% EtOAc in *n*-heptane, v/v); ESI-MS *m*/*z* found 455.0 ([MH]⁺, calcd 455.1); ¹H NMR (DMSO-d₆) δ 11.38 (br s, 1H, NH), 7.50 (d, *J* = 1.1, 1H, H6), 7.36-7.27 (m, 5H, Ph), 5.77 (s, 1H, H1′), 4.68 (d, *J* = 11.7, 1H), 4.61 (d, *J* = 11.7, 1H), 4.60 (d, *J* = 11.7, 1H), 4.56 (d, *J* = 11.5, 1H) (H5', CH₂Ph), 4.20 (d, *J* = 1.8, 1H, H3'), 4.00 (d, *J* = 2.0, 1H, H2'), 3.29 (s, 3H, Ms), 3.02 (d, *J* = 10.6, 1H, H1"a), 2.90 (d, *J* = 10.4, 1H, H1"b), 1.78 (s, 3H, CH₃); ¹³C NMR (DMSO-d₆) δ 163.9 (C4), 150.1 (C2), 137.5, 134.1, 128.3, 127.7 (C6, Ph), 108.3 (C5), 90.5 (C1'), 86.6 (C4'), 76.9 (C3'), 70.9, 66.8 (C5', CH₂Ph), 49.5 (C2'), 36.8 (Ms), 35.1 (C1"), 12.3 (CH₃); Anal. calcd for C₁₉H₂₂N₂O₇S₂·0.33EtOAc: C, 50.5; H, 5.1; N, 5.8. Found: C, 50.8; H, 5.1; N, 5.8.

**9-(3-*O*-benzyl-5-*O*-(methanesulfonyl)-4-*C*-[[(methanesulfonyl)oxy]methyl]-2-*O-*trifluormethanesulfonyl-α-L-*threo*-pentofuranosyl)6-*N*-benzoyladenine (62).** Compound **61**¹ (9.58 g, 15 mmol) was concentrated from dry acetonitrile in order to remove residual water. The residue was dissolved in dry dichloromethane (100 ml) and cooled to -30 °C while stirred under Ar. The solution was added dry pyridine (3.6 ml, 44 mmol), followed by dropwise addition of Tf₂O (3.7 ml, 22 mmol). The reaction mixture was allowed to reach 0 °C. TLC (eluent: EtOAc) shows full conversion to product (R_{f} = 0.66). The reaction was quenched by addition of sat. NaHCO₃-soln. (100 ml) and diluted with dichloromethane (100 ml). The layers were separated and the org. layer was washed with sat. NaHCO₃-soln (100 ml), brine (100 ml), dried (Na₂SO₄), filtered and the solvent removed *in vacuo* to afford an orange foam, which was purified by dry column chromatography (eluent: Heptane → EtOAc) to afford pure triflate **62** (8.53 g, 74% yield). R_{f} = 0.60 (eluent: EtOAc). ESI-MS *m*/*z* found 780.0 ([MH]⁺, calcd 780.0); ¹H-NMR (CDCl₃, 400 MHz): δ 9.05 (1H, s, N-H), 8.80 (1H, s, base), 8.21 (1H, s, base), 8.00 (2H, d, *J* = 7.3 Hz, Bz), 7.61 (1H, t, *J* = 7.3 Hz, Bz), 7.52 (2H, t, *J* = 7.3 Hz, Bz), 7.41-7.30 (5H, m, Bn), 6.56 (1H, t, *J* = 5.5 Hz, H-2'), 6.34 (1H, d, *J* = 5.5 Hz, H-1'), 4.81 (2H, d, *J* = 10.4 Hz, CH₂), 4.73 (1H, d, *J* = 5.9 Hz, H-3'), 4.65 (1H, d, *J* = 11.3 Hz, CH₂), 4.44 (1H, d, *J* = 11.3 Hz, CH₂), 4.34 (1H, d, *J* = 11.1 Hz, CH₂), 4.14 (1H, d*, J =* 11.4 Hz, CH₂), 3.05 (3H, s, OMs), 2.91 (3H, s, OMs); ¹³C-NMR (CDCl₃, 100 MHz): δ 164.34, 152.94, 151.26, 149.88, 141.55, 135.07, 133.24, 132.84, 128.98, 128.83, 128.80, 128.49, 127.70, 86.49, 85.03, 83.62, 80.33, 74.49, 67.51, 67.22, 37.76 (OMs), 37.41 (OMs);¹ Compound **61** was made according to procedure described in JACS, 124, p. 2164-2176 (2002). Triflate **62** is also described in this article, but not as an isolated product.

**(1*S*,3*R*,4*S*,7*R*)-7-benzyloxy-3-(6-*N*-b*e*nzoyladenin-9-yl)-2,5-dioxabicyclo[2.2.1]heptane (53)** Pure **62** (100 mg, 0.128 mmol) was dissolved in THF (7 ml), cooled to 0 °C and added 1 M LiOH (1.3 ml, 10 equiv.). The reaction mixture was allowed to slowly reach r.t. When LCMS confirmed full conversion of **62** to **63**, the reaction was neutralized with 1 M HCl satd. with NaCl (1.3 ml), diluted with DCM (20 ml) and brine (10 ml). Layers were separated and the aqueous layer was extracted with DCM (2 × 20 ml). Comb. organic layers were dried (Na₂SO₄), filtered and the solvent removed *in vacuo* to afford a clear oil **(63)**² (quantitative yield). R_{f} = 0.49 (Eluent: EtOAc). ESI-MS *m*/*z* found 552.2 ([MH]⁺, calcd 552.1); ¹H-NMR(CDCl₃, 400 MHz): 8.64 (1H, s, N-H), 8.44 (1H, s, Adenin), 7.95 (2H, d, *J* = 7.1 Hz, Bz), 7.50 (1H, t, *J* = 7.3 Hz, Bz), 7.40 (1H, t, *J* = 7.3 Hz, Bz), 7.07-6.79 (5H, m, OBn), 6.11 (1H, s, H-1'), 4.66 (1H, d, *J* = 11.5 Hz, CH₂), 4.61 (1H, d, *J* = 11.5 Hz, CH₂), 4.48 (1H, d, *J* = 1.8 Hz, H-2'/H-3'), 4.30 (1H, d, *J* = 11.9 Hz, CH₂), 4.12 (1H, d, *J* = 11.9 Hz, CH₂), 4.07 (1H, d, *J* = 1.8 Hz, H-3'/H-2'), 4.02 (1H, d, *J* = 8.6 Hz, CH₂), 3.94 (1H, d, *J* = 8.6 Hz, CH₂), 3.02 (3H, s, OMs); ¹³C-NMR(CDCl₃, 100 MHz): δ 165.31, 152.03, 150.45, 148.54, 141.99, 135.38, 132.90, 132.84, 128.63, 128.37, 128.26, 127.98, 127.88, 121.34, 87.90, 86.16, 79.84, 76.29, 73.45, 72.51, 67.76, 64.47, 37.48 (OMs). ²Compound **63** is also described in JACS 124, p. 2164-2176 (2002) but not as an isolated product.

**1-(2-azido-3-*O*-benzyl-4-*C*-methanesulfonyloxymethyl-5-*O*-methanesulfonyl-2-deoxy-α-L-erytro-pentofuranosyl)-6-benzoyl adenine-9-yl-(64).** Not quite pure **62** (6.23 g, 0.008 mol) was dissolved in dry DMF (70 ml), added NaN₃ (5.2 g, 10 equiv.) and allowed to stir at r.t. for 3 days. Quenched by addition of water (100 ml) and diluted with DCM (200 ml). Layers were separated and the org. layer was washed with brine (2 × 125 ml), dried (Na₂SO₄) and the solvent removed *in vacuo.* The residue was purified by dry column liquid chromatography (eluent: heptane → EtOAc) to afford pure **64** (5.38 g, quantitative yield). R_{f} = 0.60 (Eluent: EtOAc). ESI-MS *m*/*z* found 673.0 ([MH]⁺, calcd 673.1);¹H-NMR(CDCl₃, 400 MHz): δ 9.14 (1H, s), 8.70 (1H, s), 8.93 (1H, s), 8.00 (3H, d, *J* = 7.3 Hz), 7.59-7.50 (3H, 2 × t, *J* = 7.3 Hz), 7.41-7.37 (5H, m), 6.51 (1H, d, *J* = 4 Hz, H-1), 4.92 (1H, d, *J* = 11.7 Hz), 4.77 (1H, d, *J* = 11.3 Hz), 4.75 (1H, d, *J* = 4.8 Hz, H-3), 4.70 (1H, d, *J* = 11.3 Hz), 4.50 (1H, dd, *J* = 4.2 Hz, *J* = 4.6 Hz, H-2), 4.41 (2H, d, *J* = 11-12 Hz), 4.27 (1H, d, *J* = 11 Hz), 3.05 (3H, s, OMs), 3.02 (3H, s, OMs).¹³C-NMR(CDCl₃, 100 MHz): δ 164.4, 162.3, 152.5, 151.1, 149.3, 142.1, 135.5, 133.3, 132.6, 128.9, 128.8, 128.8, 128.7, 128.4, 127.6, 122.3 (A^{BZ} and OBn), 82.35, 81.79, 79.55, 74.58 (OBn), 68.51, 68.06, 62.59, 37.78 (OMs), 37.57 (OMs)

**(1*S*,3*R*,4*R*,7*S*)-7-Benzyloxy-1-methansulfonyloxymethyl-3-(6-benzoyladenin-9-yl)-2-oxa-5-azabicyclo[2:2:1]heptane (65).** To a solution of **64** (2.28 g, 3.4 mmol) in THF (100 ml) at rt aq NaOH (2.0 M,34 ml) and PMe₃ in THF (1.0 M, 7 ml) was added with stirring. After over night at r.t. the THF was partly removed under reduced pressure. Brine (100 mL) and EtOAc (200 mL) was added and the phases were separated. The org. layer was washed with brine (100 ml). The comb. aqueous layer was extracted with dichloromethane (200 mL). The combined organic phases were dried (Na₂SO₄), filtered and concentrated in vacuo to give a yellow foam (1.73 g) which was purified by dry column liquid chromatography to afford pure nucleoside **65** (848mg) as a yellow foam. R_{f} = 0.13 (EtOAc). *Comb. with residues from similar reactions before purification; R_{f} = 0.21 (eluent: EtOAc). ESI-MS m/z found 551.1 ([MH]⁺, calcd 551.1); ¹H NMR (DMSO-d₆, 400 MHz): δ 11.18 (1H, br s, NH), 8.77 (1H, s, A^{Bz}), 8.73 (1H, s, A^{Bz}), 8.06 (2 H, d, *J* = 7.3 Hz), 7.64 (1H, t, *J* = 7.3 Hz, Bz), 7.55 (2H, t, *J* = 7.3 Hz, Bz), 7.45 (2H, d, *J =* 7.2 Hz, Bn), 7.38 (2H, t, *J* = 7.2 Hz, Bn), 7.31 (1H, t, *J* = 7.2 Hz, Bn), 6.52 (1H, d, *J =* 1.6 Hz, H-1'), 4.74 (1H, d, *J* = 11.9 Hz, H-5'a/H-1"a), 4.65 (1H, d, *J* = 11.9 Hz, H-5'b/H-1"b), 4.59 (1H, d, *J* = 11.9 Hz, H-1"a/H-5'a), 4.52 (1H, d, *J* = 11.8 Hz, H-1"b/H-5'b), 4.44 (1H, s, H-3'), 4.04 (1H, d, *J* = 7.2 Hz, Bn), 4.01 (1H, d, *J* = 7.2 Hz, Bn), 3.91 (1H, br s, H-2'), 3.22 (3H, s, OMs); ¹³C-NMR (DMSO-d₆, 100 MHz): δ 170.34, 165.59, 152.12, 151.47, 150.09, 143.20, 137.89, 133.44, 132.43, 128.48, 128.31, 127.70, 125.19 (Bz and Bn), 87.30, 84.45, 80.47, 71.13 (Bn), 66.99, 59.92, 59.80, 51.27, 36.93 (OMs)

**2',3'-epoxide (66)**. To a solution of **62** (50 mg) in dry DCM (1.5 ml) at r.t. was added MsOH (0.5 ml) dropwise. Reaction was stirred at r.t. until full conversion of s.m. was confirmed by LCMS. Reaction was diluted with DCM (20 ml), cooled to 0 °C, neutralized with Et₃N (1.1 ml), washed with sat. NaHCO₃-soln (20 ml), brine (20 ml), dried (Na₂SO₄), filtered and the solvent removed *in vacuo* to afford a clear oil **(66)** (49 mg, quantitative yield). R_{f} = 0.24 (eluent: EtOAc). ESI-MS *m*/*z* found 540.2 ([MH]⁺, calcd 540.1); ¹H NMR (CDCl₃, 400 MHz): δ 9.3 (1H, br s, N-H), 8.67 (1H, s, base), 8.33 (1H, s, base), 7.94 (2H, d, *J* = 7.5 Hz), 7.51 (1H, t, *J* = 7.4 Hz), 7.42 (2H, t, *J* = 7.5 Hz), 6.61 (1H, s, H-1'); 4.57 (1H, d, *J* = 11.3 Hz), 4.47 (1H, d, *J* = 10.8 Hz), 4.44 (1H, d, *J* = 11.3 Hz), 4.36 (1H, d, *J* = 10.8 Hz), 4.25 (1H, d, *J* = 2.7 Hz, H-2'/H-3'), 4.13 (1H, d, *J* = 2.7 Hz, H-3'/H-2'), 3.11 (3H, s, OMs), 3.01 (3H, s, OMs); ¹³C-NMR (CDCl₃, 100 MHz): δ 164.7, 152.6, 151.5, 149.4, 141.3, 133.2, 132.6, 128.6, 128.6, 128.3, 128.3, 127.7, 122.2 (A^{Bz}), 81.45, 81.23, 68.64, 66.58, 57.59, 57.27, 37.66 (OMs), 37.50 (OMs);

**1-(2-azido-3-*O*-benzyl-4-*C*-methanesulfonyloxymethyl-5-*O*-methanesulfonyl-2-deoxy-α-L-*threo*-pentofuranosyl)-6-benzoyl adenine-9-yl (67).** To a solution of **66** (50 mg, 0.093 mmol) in anh. DMF (2 ml) was added NaN₃ (60 mg, 10 equiv.). The mixture was heated to 50 °C overnight. LCMS confirms full conversion of **66** to **67**. Reaction mixture was diluted with water (15 ml) and DCM (15 ml). Layers were separated and the org. layer was washed with brine (15 ml), dried (Na₂SO₄), filtered and the solvent removed *in vacuo* to afford **67** (43 mg, 80% yield). R_{f} = 0.51 (eluent: EtOAc). ESI-MS *m*/*z* found 583.0 ([MH]⁺, calcd 583.1); ¹H NMR (CDCl₃, 400 MHz): δ 11.27 (1H, s, N-H), 8.79 (1H, s, base), 8.05 (2H, d, *J* = 7.3 Hz, Bz), 7.95 (1H, s, base), 7.65 (1H, t, *J* = 7.5 Hz), 7.55 (2H, t, *J* = 7.5 Hz), 6.70 (1H, d, *J* = 5.5 Hz, H-1'), 6.18 (1H, d, *J* = 8.6 Hz, 3'-OH), 5.27 (1H, t, *J* = 8.6 Hz, H-3'), 4.66 (1H, d, *J* = 10.7 Hz, CH₂), 4.57 (1H, dd, *J* = 5.6 Hz, *J* = 8.5 Hz, H-2'), 4.47 (1H, d, *J* = 10.8 Hz, CH₂), 4.41 (2H, d, *J* = 10.8 Hz, CH₂), 3.29 (3H, s, OMs), 3.22 (3H, s, OMs); ¹³C-NMR (CDCl₃, 100 MHz): δ 165.67, 162.33, 152.33, 152.20, 150.75, 142.63, 133.28, 132.56, 128.57, 128.52, 125.52, 82.34, 81.79, 74.77, 69.00, 68.46, 66.11, 36.87 (OMs), 35.85 (OMs)

**(1*R*,3*R*,4*R*,7*S*)-7-hydroxy-1-methansulfonyloxymethyl-3-(6-benzoyladenin-9-yl)-2-oxa-5-thiobicyclo[2:2:1]heptane (68).** Compound **66** (1.0 g, 1.9 mmol) was dissolved in dry DMF and added Na₂S (290 mg, 2 equiv.). Reaction turns green. Allowed to stir at r.t. overnight. LCMS confirms full conversion of compound 1. Reaction mixture was partitioned between brine (100 ml) and EtOAc (100 ml). Layers were separated and the aq. layer was extracted with EtOAc (2 × 100 ml) and DCM (2 × 100 ml). Combined organic layer was washed with brine (200 ml), dried (Na₂SO₄), filtered and the solvent removed *in vacuo.* Residue was purified by DCLC to afford compound 2 (268 mg, 30% yield). LCMS: found 478.0, calc. 478.0 (M+H). ¹H-NMR(CDCl₃, 400 MHz): δ 9.5-7.3 (8H, A^{Bz}), 6.46 (1H, s, H-1), 4.64 (2H, 2 × d, *J* = 11.4 Hz, H-1"a and b), 4.56 (1H, d, *J* = 1 Hz, H-3'), 3.75 (1H, d, *J* = 1 Hz, H-2'), 3.04, 5.97 (2H, 2 × d, *J* = 10.8 Hz, H-5' a and b), 3.02 (3H, s, OMs).

(**1*S*,3*R*,4*S*,7*S*)-7-Benzyloxy-1-methansulfonyloxymethyl-3-(6-benzoyladenin-9-yl)-2-oxa-5-thiobicyclo[2:2:1]heptane (69)**. Compound **62** (3.30 g, 4.2 mmol) was dissolved in dry DMF (33 ml) and added Na₂S (1.65 g, 5 equiv.). Reaction colour goes from green to orange in 30 min. LCMS confirms full conversion to compound 2. Reaction mixture was partitioned between sat. NaHCO₃-soln. (150 ml) and DCM (150 ml). Layers were separated and the aqueous layer was extracted with DCM (2 × 75 ml). Combined org. layer was washed with sat. NaHCO₃-soln. (150 ml), brine (150 ml), dried (Na₂SO₄), filtered and the solvent removed *in vacuo* to afford an oily residue (∼3 g) which was used in the next step without further purification. LCMS: found 568.0, calc. 568.1 (M+H). ¹H-NMR(400 MHz, CDCl₃) : δ 9.5 (1H, br s, N-H), 8.65 (1H, s, A^{Bz}), 8.36 (1H, s, A^{Bz}), 7.99 (2H, 2 × d, *J* = 7.3 Hz, A^{Bz}), 7.54 (1H, t, *J* = 7.3 Hz, A^{Bz}), 7.45 (2H, t, *J* = 7.3 Hz, A^{Bz}), 7.30-7.36 (5H, m, OBn), 6.61 (1H, d, *J* = 2.2 Hz, H-1'), 4.72 (1H, d, *J* = 11.6 Hz, H-1"a), 4.59 (1H, d, *J* = 11.3 Hz, H-1"b), 4.59 (1H, d, *J* = 1.6 Hz, H-3'), 4.91, (2H, s, OBn), 4.05 (1H, t, *J* = 2.0 Hz, H-2'), 3.17 (1H, d, *J* = 10.5 Hz, H-5'a), 3.05 (1H, d, *J* = 11.0 Hz, H-5'b), 3.02 (3H, s, OMs).¹³C-NMR(100 MHz, CDCl₃): δ 152.1, 150.8, 149.2, 141.3, 136.1, 133.4, 132.5, 128.6, 128.6, 128.5, 128.2, 127.8, 127.7, 123.0, (A^{Bz}, OBn), 87.34 (C-4'), 87.25 (C-1'), 80.35 (C-3'), 72.05 (C-1"), 66.48 (OBn), 51.80 (C-2'), 37.67 (OMs), 36.00 (C-5').

### Compound 70

Compound **69** (2.38 g, 4.2 mmol) was dissolved in dry DMSO (25 ml), added NaOBz (1.24 g, 2 equiv.) and heated to 100 °C overnight. LCMS confirms full conversion to compound 4. Reaction mixture was partitioned between water (150 ml) and DCM (150 ml). Layers were separated and the aqueous layer was extracted with DCM (2 × 100 ml). Comb. organic layer was washed with brine (2 × 150 ml), dried (Na₂SO₄), filtered and concentrated onto silica. Purified by DCLC to afford compound 3 (945 mg, 38% over two steps). LCMS: found 594.2, calc. 594.1 (M+H). ¹H-NMR(400 MHz, CDCl₃): δ 8.63-7.18 (17 H, A^{Bz}, OBz, OBn), 6.56 (1H, d, *J* = 2.2 Hz, H-1'), 4.72 (1H, d, *J* = 11.5 Hz, H-1"a), 4.69 (1H, d, *J* = 11.0 Hz, H-1"b), 4.57 (1H, d, *J* = 1.6 Hz, H-3'), 4.53 (1H, d, *J* = 11.6 Hz, OBn), 4.49 (1H, d, *J* = 12 Hz, OBn), 4.01 (1H, br s, H-2'), 3.24 (1H, d, J = 10.4 Hz, H-5'a), 2.99 (1H, d, J = 10.4 Hz, H-5'b). ¹³C-NMR(100 MHz, CDCl₃): δ 165.5, 152.1, 150.7, 149.2, 141.4, 136.2, 133.5, 133.2, 132.5, 129.5, 129.1, 128.6, 128.4, 128.3, 128.1, 127.7, 127.6 (A^{Bz}, OBz, OBn), 87.73 (C-4'), 87.32 (C-1'), 80.47 (C-3'), 71.88 (C-1"), 61.73 (OBn), 51.80 (C-2'), 36.43 (C-5').

### Compound 71

Compound **70** (966 mg, 1.627 mmol) was dissolved in dry DCM (27 ml) and added MSOH (9 ml). Stirred at r.t. for 1 hr. LCMS confirms full debenzylation.* Reaction mixture was diluted with DCM (30 ml), washed with brine (50 ml), sat. NaHCO₃-soln. (50 ml), dried (Na₂SO₄), filtered and the solvent removed *in vacuo* to afford compound 6 (739 mg, 90% yield). LCMS: found 504.1, calc. 504.1 (M+H). *Depurination is also detected, so cooling might be advantageous.

### Compound 73

Compound **71** (739 mg, 1.468 mmol) was dissolved in THF (60 ml) and added 1 M LiOH (7.5 ml). The reaction was stirred at r.t. More 1 M LiOH (1 ml) was added after 90 min. Completion of reaction was confirmed by TLC (eluent: EtOAc/MeOH 9: 1) after another 60 min. The reaction was quenched with 1 M HCl satd. with NaCl (8.5 ml) and the mixture was partitioned between brine (100 ml) and EtOAc (100 ml). Layers were separated and the aqueous layer was extracted with EtOAc (2 × 100 ml). Combined org. layer was washed with brine (100 ml), dried (Na₂SO₄), filtered and the solvent removed *in vacuo* to afford a yellow solid (compound **72**), which was co-evaporated with dry pyridine. The residue was dissolved in dry pyridine (25 ml), added DMAP (180 mg, 1 equiv.) followed by DMTCI (597 mg, 1.2 equiv.) and stirred at r.t. Added more DMTCl (200 mg). TLC (eluent: EtOAc/MeOH 9:1) after 24 hrs shows full conversion to compound **73**. Reaction was diluted with DCM (100 ml) and washed with water (100 ml). Aqueous layer was extracted with DCM (50 ml) and combined org. layer was washed with sat. NaHCO₃-soln. (100 ml), brine (100 ml), dried (Na₂SO₄), filtered and the solvent removed *in vacuo* to afford a residue which was purified by DCLC to afford compound 6 (518 mg, 50% over two steps). LCMS: found 702.2, calc. 702.2 (M+H).

### Compound 74

Compound **73** (518 mg, 0.738 mmol) was dissolved in DCM (10 ml), added 1 M DCI (520 µl, 0.7 equiv., dissolved in acetonitrile) followed by bisamidite reagent (244 µl, 1 equiv.) and stirred at r.t., under an atmosphere of N₂. More bisamidite reagent was added (2 × 40 µl) and the flask was transferred to the frigde over weekend. LCMS confirms full conversion to amidite. The reaction mixture was diluted with DCM (100 ml), washed with sat. NaHCO₃-soln. (2 × 100 ml), brine (100 ml), dried (Na₂SO₄), filtered and the solvent removed *in vacuo* to afford compound **74** (642 mg, 97% yield). LCMS: found 902.2, calc. 903.3 (M+H).

**9-(2-*O*-Acetyl-3-*O*-benzyl-5-*O*-methanesulfonyl-4-*C*-methanesulfonyloxymethyl-β-L-*threo*-furanosyl)-2-amino-6-chlorpurine (75).** 1,2-Di-*O*-acetyl-5-*O*-methanesulfonyl-4-*C*-methanesulfonyloxymethy-3-*O*-benzyl-L-*threo*pentofuranose (20.6 g, 40.0 mmol) is dissolved in anh. 1,2-dichloroethane (250 mL) and 2-amino-6-chloropurine (7.5g, 44.4 mmol) was added followed by *N,O-*bis(trimethylsilyl)acetamide (19.6 mL, 80.0 mmol). The reaction mixture was refluxed until it turned clear (1 h) and cooled to room temperature. Trimethylsilyl triflate (14.5 mL, 80.0 mmol) was added over 15 min and the reaction mixture was refluxed for 3 h. The reaction mixture was allowed to cool to room temperature and was poured into sat. aq NaHCO₃ (500 mL). CHCl₃ (300 mL) was added and after 30 min of vigorous stirring the mixture was transferred to a separatory funnel. The phases were separated and the aq-phase was extracted with CHCl₃ (3 × 250 mL). The combined organic phases were washed with sat. aq NaHCO₃:brine (1:1, 500 mL), dried (Na₂SO₄), filtered and evaporated *in vacou* to give a red foam. The product was purified by Dry Column Vacuum Chromatography (Ø 10 cm, 50-100% EtOAc in *n*-heptane v/v, 10% increments, 2 × 100 mL fractions, followed by: 1-10% MeOH in EtOAc v/v, 1% increments, 100 ml fractions). The fractions containing nucleoside **75** were pooled and evaporated *in vacou* to give a white foam (15.6 g, 65%). Further was isolated the N7 isomere (2.0g). Compound **75**: R_{f} = 0.59 (10% MeOH in EtOAc, v/v), ESI-MS *m*/*z* found 620.1; 622.0 ([MH]⁺, calcd. 620.1).¹H NMR (CDCl₃, 400 MHz): δ 8.03 (s, 1H, H8), 7.38-7.29 (m, 5H, Ar-H), 6.14 (d, 1H, *J* = 3.3 Hz, H¹'), 5.90 (dd (looks like t), 1H, *J* = 3.3 Hz and 3.0 Hz, H2'), 5.29 (s br, 2H, NH₂), 4.78 (d, 1 H, *J* = 10.6 Hz, CH₂), 4.70 (d, 1 H, *J* = 11.3 Hz, CH₂), 4.67 (d, 1 H, *J* = 11.8 Hz, CH₂), 4.44 (d, 1 H, *J* = 11.3 Hz, CH₂), 4.37 (d, 1 H, *J* = 10.6 Hz, CH₂), 4.37 (d, 1H, *J* = 3.0 Hz, H3'), 3.01 (s, 3H, Ms), 2.96 (s, 3 H, Ms), 2.14 (s, 3H, Ac). ¹³C NMR (CDCl₃, 100 MHz): δ 169.4 (CH₃C(O)), 159.1, 153.2, 151.7 (C2, C4, C6), 140.4 (C8), 136.5, 128.8, 128.5, 128.4 (Ph), 125.3 (C5), 87.0 (C1'), 85.4 (C4'), 81.2 (C3'), 78.8 (C2'), 73.4 (CH₂), 67.5, 65.8 (2×CH₂), 37.7, 37.6 (2xMs), 20.6 (CH₃C(O)). Anal. calcd. for C₂₂H₂₆CIN₅O₁₀S₂ : C, 42.6; H, 4.2; N, 11.3. Found: C, 42.5; H, 4.2; N, 11.0.

**9-(3-*O*-benzyl-5-*O*-methanesulfonyl-4-*C*-methanesulfonyloxymethyl-β-L-*threo*furanosyl)-2-amino-6-chlorpurine (76).** Compound **75** (5.0 g, 8.1 mmol) is dissolved in methanol (100 mL) and cooled to 0°C and sat. methanolic ammonia (100 ml) was added. The mixture was stirred at 0°C for 1 h and then the reaction was quenched by neutralisation with glacial acetic acid (app. 30 mL). Sat. aq NaHCO₃ (100 mL) and CHCl₃ (100 mL) was added and after 5 min of vigorous stirring the mixture was transferred to a separatory funnel. The phases were separated and the aq-phase was extracted with CHCl₃ (3 × 200 mL). The combined organic phase was dried (Na₂SO₄), filtered and the solvent removed in vacuo to afford **76** (4.60 g, 99%) as a white solid. R_{f} = 0.67 (EtOAc). ESI-MS *m*/*z* found 578.1; 580.0 ([MH]⁺, calcd. 578.1). ¹H NMR (CD₃CN, 400 MHz): δ 8.03 (s, 1H, H8), 7.41-7.33 (m, 5H, Ar-H), 5.86 (d, 1H, *J* = 6.2 Hz, H1'), 5.71 (s br, 2H, NH₂), 5.90 ("q" 1H, *J* = 4.6 Hz, H2'),4.82 (d, 1 H, *J* = 11.5 Hz, CH₂), 4.72 (d, 1 H, J = 11.5 Hz, CH₂), 4.68 (d, 1 H, J = 11.0 Hz, CH₂), 4.44-4.32 (m, 5H, CH₂(3), H3', OH), 3.10 (s, 3H, Ms), 2.98 (s, 3 H, Ms).¹³C NMR (CD₃CN, 100 MHz): δ 160.6, 154.7, 151.5, 142.3 (C2, C4, C6, C8), 138.4, 129.3, 129.0, 128.9 (Ph), 125.8 (C5), 88.4 (C1'), 83.8, 83.6 (C2', C4'), 77.5 (C3'), 73.9 (CH₂), 69.6, 69.5 (2×CH₂), 37.7, 37.5 (2×M₅). Anal. calcd. for C₂₂H₂₆ClN₅O₁₀S₂: C, 42.6; H, 4.2; N, 11.3. Found: C, 42.5; H, 4.2; N, 11.0.

**9-(3-*O*-benzyl-5-*O*-methanesulfonyl-4-*C*-methanesulfonyloxymethyl-2-*O-*trifluoromethanesulfonyl-*β*-L -*threo*-furanosyl)-2-amino-6-chlorpurine (77).** Compound **75** (4.50 g, 7.8 mmol) was dissolved in anh. CH₃CN (2 × 50 mL) and concentrated in vacuo to remove water traces. The compound was dissolved in anh. dichloromethane (50 mL) and anh pyridine (6.30 mL, 77.8 mmol) was added followed by the addition of DMAP (3.80 g, 31.1 mmol). After cooling to 0 °C trifluoromethanesulfonic anhydride (2.57 mL, 15.6 mmol) was added dropwise during 20 min. The reaction mixture was stirred for an additional 40 min and ice cooled sat. aq NaHCO₃ (100 mL) was added and after 5 min of vigorous stirring the mixture was transferred to a separatory funnel. The phases were separated and the aq phase was extracted with CH₂Cl₂ (2 × 100 mL). The combined organic phase was washed successively with aq HCl (0.1 M, 2 x100 mL) and sat. aq NaHCO₃ (100 mL), dried (Na₂SO₄), filtered and evaporated in vacuo. The residue was purified by DCVC (Ø = 6 cm, 0-100% EtOAc in *n*-heptane v/v, 5% increments, 100 mL fractions) yielding nucleoside **77** (5.05g, 91%) as a white powder. R_{f} = 0.18 (1:1 EtOAc in *n*-heptane v/v). ESI-MS *m*/*z* found 710.0; 711.9 ([MH]⁺, calcd. 710.0). ¹H NMR (DMSO-d₆, 400 MHz): δ 8.45 (s, 1H, H8), 7.42-7.36 (m, 5H, Ar-H), 7.16 (br. s, 2H NH₂), 6.48-6.48 (m, 2H), 5.02 (dd, 1H, *J* = 6.2, 1.6 Hz), 4.85 (dd, 2H, *J* = 10.8, 1.1 Hz), 4.67 (d, 1H, *J* = 11.0 Hz), 4.57-4.48 (m, 3H), 3.34 (s, 3H, Ms), 3.18 (s, 3 H, Ms). ¹³C NMR (DMSO-d₆, 100 MHz): δ 160.0, 153.8, 150.2, 141.2 (C2, C4, C6, C8), 136.4, 128.5, 128.5, 128.4 (Ph), 123.4 (C5), 117.7 (q, *J* = 319.7 Hz, CF₃), 87.0 (C1'), 80.8, 80.2 (C3', C4'), 73.8 (CH₂), 68.6, 68.4 (2×CH₂), 59.8 (C2'), 36.9, 36.5 (2×Ms). Anal. calcd. for C₂₁H₂₃ClF₃N₅O₁₁S₃·0.25 EtOAc: C, 36.1; H, 3.4; N, 9.6. Found: C, 36.1; H, 3.2; N, 9.5. NOTE: ¹⁹F was also recorded and showed only a single peak.

**(*1S,3R,4S,7R)*-7-benzyloxy-1-(mesyloxymethyl)-3-(guanin-9-yl)-2,5-dioxabicyclo[2.2.1]heptane (78)**. 3-Hydroxypropionitrile (3.55 mL, 52 mmol) was dissolved in anh. THF (75 mL) and cooled to 0°C. Sodiumhydride (60% in mineral oil, 2.50 g, 62.4 mmol) was added in portions and the temperature was allowed to raise to rt and the mixture was stirred for 30 min at rt. The reaction mixture was cooled to 0°C again and 9-(3-*O*-benzyl-5-*O*-methanesulfonyl-4-*C*-methanesulfonyloxymethyl-2-*O-*trifluoromethanesulfonyl-β-L-*threo*-furanosyl)-2-amino-6-chlorpurine **(77)** (7.37g, 10.4 mmol) dissolved in anh. THF (75 mL) was added dropwise over 20 min and the temperature was allowed to raise to rt. After 8 h the reaction was quenched by addition of HCl (1 M, aq):brine (1:9, 250 mL) and the mixture was transferred to a separatory funnel. The phases were separated and the aq.-phase was extracted with EtOAc (3 × 200 mL). The combined organic phases were dried (Na₂SO₄), filtered and evaporated *in vacuo* to give a red oil. The product was purified by first filtration through a short silica plug (∅ 6 cm, 10% MeOH in EtOAc v/v, 500 mL) and the resulting material was then precipitated from EtOH:H₂O (1:1) resulting in **78** as a tan solid (4.64g, 96%). R_{f} = 0.31 (10% MeOH in EtOAc v/v); ESI-MS *m*/*z* found 464.1 ([MH]⁺, calcd. 464.1); ¹H NMR (DMSO-d₆, 400 MHz): δ = 10.63 (s br, 1 H, NH), 7.72 (s, 1 H, H8), 7.30-7.24 (m, 3 H, Ar-H), 7.16-7.11 (m, 2 H, Ar-H), 6.65 (s br, 2 H, NH₂), 5.86 (s, 1 H, H1'), 4.83 (d, 1 H, *J* = 11.5 Hz, H1''), 4.71 (d, 1 H, *J* = 11.4 Hz, H1"), 4.60 (d, 1 H, *J* = 1.8 Hz, H2'/H3'), 4.52 (d, 1 H, *J* = 11.9 Hz, PhCH₂), 4.34 (d, 1 H, *J* = 11.9 Hz, PhCH₂), 4.27 (d, 1 H, *J* = 1.8 Hz, H2'/H3'), 4.08 (d, 1 H, *J* = 8.4 Hz, H5'), 3.86 (d, 1 H, *J* = 8.2 Hz, H5'), 3.27 (s, 3 H, Ms); ¹³C NMR (DMSO-d₆, 100 MHz): δ = 156.7 (C6), 153.8 (C2), 150.5 (C4), 137.0 (Ph), 135.6 (C8), 128.3, 127.9, 127.9 (Ph), 116.2 (C5), 86.8 (C4'), 85.5 (C1'), 80.2 (C3'), 76.4 (C2'), 72.5, 72.2 (Ph CH₂, C5'), 66.4 (C1"), 36.8 (Ms). Anal. calcd. for C₁₉H₂₁N₅O₇S: C, 49.2; H, 4.6; N, 15.1. Found: C, 49.4; H, 4.5; N, 15.2.

**(1*S*,3*R*,4*S*,7*R*)-7-benzyloxy-1-(benzoyloxymethyl)-3-(guanin-9-yl)-2,5-dioxabicyclo[2.2.1]heptane (79).** Compound **78** was dissolved in DMSO (25 mL) and BzONa (2.22 g, 15.24 mmol) was added. Heated to 100°C for 6 h and then to 120°C for 3 h. The reaction was diluted with EtOAc (50 mL) and quenched with water:sat. aq. NaHCO₃ (1:1, 100 mL). The phases were separated and the aq phase was extrated with EtOAc (3 × 50 mL). The comb. org. phases were washed with Brine (2 × 100 mL) dried (Na₂SO₄), filtered and concentrated. The product was purified by Dry Column Vacuum Chromatography (∅ 4 cm, 0-15% MeOH in dichloromethane v/v, 1% increments, 100 mL fractions). The fractions containing nucleoside **79** were pooled and evaporated *in vacuo* to give a white solid (1190 mg, 95%). R_{f} = 0.15 (5% MeOH in DCM v/v); ESI-MS *m*/*z* found 488.3 ([M-H]⁻, calcd. 488.2); ¹H NMR (DMSO-d₆, 400 MHz): δ = 10.63 (s, 1 H, NH), 7.95 (d, 2 H, *J* = 7.3 Hz, Bz), 7.67 (s, 1 H, H8), 7.64 (t, 1 H, *J* = 7.3 Hz, Bz), 7.50 (t, 3 H, *J* = 7.3 Hz, Bz), 7.24-7.18 (m, 3 H, Bn), 7.12-7.06 (m, 2 H, Bn), 6.54 (br s, 2 H. -NH₂), 5.86 (s, 1 H, H1'), 4.79 (s, 2 H, H1"), 4.59 (d, 1 H, J = 1.8 Hz, H2'/H3'), 4.49 (d, 1 H, *J* = 11.9 Hz, PhCH₂O), 4.34 (d, 1 H, *J* = 11.9 Hz, PhCH₂O), 4.29 (d, 1 H, *J* = 1.8 Hz, H2'/H3'), 4.11 (d, 1 H, *J* = 8.4 Hz, H5'a), 3.93 (d, 1 H, *J* = 8.2 Hz, H5'b); ¹³C NMR (DMSO-d₆, 100 MHz): δ = 165.3 (C(O)Ph), 156.7, -153.8, 150.1 (C2; C4; C6), 137.0 (Bn), 135.5 (C8), 133.7 (Bz), 129.4, 129.1, 128.9, 128.3, 127.9 (Ph), 116.3 (C5), 86.8, 85.9 (C4', C1'), 80.0, 76.4 (C2', C3'), 72.7, 72.2 (Ph CH₂, C5'), 60.6 (C1")

(**1*R*,3*R*,4*S*,7*R*)-3-(guanin-9-yl)-7-hydroxy-1-hydroxymethyl-2,5-dioxabicyclo-[2.2.1]heptane (80).** Compound **79** (2.33 g, 4.16 mmol) was suspended in MeOH (100 mL) and Pd(OH)₂-C (20%, 292 mg, 10% mol Pd) and ammounium formiat (5.24 g; 83.2 mmol) were added. The mixture was heated to reflux. After 4 h further Pd(OH)₂-C (20%, 292 mg, 10% mol Pd) was added and after an additional 4 h the last Pd(OH)₂-C (20%, 292 mg, 10% mol Pd) was added. Reflux for another 12 h.
The catalysis was removed by filtration through paper, the filter paper with catalyst was boiled in MeOH for 30 min and then filter again. The two methanolic solutions are pooled and filtred through Celite. The Celite was washed thoroughly with hot MeOH. All the methanolic solutions were pooled and concentrated. Taken up in H₂O and lyophilized twice resulted in **79** as white solid. (1100 mg, 90%). R_{f} = 0.01 (10% MeOH in EtOAc v/v); ESI-MS *m*/*z* found 296.1 ([MH]⁺, calcd. 296.1); ¹H NMR (D₂O, 400 MHz): δ = 7.90 (s, 1 H, H8), 5.91 (s, 1 H, H1'), 4.74 (d, 1 H, *J* = 2.4 Hz, H2'/H3'), 4.40 (d, 1 H, *J* = 2.4 Hz, H2'/H3'), 4.12 (d, 1 H, *J* = 8.6 Hz, H5'), 4.02 (d, 1 H, *J* = 8.7 Hz, H5'), 4.01 (s, 2 H, H5'); ¹³C NMR (D₂O, 100 MHz): δ = 158.7 (C6), 153.7 (C2), 150.8 (C4), 138.4 (C8), 115.4 (C5), 88.7 (C4'), 86.3 (C1'), 78.1 (C3'), 73.2, 72.4 (C2', C5'), 57.4 (C1"); Anal. calcd. for C₁₁H₁₃N₅O₅·H₂O : C, 42.2; H, 4.8; N, 22.4. Found: C, 42.0; H, 4.5; N, 22.2.

**(1*S*,3*R*,4*S*,7*R*)-1-(4,4'-dimethoxytrityloxymethyl)-3-(2-*N-*((dimethylamino)methylidene)-7-hydroxy-guanin-9-yl)-2,5-dioxabicyclo[2.2.1]heptane (82)**. Compound **80** (860 mg, 2.91 mmol) was dissolved in anh. DMF (25 mL) and *N,N*-Dimethylformamide dimethyl acetal (0.77 mL, 5.83 mmol) was added. After 4 h the reaction was completed and most of the DMF was remove *in vacuo.* The resulting slurry **81** was coevaporated twice from anh. pyridine (2 × 25 mL) and suspended in anh. pyridine (10 mL). 4,4'-dimethoxytritylchloride (1.48 g, 4.37 mmol) was added and the reaction mixture was stirred for 16 h at rt. Most of the pyridine was removed in vacuo and the residue was taken up in DCM (50 mL) and washed with half sat. aq. NaHCO₃ (2 × 50 mL) and brine (50 mL). The comb. aq. phases were extracted with DCM (2 × 50 mL) and the comb. org. phases were dried (Na₂SO₄), filtered and evaporated *in vacuo* to give a yellow foam. The product was purified by Dry Column Vacuum Chromatography (∅ 4 cm, 0-10% MeOH in DCM v/v, 0.5% increments, 100 ml fractions). The fractions containing nucleoside **82** were pooled and evaporated *in vacuo* to give a white foam (1.10 g, 58%). R_{f} = 0.08 (10% MeOH in EtOAc, v/v); ESI-MS *m*/*z* found 653.3 ([MH]⁺, calcd. 653.3; ¹H NMR (DMSO, 400 MHz): δ = 11.29 (s, 1H, NH), 8.57 (s, 1H, N=CH), 7.87 (s, 1 H, H8), 7.46-7.40 (m, 2H, DMT), 7.35-7.22 (m, 7H, DMT), 6.93-6.88 (m, 4H, DMT), 6.00 (s, 1 H, H1'), 5.92 (d, 1 H, *J* = 3.8 Hz, H2'), 4.51 (d, 1 H, *J* = 2.0 Hz, OH), 4.21 (dd, 1 H, *J* = 3.5 ,2.2 Hz, H3'), 4.05 (d, 1 H, *J* = 8.2 Hz, H1"), 3.98 (d, 1 H, *J* = 8.2 Hz, H1"), 3.74 (s, 6H, OCH₃), 3.51 (d, 1 H, *J* = 10.2 Hz, H5'), 3.38 (d, 1 H, *J* = 10.2 Hz, H5'), 3.33 (s, 3H, NCH₃), 3.15 (s, 3H, NCH₃); ¹³C NMR (DMSO, 100 MHz): δ 158.0 (DMT), 157.8, 157.4, 157.1 (C2, C6, N=CH), 149.2 (C4), 144.5 (DMT), 137.3 (C8), 135.2 (DMT), 129.6, 129.6, 127.7, 127.5, 126.6 (DMT), 118.9 (C5), 113.1 (DMT), 87.3, (C4'), 86.1 (C1'), 85.5 (DMT), 78.1 (C3'), 73.0, 72.7 (C1", C2'), 60.0 (C5'), 54.9 (OCH₃), 40.5, 34.6 (N(CH₃)₂); Anal. calcd. for C₃₅H₃₆N₆O₇H₂O: C, 62.7; H, 5.7; N, 12.5. Found: C, 62.8; H, 5.4; N, 12.6.

(**1*S*,3*R*,4*S*,7*R*)-1-(4,4'-dimethoxytrityloxymethyl)-7-(2-cyanoethoxy(diisopropylamino)phosphinoxy)-3-[2-*N-((N',N'-*dimethylamino)methylidene)-guanin-9-yl]-2,5-dioxabicyclo[2.2.1]heptane (83)**. Compound **82** (750 mg, 1.15 mmol) was dissolved in anh DMF (25 mL) and 4,5-dicyanoimidazole in MeCN (1.0 M, 0.80 mL, 0.8 mmol) was added at ambient temperature with stirring. 2-Cyanoethyl-*N,N,N;N'-*tetraisopropylphosphorodiamidite (0.40 mL, 1.26 mmol) was added dropwise to the reaction mixture. After 4 h the reaction was diluted with EtOAc (70 mL) and transferred to a separatory funnel and extracted with sat. aq NaHCO₃ (2 × 50 mL) and brine (50 mL). The combined aq phases were extracted with EtOAC (100 mL). The organic phases were pooled and dried (Na₂SO₄). After filtration the organic phase was evaporated *in vacuo* to give a yellow foam. Purification by DCVC (Ø 2 cm, 1-10% MeOH, EtOAc, v/v, 0.5% increments, 50 mL fractions (the column was pretreated with 1% Et₃N in EtOAc v/v)) afforded amidite **83** (480 mg, 49%) as a white solid. R_{f} = 0.50 (1%, TEA, 10% MeOH in DCM v/v/v); ESI-MS *m*/*z* found 853.2 ([MH]⁺, calcd. 853.4); ³¹P NMR (CDCl₃ 121 MHz) δ 151.7, 150.3.

**(1*S*,*3R,4S,7R*)-7-benzyloxy-3-(2-amino-6-chloro-purine-9-yl)-1-(methanesulfonyloxymethyl)-2,5-dioxabicyclo[2.2.1]heptane (84).** Compound **77** (7.44 g, 10.4 mmol) was dissolved in THF (300 mL). After cooling to 0°C aq LiOH (1.0M, 105 mL) was added. The reaction mixture was stirred at 0°C for 4 h and then for additional 2 h at rt. The reaction was quenched by addition of aq HCl (1.0M, sat. with NaCl, 100 mL) and after 5 min of vigorous stirring the mixture was transferred to a separatory funnel. The phases were separated and the aq-phase was extracted with EtOAc (3 × 150 mL). The combined organic phase was washed with brine: sat. aq NaHCO₃ (1:1, 200 mL), dried (Na₂SO₄), filtered and evaporated in vacuo. The residue was purified by DCVC (Ø=6 cm, 50-100% EtOAc in *n*-heptane v/v, 5% increments, 2 × 100 mL fractions) yielding nucleoside **84** (4.49 g mg, 89%) as a white powder. R_{f} = 0.49 (20% *n*-heptane in EtOAc (v/v)). ESI-MS *m*/*z* found 482.1.; 484.0 ([MH]⁺, calcd. 482.1). ¹H NMR (DMSO-d₆, 400 MHz): δ 8.09 (s, 1H, H8), 7.26-7.19 (m, 3H, Ar-H), 7.08-7.04 (m, 2H, Ar-H), 7.01 (br. s, 2H, NH₂), 5.96 (s, 1H, H1'), 4.86 (d, 1H, *J* = 11.3 Hz, H5"), 4.76 (d, 1H, *J* = 11.3 Hz, H5"), 4.65 (d, 1H, *J* = 2.0 Hz, H2'), 4.51 (d, 1H, *J* = 11.9 Hz, CH₂), 4.32 (d, 1H, *J* = 11.7 Hz, CH₂), 4.31 (d, 1H, *J* = 2.0 Hz, H3'), 4.10 (d, 1H, *J* = 8.2 Hz, CH₂), 3.89 (d, 1H, *J* = 8.4 Hz, CH₂), 3.28 (s, 3H, Ms). ¹³C NMR (DMSO-d₆, 100 MHz): δ 159.6, 153.0, 149.0 (C2, C4, C6), 140.8 (C8), 136.7, 128.0, 127.8, 127.7 (Ph), 123.2 (C5), 86.8 (C4'), 85.6 (C1'), 80.0 (C3'), 75.8 (C2'), 72.3, 72.2 (C5', CH₂Ph), 66.2 (C5"), 36.6 (Ms).

**(1*S*,3*R*,4*S*,7*R*)-7-benzyloxy-3-(2-amino-6-chloro-purine-9-yl)-1-(benzoyloxymethyl)-2,5-dioxabicyclo[2.2.1]heptane (85).** Compound **84** (4.49 g, 9.32 mmol) was dissolved in DMSO (200 mL) and BzONa (6.76g, 46.6 mmol) was added. The reaction was stirred at 100°C for 16 h. The reaction allowed to cool to room temperature and EtOAc (200mL) and brine : sat. aq NaHCO3 (1:1, 400 mL) was added. The mixture was transferred to a separatory funnel. The phases were separated and the aq-phase was extracted with EtOAc (3 × 200 mL). The combined organic phase was washed with brine (half-sat., 2 × 200 mL), dried (Na₂SO₄), filtered and evaporated in vacuo. The residue was purified by DCVC (Ø=6 cm, 50-100% EtOAc in *n*-heptane v/v, 5% increments, 2 × 100 mL fractions, 0-10% MeOH in EtOAc v/v, 1% increments, 100mL) yielding nucleoside **85** (3.30 g , 70%) as a white powder. R_{f} = 0.40 (35% *n*-heptane in EtOAc (v/v)). ESI-MS *m*/*z* found 508.2.; 510.1 ([MH]⁺, calcd. 508.1). ¹H NMR (DMSO-d₆, 400 MHz): δ 8.05 (s, 1H, H8), 7.98 (d, 2H, *J* = 7.3 Hz, Bz), 7.68 (t, 1H, *J* =7.3 Hz, Bz), 7.53 (t, 2H, *J* =7.7 Hz, Bz), 7.25-7.15 (m, 3H, Bn), 7.05-7.00 (m, 4H, Bn, NH₂), 5.98 (s, 1H, H1'), 4.85 (s, 2H, H5"), 4.67 (d, 1H, *J* = 1.8 Hz, H2'), 4.50 (d, 1H, *J* = 12.1 Hz, CH₂), 4.35 (d, 1H, *J* = 2.0 Hz, H3'), 4.34 (d, 1H, *J* = 11.7 Hz, CH₂), 4.16 (d, 1H, *J* = 8.4 Hz, CH₂), 3.98 (d, 1H, J = 8.1 Hz, CH₂). ¹³C NMR (DMSO-d₆, 100 MHz): δ 165.1 (PhCO), 159.6, 152.9, 149.0 (C2, C4, C6), 140.6 (C8), 136.7, 133.5, 129.2, 128.9, 128.7, 128.0, 127.9, 127.7,127.6 (Ph), 123.2 (C5), 86.7 (C4'), 85.9 (C1'), 79.9 (C3'), 75.8 (C2'), 72.5, 72.1 (5', CH₂Ph), 60.4 (C5").

**9-(3-*O*-Benzyl-2-deoxy-2-iodo-5-*O*-methanesulfonyl-4-*C-*(methanesulfonyloxymethyl)-β-D-*threo*-pentofuranosyl)-6-*N*-benzoyladenine (87).** 9-(3-*O*-Benzyl-5-*O*-methanesulfonyl-4-*C*-(methanesulfonyloxymethyl)-2-*O-*trifluoromethanesulfonyl-β-D-*erythro*-pentofuranosyl)-6-*N*-benzoyladenine (589 mg, 0.755 mmol) was dissolved in dry acetonitrile (15 ml), added lithiumiodide (202 mg, 2 equiv.) and heated to reflux. After 2 hrs, LCMS shows full conversion. Solvent was removed in vacuo and the residue was partitioned between DCM (50 ml) and water (50 ml). Layers were separated and the organic layer was washed with brine (50 ml), dried (Na₂SO₄), filtered and the solvent removed in vacuo to afford an orange foam (514 mg, 90% yield)

**9-(2-azido-3-*O*-Benzyl-2-deoxy-5-*O*-methanesulfonyl-4-*C-*(methanesulfonyloxymethyl)-β-D-*erythro*-pentofuranosyl)-6-*N*-benzoyladenine (88)** Compound **87** (30 mg) was dissolved in DMF/water 1:1 (2 ml) and followed by sodium azide (26 mg, 10 equiv.). The reaction mixture stirred at 80 °C overnight. LCMS confirms conversion of starting material to product substituted with azide.

## Claims

1. A method for the synthesis of an LNA analogue of the general formula IV 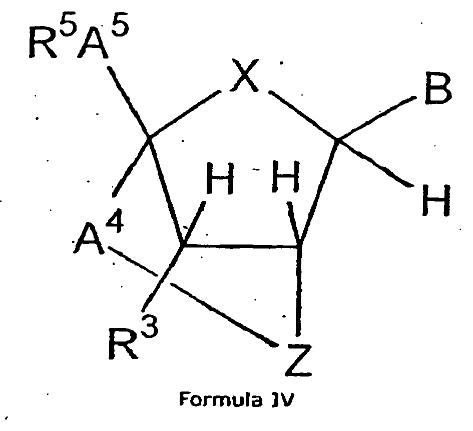 wherein
X is selected from -CH₂-, -NR^{H}-, -O-, and -S-;
Z is selected from -CH₂-, -NR^{H}-, -S-, and -Se-;
B is a nucleobase;
R³ is selected from -R^{H}, -N₃, -NR^{H}R^{H*}, -NR^{H}C(O)R^{H*}, -C(O)NR^{H}R^{H*}, -OR^{H}, -OC(O)R^{H}, -C(O)OR^{H}, -SR^{H}, -SC(O)R^{H}, and tri(C₁₋₆-alkyl/aryl)silyloxy;
each R^{H} and R^{H*} independently being selected from hydrogen, optionally substituted C₁₋₆-alkyl, optionally substituted aryl, and optionally substituted aryl-C₁₋₆-alkyl;
A⁴ and A⁵ independently are selected from C₁₋₆-alkylene; and
R⁵ is selected from iodo, bromo, chloro, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen;
said method comprising the following steps:
treating an intermediate of the general formula I: 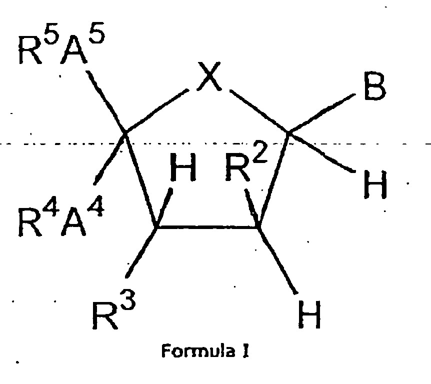
wherein
X, B, R³, A⁴, and A⁵ are as defined above;
R² is selected from iodo, C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen; R³ and
R² may together form an epoxide and
R⁴ and R⁵ independently are as defined for R⁵ above;
with a nucleophile selected from halogen, ⁻N₃, ⁻NR^{H}R^{H*}, ⁻SR^{H}, ⁻⁻S, ⁻SeR^{H}, ⁻⁻Se, ⁻NR^{H}C(O)R^{H*}, ⁻SC(O)R^{H}, and organometallic hydrocarbyl radicals,
so as to substitute R², and
effecting ring-closure between the C2' and C4' positions so as to yield the LNA analogue of the formula IV; or
A method for the synthesis of an LNA analogue of the general formula VIII 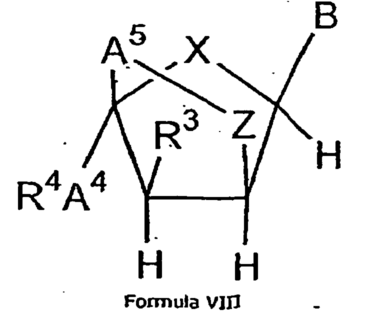 said method comprising the following steps:
treating an intermediate of the general formula IX 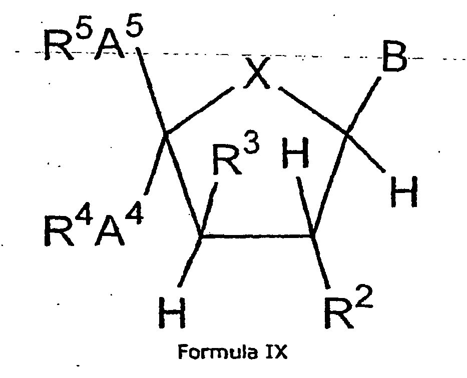
with a nucleophile selected from halogen, ⁻N₃, ⁻NR^{H}R^{H*}, ⁻OR^{H}, ⁻OH, ⁻SR^{H}, ⁻⁻S, ⁻SeR^{H}, ⁻⁻Se, ⁻NR^{H}C(O)R^{H*}, ⁻SC(O)R^{H}, and organometallic hydrocarbyl radicals,
so as to substitute R², and
effecting ring-closure between the C2' and C4' positions so as to yield the LNA analogue of the formula VIII,
wherein X, B, R², R³, R⁴, R⁵, A⁴ and A⁵ are as defined above and wherein Z is selected from -CH₂-, -NR^{H}-, -O- -S-, and -Se-.

2. The method according to claim 1, wherein
R² is selected from C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more-halogen ;
R³ is optionally substituted aryl(C₁₋₆-alkyl)oxy; and
R⁴ and R⁵ are independently selected from C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen.

3. The method according to any one of the preceding claims, wherein A⁴ and A⁵ are methylene.

4. The method according to any of the preceding claims, wherein R⁴ and R⁵ are identical.

5. The method according to any one of the preceding claims, wherein X is -O-.

6. The method according to any one of the preceding claims, wherein B is selected from adenine, guanine, 2,6-diaminopurine, thymine, 2-thiothymine, cytosine, methyl cytosine, uracil, 5-fluorocytosine, xanthine, 6-aminopurine, 2-aminopurine, 6-chloro-2-aminopurine, and 6-chloropurine; R² is selected from C₁₋₆-alkylsulfonyloxy substituted with one or more halogen; R³ is benzyl ; and R⁴ and R⁵ are independently selected from C₁₋₆-alkylsulfonyloxy optionally substituted with one or more halogen, and arylsulfonyloxy optionally substituted with one or more substituents selected from nitro, halogen, C₁₋₆-alkyl, and C₁₋₆-alkyl substituted with one or more halogen.

7. The method according to any one of the preceding claims, wherein R⁴ and R⁵ are independently selected from methanesulfonyloxy, trifluoromethanesulfonyloxy, ethanesulfonyloxy, 2,2,2-trifluoroethanesulfonyloxy, propanesulfonyloxy, isopropanesulfonyloxy, butanesulfonyloxy, nonafluorobutanesulfonyloxy, pentanesulfonyloxy, cyclopentanesulfonyloxy, hexanesulfonyloxy, cyclohexanesulfonyloxy, α-toluenesulfonyloxy 2-chloro-α-toluenesulfonyloxy, *ortho*-toluenesulfonyloxy, *meta-*toluenesulfonyloxy, *para*-toluenesulfonyloxy, benzenesulfonyloxy, *ortho-*bromobenzenesulfonyloxy, *meta*-bromobenzenesulfonyloxy, *para*-bromobenzenesulfonyloxy, *ortho*-nitrobenzenesulfonyloxy, *meta*-nitrobenzenesulfonyloxy, and *para*-nitrobenzenesulfonyloxy.

8. The method according to any one of the preceding claims, wherein the intermediate has the formula III 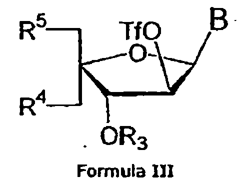 wherein B, R⁴ and R⁵ are as defined in any of the preceding claims and wherein R³ is -OR^{H} or -OC(O)R^{H} where R^{H} is as defined in claim 1.

9. The method according to any one of claims 1-7, wherein the intermediate has the formula X 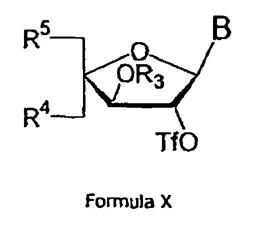 wherein B, R⁴ and R⁵ are as defined in any of the preceding claims and wherein R³ is -OR^{H} or -OC(O)R^{H} where R^{H} is as defined in claim 1.

10. The method according to any one of the preceding claims, wherein B is selected from adenine, guanine, 2,6-diaminopurine, thymine, 2-thiothymine, cytosine, methyl cytosine, uracil, 5-fluorocytosine, xanthine, 6-aminopurine, 2-aminopurine, 6-chloro-2-aminopurine, and 6-chloropurine; R³ is benzyloxy; and R⁴ and R⁵ are both methylsulfonyloxy.

11. The method according to any one of the preceding claims, wherein the nucleophile is selected from ⁻N₃, ⁻NR^{H}R^{H*}, ⁻SR^{H}, ⁻⁻S, ⁻NR^{H}C(O)R^{H*}, and ⁻SC(O)R^{H}.

12. The method according to any one of the preceding claims, wherein Z is -S-.

13. The method according to claim 12, wherein the nucleophile is Na₂S or potassium thioacetate.

14. The method according to claim 13, wherein the ring-closure is effected under the influence of lithium hydroxide in a polar aprotic solvent.

15. The method according to any one of the claims 1-11, wherein Z is -NH-.

16. The method according to claim 15, wherein the nucleophile is sodium azide.

17. The method according to claim 16, wherein the ring-closure is effected under the influence of sodium hydroxide and trimethylphosphane in a polar aprotic solvent.

18. The method according to any one of the claims 15-17, wherein the synthesis further comprises the step of converting the LNA analogue wherein Z is -NH- to an LNA analogue where Z is -N(C₁₋₆-alkyl)- or 14(aryl) by reacting a solution of the former LNA analogue with a reducing agent and a C₁₋₆-alkanal or an aromatic aldehyde or where Z is N(acyl) by reacting with an acid chloride or an acid anhydride.

19. The method according to claim 18, wherein the C₁₋₆-alkanal is formaldehyde, or the aromatic aldehyde is benzaldehyde, pyrene-1-carbaldehyde, or phthalimidoacetaldehyde and the reducing agent is NaBCNH₃, or wherein the acid chloride-is benzoyl chloride or pyren-1-ylcarbonyl chloride.

20. A compound of the formula 1 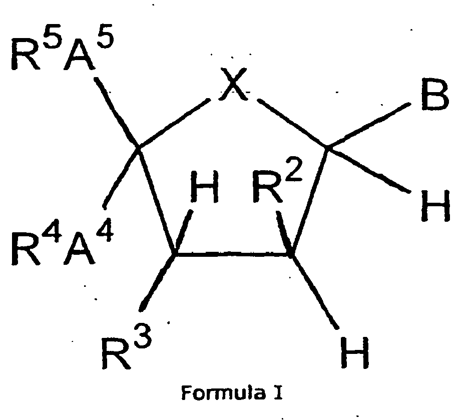 wherein X, B, R², R³, R⁴, R⁵, A⁴ and A⁵ are as defined in any one of claims 1-19
with the proviso that the compound is not selected from
1-(3-azido-3-deoxy-2,5-di-*O*-methanesulfonyl-4-*C*-(methansulfonyloxymethyl)-*β*-D-*erythro-*pentofuranosyl)thymine and
1-(3-*O*-bentyl-2,5-di-*O*-methanesulfonyl-4-*C*-(methansulfonyloxymethyl)-*β*-D-*erythro*pentofuranosyl)thymine.

21. A compound of the formula IX 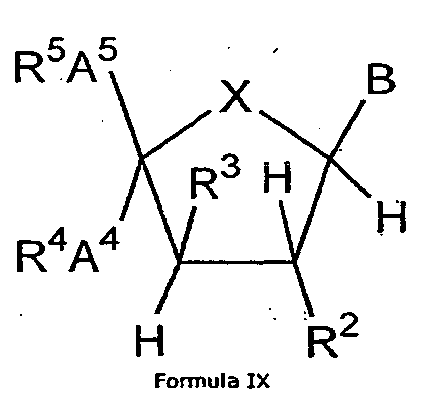 wherein X, B, R², R³, R⁴, R⁵, A⁴ and A⁵ are as defined in any one of claims 1-19
with the proviso that the compound is not 1-(3-*O*-benzyl-2,5-di-*O*-methanesulfonyl-4-*C-*(methansulfonyloxymethyl)-α-L-*threo*-pentofuranosyl)thymine.

22. The compound according to claim 20, wherein the compound has the formula III 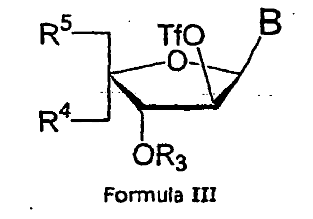 and wherein B, R⁴ and R⁵ are as defined in any of the preceding claims and wherein R³ is -OR^{H} or -OC(O)R^{H} where R^{H} is as defined in claim 1.

23. The compound according to claim 21, wherein the compound has the formula X 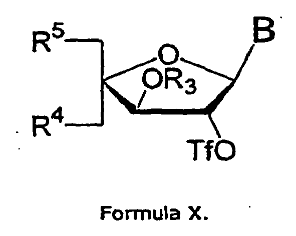 and wherein B, R⁴ and R⁵ are as defined in any of the preceding claims and wherein R³ is -OR^{H} or -OC(O)R^{H} where R^{H} is as defined in claim 1.

24. An oligonucletiode comprising a compound of formula XI 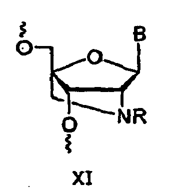 wherein R is selected from benzoyl, benzyl, pyrine-1-ylcarbonyl, 1-pyren-1-ylmethyl and 2-aminoethyl.

25. The oligonucleotide according to claim 24, wherein said oligonucleotide is a 9-mer and contains two or three LNA monomers of formula XI.

## Patentansprüche

1. Verfahren zur Synthese eines LNA-Analogs der allgemeinen Formel IV 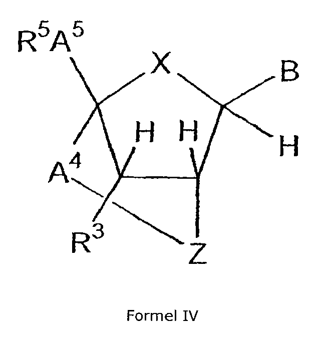 worin
X aus -CH₂-, -NR^{H}-, -O- und -S- ausgewählt ist;
Z aus -CH₂-, -NR^{H}-, -S- und -Se- ausgewählt ist;
B eine Nukleobase ist;
R³ aus -R^{H}, -N₃, -NR^{H}R^{H*}, -NR^{H}C(O)R^{H*}, -C(O)NR^{H}R^{H*}, -OR^{H}, -OC(O)R^{H}, -C(O)OR^{H}, -SR^{H}, -SC(O)R^{H} und tri(C₁₋₆-Alkyl/Aryl)silyloxy ausgewählt ist;
R^{H} und R^{H*}, jeweils unabhängig ausgewählt werden aus Hydrogen, gegebenenfalls substituiertem C₁₋₆-Alkyl, gegebenenfalls substituiertem Aryl und gegebenenfalls substituiertem Aryl-C₁₋₆-Alkyl;
A⁴ und A⁵ unabhängig ausgewählt sind aus C₁₋₆-Alkylen; und
R⁵ ausgewählt ist aus Jod, Brom, Chlor, C₁₋₆-Alkylsulfonyloxy, gegebenenfalls substituiert mit einem oder mehreren Halogen(en), und Arylsulfonyloxy, gegebenenfalls substituiert mit einem oder mehreren Substituent(en), ausgewählt aus Nitro, Halogen, C₁₋₆-Alkyl und C₁₋₆-Alkyl, substituiert mit einem oder mehreren Halogen(en);
das Verfahren folgende Stufen umfasst:
Behandlung eines Zwischenprodukts der allgemeinen Formel I: 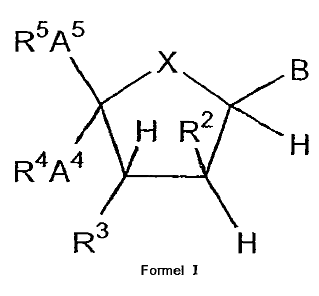
worin
X, B, R³, A⁴ und A⁵ wie oben definiert sind;
R² ausgewählt ist aus Jod, C₁₋₆-Alkylsulfonyloxy, gegebenenfalls substituiert mit einem oder mehreren Halogen(en), und Arylsulfonyloxy, gegebenenfalls substituiert mit einem oder mehreren Substituent(en), ausgewählt aus Nitro, Halogen, C₁₋₆-Alkyl, und C₁₋₆-Alkyl, substituiert mit einem oder mehreren Halogen(en); R³ und R² zusammen ein Epoxid bilden können und
R⁴ und R⁵ unabhängig wie für R⁵ oben definiert sind;
mit einem Nucleophil, ausgewählt aus Halogen, ⁻N_{3,} ⁻NR^{H}R^{H*}, ⁻SR^{H}, ⁻⁻S, ⁻SeR^{H}, ⁻⁻Se, ⁻NR^{H}C(O)R^{H*}, ⁻SC(O)R^{H} und organometallischen Kohlenwasserstoffresten,
um R² zu ersetzen, und
den Ringschluss zwischen den C2'- und C4'-Positionen durchzuführen, um das LNA-Analog der Formel IV hervorzubringen; oder
Verfahren zur Synthese eines LNA-Analogs der allgemeinen Formel VIII, 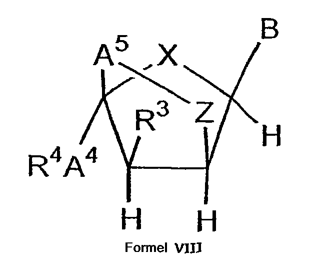 wobei das Verfahren folgende Stufen umfasst:
Behandlung eines Zwischenprodukts der allgemeinen Formel IX 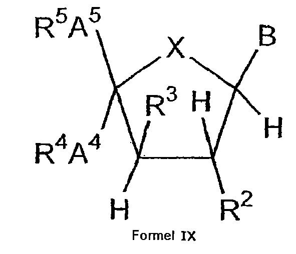 mit einem Nucleophil, ausgewählt aus Halogen, ⁻N₃, ⁻NR^{H}R^{H*}, ⁻OR^{H}, ⁻OH, ⁻SR^{H}, ⁻⁻S, ⁻SeR^{H}, ⁻⁻Se,⁻NR^{H}C(O)R^{H*}, ⁻SC(O)R^{H} und organometallischen Kohlenwasserstoffresten,
um R² zu ersetzen, und
die Ringschluss zwischen den C2'- und C4'-Positionen durchzuführen, um das LNA-Analog der Formel VIII hervorzubringen,
worin X, B, R², R³, R⁴, R⁵, A⁴ und A⁵ wie oben definiert sind, und worin Z aus -CH₂-, -NR^{H}-, -O- -S- und -Se- ausgewählt ist.

2. Verfahren nach Anspruch 1, worin
R² ausgewählt ist aus C₁₋₆-Alkylsulfonyloxy, gegebenenfalls substituiert mit einem oder mehreren Halogen(en), und Arylsulfonyloxy, gegebenenfalls substituiert mit einem oder mehreren Substituent(en), ausgewählt aus Nitro, Halogen, C₁₋₆-Alkyl und C₁₋₆-Alkyl, substituiert mit einem oder mehreren Halogen(en);
R³ gegebenenfalls substituiertes Aryl(C₁₋₆-alkyl)oxy ist; und
R⁴ und R⁵ unabhängig ausgewählt sind aus C₁₋₆-Alkylsulfonyloxy, gegebenenfalls substituiert mit einem oder mehreren Halogen(en), und Arylsulfonyloxy, gegebenenfalls substituiert mit einem oder mehreren Substituent(en), ausgewählt aus Nitro, Halogen, C₁₋₆-Alkyl und C₁₋₆-Alkyl, substituiert mit einem oder mehreren Halogen(en).

3. Verfahren nach irgendeinem der vorhergehenden Ansprüche, worin A⁴ und A⁵ Methylen sind.

4. Verfahren nach irgendeinem der vorhergehenden Ansprüche, worin R⁴ und R⁵ identisch sind.

5. Verfahren nach irgendeinem der vorhergehenden Ansprüche, worin X -O- ist.

6. Verfahren nach irgendeinem der vorhergehenden Ansprüche, worin B ausgewählt ist aus Adenin, Guanin, 2,6-Diaminopurin, Thymin, 2-Thiothymin, Cytosin, Methylcytosin, Uracil, 5-Fluorcytosin, Xanthin, 6-Aminopurin, 2-Aminopurin, 6-Chlor-2-Aminopurin und 6-Chlorpurin; R² ausgewählt ist aus C₁₋₆-Alkylsulfonyloxy, substituiert mit einem oder mehreren Halogen(en); R³ Benzyl ist; und R⁴ und R⁵ unabhängig ausgewählt sind aus C₁₋₆-Alkylsulfonyloxy, gegebenenfalls substituiert mit einem oder mehreren Halogen(en), und Arylsulfonyloxy, gegebenenfalls substituiert mit einem oder mehreren Substituenten, ausgewählt aus Nitro, Halogen, C₁₋₆-Alkyl, und C₁₋₆-Alkyl, substituiert mit einem oder mehreren Halogen(en).

7. Verfahren nach irgendeinem der vorhergehenden Ansprüche, worin R⁴ und R⁵ unabhängig ausgewählt sind aus Methansulfonyloxy, Trifluormethansulfonyloxy, Ethansulfonyloxy, 2,2,2-Trifluorethansulfonyloxy, Propansulfonyloxy, Iso-Propansulfonyloxy, Butansulfonyloxy, Nonafluorbutansulfonyloxy, Pentansulfonyloxy, Cyclopentansulfonyloxy, Hexansulfonyloxy, Cyclohexansulfonyloxy, α-Toluolsulfonyloxy, 2-Chlor-α-Toluolsulfonyloxy, *Ortho*toluolsulfonyloxy, *Meta*-toluolsulfonyloxy, *Para*-Toluolsulfonyloxy, Benzolsulfonyloxy, *Ortho*-Brombenzolsulfonyloxy, *Meta*-Brombenzolsulfonyloxy, *Para*-Brombenzolsulfonyloxy, *Ortho*-Nitrobenzolsulfonyloxy, *Meta-*Nitrobenzolsulfonyloxy und *Para*-Nitrobenzolsulfonyloxy.

8. Verfahren nach irgendeinem der vorhergehenden Ansprüche, worin das Zwischenprodukt die Formel III hat, 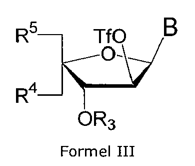 worin B, R⁴ und R⁵ wie in irgendeinem der vorhergehenden Ansprüche definiert sind, und worin R³ -OR^{H} oder -OC(O)R^{H} ist, wo R^{H} wie in Anspruch 1 definiert ist.

9. Verfahren nach irgendeinem der Ansprüche 1-7, worin das Zwischenprodukt die Formel X hat, 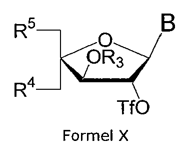 worin B, R⁴ und R⁵ wie in irgendeinem der vorhergehenden Ansprüche definiert sind, und worin R³ -OR^{H} oder -OC(O)R^{H} ist, wo R^{H} wie in Anspruch 1 definiert ist.

10. Verfahren nach irgendeinem der vorhergehenden Ansprüche, worin B ausgewählt ist aus Adenin, Guanin, 2,6-Diaminopurin, Thymin, 2-Thiothymin, Cytosin, Methylcytosin, Uracil, 5-Fluorcytosin, Xanthin, 6-Aminopurin, 2-Aminopurin, 6-Chlor-2-Aminopurin und 6-Chlorpurin; R³ Benzyloxy ist; und R⁴ und R⁵ beide Methylsulfonyloxy sind.

11. Verfahren nach irgendeinem der vorhergehenden Ansprüche, worin das Nucleophil ausgewählt ist aus ⁻N₃, ⁻NR^{H}R^{H*}, ⁻SR^{H}, ⁻⁻S, ⁻NR^{H}C(O)R^{H*} und ⁻SC(O)R^{H}.

12. Verfahren nach irgendeinem der vorhergehenden Ansprüche, worin Z -S- ist.

13. Verfahren nach Anspruch 12, worin das Nucleophil Na₂S oder Kaliumthioazetat ist.

14. Verfahren nach Anspruch 13, worin der Ringschluss unter Einfluss von Lithiumhydroxid in einem polaren aprotischen Lösungsmittel durchgeführt ist.

15. Verfahren nach irgendeinem der Ansprüche 1-11, worin Z -NH- ist.

16. Verfahren nach Anspruch 15, worin das Nucleophil Natriumazid ist.

17. Verfahren nach Anspruch 16, worin der Ringschluss unter Einfluss von Natriumhydroxid und Trimethylphosphan in einem polaren aprotischen Lösungsmittel durchgeführt ist.

18. Verfahren nach irgendeinem der Ansprüche 15-17, worin die Synthese ferner die Stufe umfasst, wo das LNA-Analog, worin Z -NH- ist, in ein LNA-Analog, wo Z
- N(C₁₋₆-Alkyl)- oder -N(aryl) ist, durch eine Reaktion einer Lösung des früheren LNA-Analogs mit einem Reduktionsmittel und einem C₁₋₆-Alkanal oder einem aromatischen Aldehyd, oder wo Z N(acyl) ist, durch Reaktion mit einem Säurechlorid oder einem Säureanhydrid umgewandelt wird.

19. Verfahren nach Anspruch 18, worin das C₁₋₆-Alkanal Formaldehyd ist, oder das aromatische Aldehyd Benzaldehyd, Pyren-1-Carbaldehyd oder Phthalimidoacetaldehyd ist, und das Reduktionsmittel NaBCNH₃ ist, oder worin das Säurechlorid Benzoylchlorid oder Pyren-1-ylcarbonylchlorid ist.

20. Verbindung der Formel I, 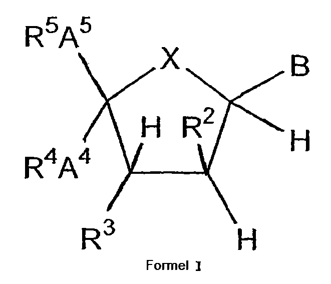 worin X, B, R², R³, R⁴, R⁵, A⁴ und A⁵ wie in irgendeinem der Ansprüche 1-19 definiert sind,
mit der Bedingung, dass die Verbindung nicht ausgewählt ist aus 1-(3-Azido-3-deoxy-2,5-di-*O*-methansulfonyl-4-*C*-(methansulfonyloxymethyl)-β-D-*erythro*-pentofuranosyl)thymin und
1-(3-*O*-Benzyl-2,5-di-*O*-methansulfonyl-4-*C*-(methansulfonyloxymethyl)-β-D-*erythro*-pentofuranosyl)thymin.

21. Verbindung der Formel IX 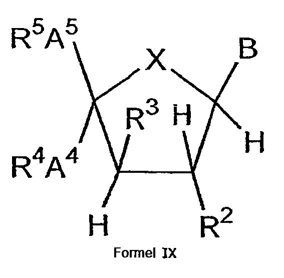 worin X, B, R², R³, R⁴, R⁵, A⁴ und A⁵ wie in irgendeinem der Ansprüche 1-19 definiert sind,
mit der Bedingung, dass die Verbindung nicht 1-(3-*O*-Benzyl-2,5-di-*O-*methanesulfonyl-4-*C*-(methansulfonyloxymethyl)-α-L-*threo*-pentofuranosyl)thymin ist.

22. Verbindung nach Anspruch 20, worin die Verbindung die Formel III hat 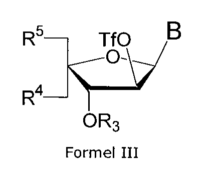 und worin B, R⁴ und R⁵ wie in irgendeinem der vorhergehenden Ansprüche definiert sind, und worin R³ -OR^{H} oder -OC(O)R^{H} ist, wo R^{H} wie in Anspruch 1 definiert ist.

23. Verbindung nach Anspruch 21, worin die Verbindung die Formel X hat 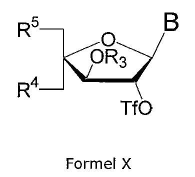 und worin B, R⁴ und R⁵ wie in irgendeinem der vorhergehenden Ansprüche definiert sind, und worin R³ -OR^{H} oder -OC(O)R^{H} ist, wo R^{H} wie in Anspruch 1 definiert ist.

24. Oligonukleotid umfassend eine Verbindung der Formel XI 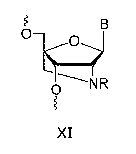 worin R aus Benzoyl, Benzyl, Pyrin-1-ylcarbonyl, 1-Pyren-1-ylmethyl und 2-Aminoethyl ausgewählt ist.

25. Oligonukleotid nach Anspruch 24, worin das Oligonukleotid ein 9-Mer ist und zwei oder drei LNA-Monomere der Formel XI enthält.

## Revendications

1. Méthode pour la synthèse d'un analogue de LNA de formule générale IV 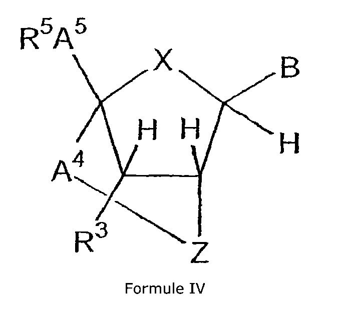 où
X est choisi parmi -CH₂-, -NR^{H}-, -O-, et -S- ;
Z est choisi parmi -CH₂-, -NR^{H}-, -S-, et -Se- ;
B est une nucléobase ;
R³ est choisi parmi -R^{H}, -N₃, -NR^{H}R^{H*}, -NR^{H}C(O)R^{H*}, -C(O)NR^{H}R^{H*}, -OR^{H}, -OC(O)R^{H}, -C(C)OR^{H}, -SR^{H}, -SC(O)R^{H}, et tri(C₁₋₆-alkyl/aryl)silyloxy ;
chaque R^{H} et R^{H*} étant choisi indépendamment parmi l'hydrogène, un alkyle C₁₋₆ facultativement substitué, un aryle facultativement substitué, et un aryl-C₁₋₆-alkyle facultativement substitué ;
A⁴ et A⁵ sont choisis indépendamment parmi les alkylènes C₁₋₆ et
R⁵ est choisi parmi un groupe iodo, bromo, chloro, un C₁₋₆-alkylsulfonyloxy facultativement substitué par un ou plusieurs halogènes, et un arylsulfonyloxy facultativement substitué par un ou plusieurs substituants choisis parmi les groupes nitro, halogéno, les alkyles C₁₋₆, et les alkyles C₁₋₆ substitués par un ou plusieurs halogènes ;
ladite méthode comprenant les étapes suivantes :
traitement d'un intermédiaire de formule générale I : 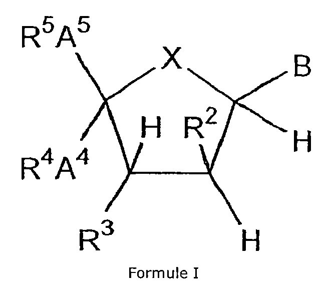
où
X, B, R³, A⁴, et A⁵ sont tels que définis ci-dessus ;
R² est choisi parmi un groupe iodo, un C₁₋₆-alkylsulfonyloxy facultativement substitué par un ou plusieurs halogènes, et un arylsulfonyloxy facultativement substitué par un ou plusieurs substituants choisis parmi les groupes nitro, halogéno, les alkyles C₁₋₆, et les alkyles C₁₋₆ substitués par un ou plusieurs halogènes ; R³ et R² peuvent ensemble former un époxyde et
R⁴ et R⁵ indépendamment sont tels que définis ci-dessus pour R⁵;
avec un nucléophile choisi parmi un halogène, ⁻N₃, ⁻NR^{H}R^{H*}, ⁻SR^{H}, ⁻⁻S, ⁻SeR^{H}, ⁻⁻Se, ⁻NR^{H}C(O)R^{H*}, ⁻SC(O)R^{H}, et les radicaux hydrocarbyles organométalliques,
de façon à substituer R², et
réalisation de la fermeture du cycle entre les positions C2' et C4' de façon à produire l'analogue de LNA de formule IV ; ou
méthode pour la synthèse d'un analogue de LNA de formule générale VIII 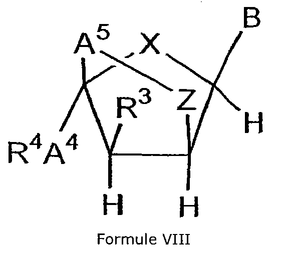 ladite méthode comprenant les étapes suivantes :
traitement d'un intermédiaire de formule générale IX : 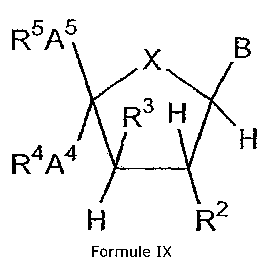 avec un nucléophile choisi parmi un halogène, ⁻N₃, ⁻NR^{H}R^{H*}, ⁻OR^{H}, ⁻OH, ⁻SR^{H}, ⁻⁻S, ⁻SeR^{H}, ⁻⁻Se, ⁻NR^{H}C(O)R^{H*}, ⁻SC(O)R^{H}, et les radicaux hydrocarbyles organométalliques,
de façon à substituer R², et
réalisation de la fermeture du cycle entre les positions C2' et C4' de façon à produire l'analogue de LNA de formule VIII,
où X, B, R², R³, R⁴, R⁵, A⁴ et A⁵ sont tels que définis ci-dessus et où Z est choisi parmi -CH₂-, -NR^{H}-, -O-, -S-, et -Se-.

2. La méthode selon la revendication 1, où
R² est choisi parmi un C₁₋₆-alkylsulfonyloxy facultativement substitué par un ou plusieurs halogènes, et un arylsulfonyloxy facultativement substitué par un ou plusieurs substituants choisis parmi les groupes nitro, halogéno, les alkyles C₁₋₆, et les alkyles C₁₋₆ substitués par un ou plusieurs halogènes ; et
R³ est un aryl(C₁₋₆-alkyl)oxy facultativement substitué ; et
R⁴ et R⁵ sont choisis indépendamment parmi un C₁₋₆-alkylsulfonyloxy facultativement substitué par un ou plusieurs halogènes, et un arylsulfonyloxy facultativement substitué par un ou plusieurs substituants choisis parmi les groupes nitro, halogéno, les alkyles C₁₋₆, et les alkyles C₁₋₆ substitués par un ou plusieurs halogènes.

3. La méthode selon une quelconque des revendications précédentes, où A⁴ et A⁵ sont méthylènes.

4. La méthode selon une quelconque des revendications précédentes, où R⁴ et R⁵ sont identiques.

5. La méthode selon une quelconque des revendications précédentes, où X est -O-.

6. La méthode selon une quelconque des revendications précédentes, où B est choisi parmi l'adénine, la guanine, une 2,6-diaminopurine, la thymine, la 2-thiothymine, la cytosine, une méthyl-cytosine, l'uracile, la 5-fluorocytosine, la xanthine, une 6-aminopurine, une 2-aminopurine, une 6-chloro-2-amino-purine, et une 6-chloropurine ; R² est choisi parmi un C₁₋₆-alkylsulfonyloxy substitué par un ou plusieurs halogènes ; R³ est benzyle ; et R⁴ et R⁵ sont indépendamment choisis parmi un C₁₋₆-alkylsulfonyloxy facultativement substitué par un ou plusieurs halogènes, et un arylsulfonyloxy facultativement substitué par un ou plusieurs substituents choisis parmi un nitro, un halogéno, un alkyle C₁₋₆, et un alkyle C₁₋₆ substitué par un ou plusieurs halogènes.

7. La méthode selon une quelconque des revendications précédentes, où R⁴ et R⁵ sont choisis indépendamment parmi un méthanesulfonyloxy, un trifluorométhanesulfonyloxy, un éthanesulfonyloxy, un 2,2,2-trifluoroéthanesulfonyloxy, un propanesulfonyloxy, un isopropanesulfonyloxy, un butanesulfonyloxy, un nonafluorobutanesulfonyloxy, un pentanesulfonyloxy, un cyclopentanesulfonyloxy, un hexanesulfonyloxy, un cyclohexanesulfonyloxy, un α-toluènesulfonyloxy, un 2-chloro-α-toluènesulfonyloxy, un *orhto*-toluènesulfonyloxy, un *méta*-toluènesulfonyloxy, un *para*-toluènesulfonyloxy, un benzènesulfonyloxy, un *ortho-*bromobenzènesulfonyloxy, un *méta*-bromobenzènesulfonyloxy, un *para-*bromobenzènesulfonyloxy, un *ortho*-nitrobenzènesulfonyloxy, un *méta-*nitrobenzènesulfonyloxy, et un *para*-nitrobenzènesulfonyloxy.

8. La méthode selon une quelconque des revendications précédentes, où l'intermédiaire a la formule III 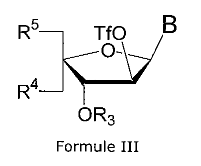 où B, R⁴ et R⁵ sont tels que définis dans une quelconque des revendications précédentes et où R³ est -OR^{H} ou -OC(O)R^{H} où R^{H} est tel que défini dans la revendication 1.

9. La méthode selon une quelconque des revendications précédentes, où l'intermédiaire a la formule X 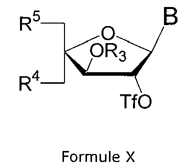 où B, R⁴ et R⁵ sont tels que définis dans une quelconque des revendications précédentes et où R³ est -OR^{H} ou -OC(O)R^{H} où R^{H} est tel que défini dans la revendication 1.

10. La méthode selon une quelconque des revendications précédentes, où B est choisi parmi l'adénine, la guanine, une 2,6-diaminopurine, la thymine, la 2-thiothymine, la cytosine, une méthyl-cytosine, l'uracile, la 5-fluorocytosine, la xanthine, une 6-aminopurine, une 2-aminopurine, une 6-chloro-2-amino-purine, et une 6-chloropurine ; R³ est benzyloxy ; et R⁴ et R⁵ sont tous deux méthylsulfonyloxy.

11. La méthode selon une quelconque des revendications précédentes, où le nucléophile est choisi parmi ⁻N₃, ⁻NR^{H}R^{H*}, ⁻SR^{H}, ⁻⁻S, ⁻NR^{H}C(O)R^{H*}, et ⁻SC(O)R^{H}.

12. La méthode selon une quelconque des revendications précédentes, où Z est -S-.

13. La méthode selon la revendication 12, où le nucléophile est Na₂S ou le thioacétate de potassium.

14. La méthode selon la revendication 13, où la fermeture du cycle est effectuée sous l'influence d'hydroxyde de lithium dans un solvant polaire aprotique.

15. La méthode selon une quelconque des revendications 1-11, où Z est -NH-.

16. La méthode selon la revendication 15, où le nucléophile est l'azide de sodium.

17. La méthode selon la revendication 16, où la fermeture du cycle est effectuée sous l'influence d'hydroxyde de sodium et du triméthylphosphane dans un solvant polaire aprotique.

18. La méthode selon une quelconque des revendications 15-17, où la synthèse comprend également l'étape de conversion de l'analogue de LNA dans lequel Z est -NH- en un analogue de LNA dans lequel Z est -N(C₁₋₆-alkyl)- ou N(aryl) par la réaction d'une solution du précédent analogue de LNA avec un agent réducteur et un alcanal C₁₋₆ ou un aldéhyde aromatique ou dans lequel Z est N(acyl) par la réaction avec un acide chlorique ou un anhydride d'acide.

19. La méthode selon la revendication 18, où l'alcanal C₁₋₆ est le formaldéhyde, ou l'aldéhyde aromatique est le benzaldéhyde, le pyrène-1-carbaldéhyde, ou le phthalimidoacétaldéhyde et l'agent réducteur est NaBCNH₃, ou dans laquelle le chlorure d'acide est un chlorure de benzoyle ou un chlorure de pyrène-1-carbonyle.

20. Composé de formule I 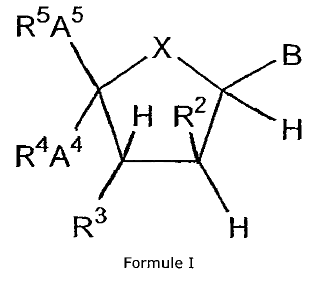 où X, B, R², R³, R⁴, R⁵, A⁴ et A⁵ sont tels que définis dans une quelconque des revendications 1-19
avec la condition que le composé ne soit pas choisi parmi
la 1-(3-azido-3-déoxy-2,5-di-*O*-méthanesulfonyl-4-*C*-(méthanesulfonyloxyméthyl)-β-D-*érythro*-pentofuranosyl)thymine et
la 1-(3-*O*-benzyl-2,5-di-*O*-méthanesulfonyl-4-*C*-(méthanesulfonyloxyméthyl)-β-D-*érythro*-pentofuranosyl)thymine.

21. Composé de formule IX 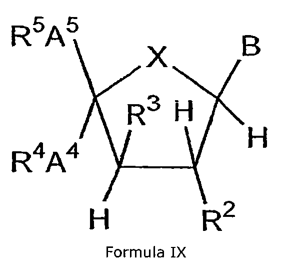 où X, B, R², R³, R⁴, R⁵, A⁴ et A⁵ sont tels que définis dans une quelconque des revendications 1-19
avec la condition que le composé ne soit pas la 1-(3-*O*-benzyl-2,5-di-*O-*méthanesulfonyl-4-*C*-(méthanesulfonyloxyméthyl)-α-L-*thréo*pentofuranosyl)thymine.

22. Le composé selon la revendication 20, où le composé a la formule III 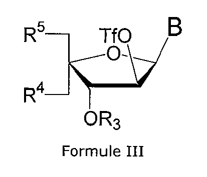 et où B, R⁴ et R⁵ sont tels que définis dans une quelconque des revendications précédentes et où R³ est -OR^{H} ou -OC(O)R^{H} où R^{H} est tel que défini dans la revendication 1.

23. Le composé selon la revendication 21, où le composé a la formule X 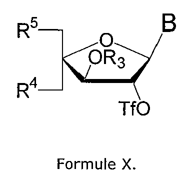 et où B, R⁴ et R⁵ sont tels que définis dans une quelconque des revendications précédentes et où R³ est -OR^{H} ou -OC(O)R^{H} où R^{H} est tel que défini dans la revendication 1.

24. Un oligonucléotide comprenant un composé de formule XI 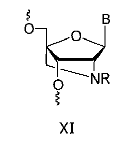 où R est choisi parmi un benzoyle, un benzyle, un pyrine-1-ylcarbonyle, un 1-pyrène-1-ylméthyle et un 2-aminométhyle.

25. L'oligonucléotide selon la revendication 24, où ledit oligonucléotide est un 9-mère et contient deux ou trois monomères de LNA de formule XI.
